(19) Europäisches Patentamt
European Patent Office
Office européen des brevets

(11) EP 2 837 626 A1

(12) **EUROPEAN PATENT APPLICATION**

(43) Date of publication:
**18.02.2015 Bulletin 2015/08**

(21) Application number: **13180792.7**

(22) Date of filing: **16.08.2013**

(51) Int Cl.:
***C07D 401/14*** *(2006.01)* ***C07D 403/06*** *(2006.01)*
***C07D 403/14*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
- **Ullrich, Axel**
  **80331 München (DE)**
- **Falcenberg, Mathias**
  **82152 Martinsried (DE)**
- **Örfi, Zoltan**
  **1161 Budapest (HU)**

(74) Representative: **Arth, Hans-Lothar**
**ABK Patent Attorneys**
**Jasminweg 9**
**14052 Berlin (DE)**

(54) **Indolinone derivatives as GRK5 modulators**

(57) The present invention relates to benzopyrrolidone derivatives and/or pharmaceutically acceptable salts thereof, the use of these derivatives as pharmaceutically active agents, especially for the prophylaxis and/or treatment of cancer or metabolic diseases, which include diabetes, obesity and impaired adipogenesis, and pharmaceutical compositions containing at least one of said benzopyrrolidone derivatives and/or pharmaceutically acceptable salts thereof. Furthermore, the present invention relates to the use of said benzo-pyrrolidone derivatives as GRK5 inhibitors, thereby regulating the glucose dependent insulin production and/or release.

EP 2 837 626 A1

## Description

The present invention relates to benzopyrrolidone derivatives and/or pharmaceutically acceptable salts thereof, the use of these derivatives as pharmaceutically active agents, especially for the prophylaxis and/or treatment of metabolic diseases or cancer, and pharmaceutical compositions containing at least one of said benzopyrrolidone derivatives and/or pharmaceutically acceptable salts thereof. Metabolic diseases include diabetes, obesity and impaired adipogenesis. Furthermore, the present invention relates to the use of said benzoypyrrolidone derivatives as GRK5 inhibitors, thereby regulating the glucose dependent insulin production and/or release.

## Background of the invention

Diabetes as a leading cause of death in developed countries is a metabolic condition characterized by high blood sugar levels. There are two main types of diabetes: type 1, resulting from insufficient insulin production of the pancreas beta cells, which requires the person to inject insulin; and type 2, resulting from insensitivity of peripheral tissues (such skeletal muscle, liver or adipose tissue) insulin release alterations, and relative insulin deficiency. Diabetes mellitus type 2 is often acquired and accompanied by obesity; it can be treated in first hand by reducing weight, diet and exercise. Type 1 diabetes is a genetic or autoimmune disease; the only effective therapy to date is the supply of exogenous insulin. This therapy does not cure diabetes; the person needs continuous supply of insulin.

The decreased insulin sensitivity of peripheral tissues in type 2 diabetes, which accounts for 90% of all cases of the disease, is initially compensated by an increased release of insulin by the beta cells of the pancreas. At a certain stage of the disease, the pancreas cannot maintain the increased release of insulin anymore. As disease progresses, drugs which are currently available and elevate insulin release have lead to beta-cell damage and loss of insulin production.

Multi-targeted receptor tyrosine kinase inhibitors like Sunitinib and Imatinib have a beneficial effect on insulin release in a dose dependent manner leading to a remission of diabetes type 1 and 2 in patients. However, treatment with multi-targeted receptor tyrosine kinase inhibitors, such Sunitinib, results in many side effects such as the classic hand-foot syndrome, stomatitis, and other dermatologic toxicities. WO 2012/028335 identifies SCYL1, ADCK1, GRK5 and CSNK2A1 as kinases specifically involved in insulin release or sensitivity, so as potential targets for developing a more specific treatment strategy for metabolic diseases, including diabetes.

G-protein-coupled receptor (GPCR) kinase 5 (GRK5) has a potential influence in the insulin regulating mechanism. GRK5 is an important member of the threonine/serine kinase family that phosphorylates GPCRs as part of feedback inhibition of GPCRs in signal transduction. The *in vivo* physiological functions of GRK5 have been ascribed to its kinase activity, phosphorylating and desensitizing specific GPCRs, as well as its kinase independent function and targeting to non-GPCR substrates. GRK5 interacts with I$\kappa$B$\alpha$ and inhibits NF$\kappa$B-mediated transcriptional responses. GRK5 also phosphorylates p53 and regulates p53-mediated apoptosis in response to DNA damage. G protein-coupled receptor kinases (GRKs) specifically phosphorylate serine/threonine residues located on the cytoplasmic loops and C terminus of the receptors. Phosphorylation of a GPCR by GRK promotes the high affinity binding of arrestins and induces receptor internalization, and thus feed-back inhibits GPCR responses to extracellular signals. The *in vivo* physiological functions of various subtypes of GRKs (GRK1-7) have been previously ascribed to phosphorylating and desensitizing specific GPCRs. GRK5 deficiency prevents obesity induced by high fat diet in mice, thus suggesting the central role of GRKS in the adipogenesis regulation and the development of diet induced obesity. (Biochem. Biophys. Res. Commun. 2012, 421, 312). A genome-wide association study in Chinese Hans identified two novel T2D loci, including GRK5 (rs10886471: P = 7.1 x 10(-9)). This study could further show that the risk increasing allele rs10886471 was also associated with higher GRK5 mRNA expression levels, higher fasting insulin, but not with fasting glucose, suggesting that it might impair insulin sensitivity.

Furthermore, GRK5 activity is essential for tumor growth (J. Clin. Neurosci. 2013, 20(7), 1014; J. Urol. 2012, 187(1), 322).

It is object of the present invention to provide compounds and/or pharmaceutically acceptable salts thereof, which can be used as pharmaceutically active agents, especially for prophylaxis and/ or treatment of cancer or metabolic diseases, said metabolic diseases including diabetes obesity and impaired adipogenesis, as well as compositions comprising at least one of those compounds and/or pharmaceutically acceptable salts thereof as pharmaceutically active ingredients.

This object is solved by the compounds and/or their pharmaceutically acceptable salts according to independent claim 1, the compounds of the present invention for use as pharmaceutically active agents, the use of the compounds of the present invention for the preparation of a pharmaceutical composition for the prophylaxis and/or treatment of cancer or metabolic diseases, including diabetes, obesity and impaired adipogenesis, the use of compounds disclosed by the present invention for glucose uptake and glucose dependent regulation of insulin production and/or release, the use of compounds according to the present invention as inhibitors for the protein kinase GRK5.

The objective of the present invention is solved by the teaching of the independent claims. Further advantageous

features, aspects and details of the invention are evident from the dependent claims, the description, the figures, and the examples of the present application.

**Description of the invention**

Thus the present invention relates to compounds of general formula **(I)**

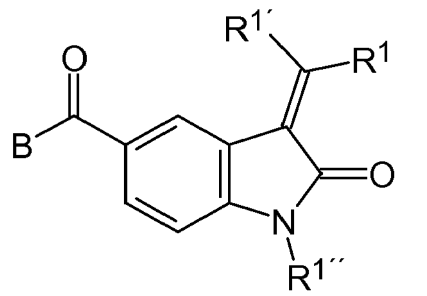

**(I)**

wherein,
B represents:

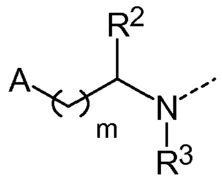

or $-OR^{16}$
$R^1$ and $R^{1'}$ are each independently selected from -Ph, -H,

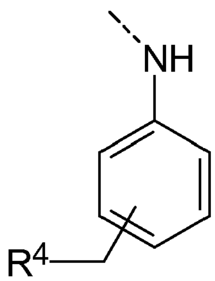

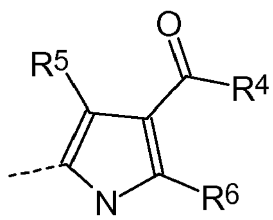

and

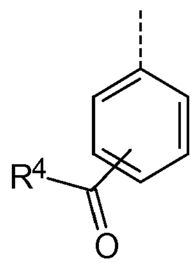

and $R^1$ and $R^{1'}$ cannot be simultaneously -H or -Ph; or in other words one of $R^1$ and $R^{1'}$ is different from hydrogen or phenyl;
$R^{1''}$ represents -H or $-C(O)R^{18}$;
$R^2$ represents: $-R^{19}$, $-C(O)NH_2$, or $-CO_2R^{20}$;
$R^4$ represents: $-R^7$, $-NH-(CH_2)_pR^{7'}$

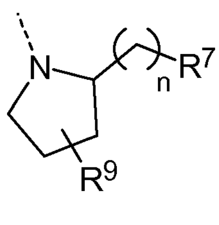

or

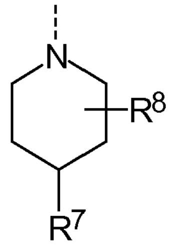

$R^7$ represents:

$-NR^{10}R^{11}$,

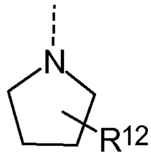
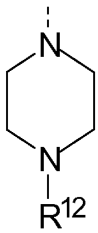
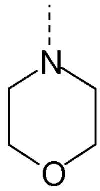

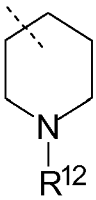
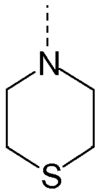
or
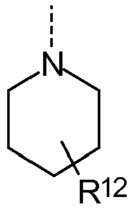

and $R^{10}$ and $R^{11}$ cannot be simultaneously -H; or in other words one of $R^{10}$ and $R^{11}$ is different from hydrogen; $R^{7'}$ represents:

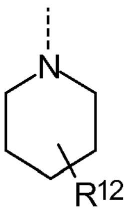
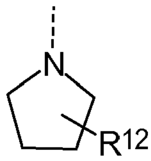
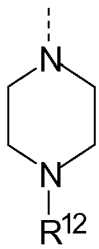
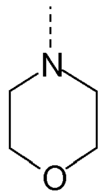

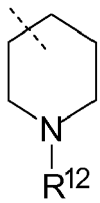
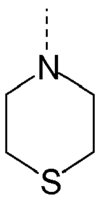
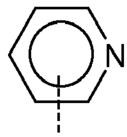
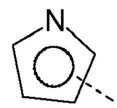

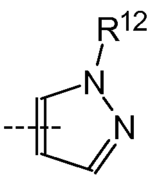
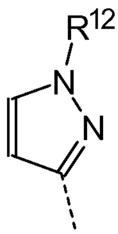
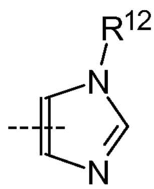
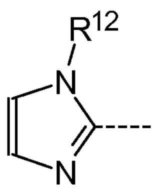

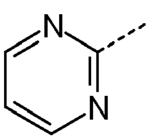
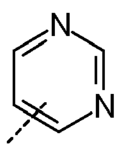
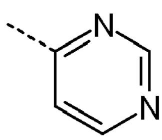
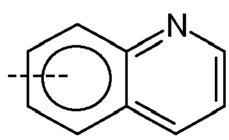

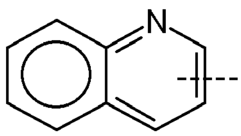 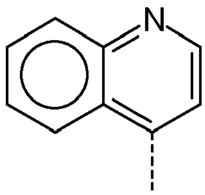 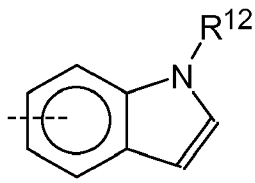 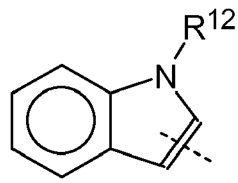

or $-NR^{10}R^{11}$, and $R^{10}$ and $R^{11}$ cannot be simultaneously -H; or in other words one of $R^{10}$ and $R^{11}$ is different from hydrogen;
A represents:

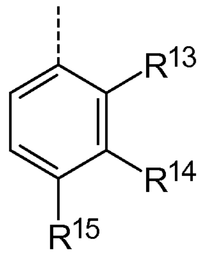

or

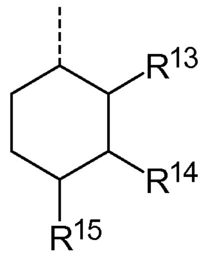

$R^{13}$, $R^{14}$ and $R^{15}$ are each independently selected from the group consisting of: $-R^{21}$, $-R^{22}$, $-R^{23}$, $-OR^{21}$, $-OR^{22}$, $-OR^{23}$, -F, -Cl, -Br, and -I,
$R^{14}$ together with $R^{15}$ may form with the two carbon of the benzene or cyclohexane they are attached to a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;
$R^{13}$ together with $R^2$ may form a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;
$R^3$, $R^8$, $R^9$, $R^{10}$, $R^{11}$, $R^{12}$, $R^{16}$, $R^{17}$ $R^{18}$, $R^{19}$, $R^{20}$, $R^{21}$, $R^{22}$ and $R^{23}$ are each independently selected from the group consisting of: -H, $-CH_3$, $-C_2H_5$, $-C_3H_7$, $-C_4H_9$, $-C_5H_{11}$, -Ph, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH_2Ph$, $-CH(CH_3)-CH=CH$, $-CH=C(CH_3)_2$, $-C(CH_3)=CH-CH_3$, $-CH=CH-CH=CH_2$, $-C{\equiv}CH$, $-C{\equiv}C-CH_3$, $-CH_2-C{\equiv}CH$, $-C_2H_4-C{\equiv}CH$, $-CH_2-C{\equiv}C-CH_3$, and $-C{\equiv}C-C_2H_5$;
m is an integer number selected from 0 and 1,
n is an integer number selected from 1, 2, 3, 4, 5 and 6,
p is an integer number selected from 0, 1 and 2,
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

Preferred compounds according to the present invention are compounds of general formula **(I)**

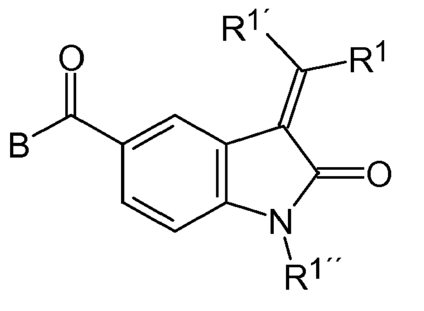

**(I)**

wherein,
B represents:

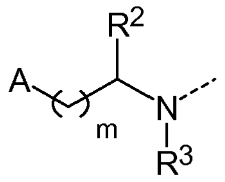

or $-OR^{16}$

$R^1$ and $R^{1'}$ represent independently of each other the following: -Ph, -H,

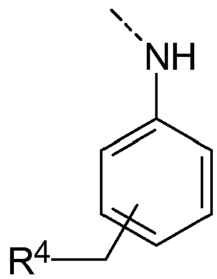

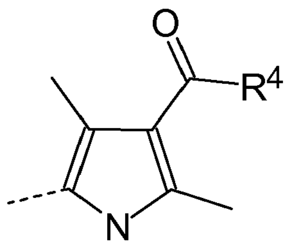

or

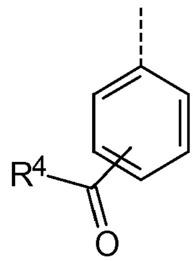

and $R^1$ and $R^{1'}$ cannot be simultaneously -H or -Ph;

$R^2$ represents: $-R^{19}$, $-C(O)NH_2$, or $-CO_2R^3$;

$R^4$ represents the following: $-R^7$, $-NH-(CH_2)_p-R^{7'}$

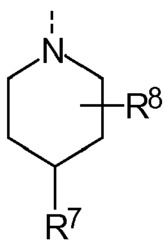

or

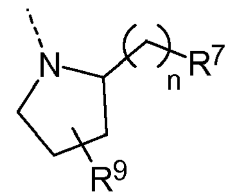

wherein $R^8$ and $R^9$ are -H, and $R^{7'}$ represents $R^7$;

A represents:

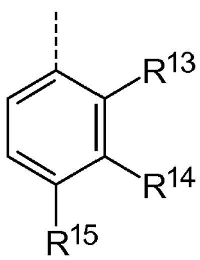

or

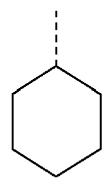

$R^{13}$, $R^{14}$ and $R^{15}$ are each independently selected from the group consisting of: $-R^{21}$, $-R^{22}$, $-R^{23}$, $-OR^{21}$, $-OR^{22}$, $-OR^{23}$, -F, -Cl, -Br, and -I,

$R^{14}$ together with $R^{15}$ may form with the two carbon of the benzene they are attached to a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;

$R^{13}$ together with $R^2$ may form a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;

$R^3$, $R^{10}$, $R^{11}$, $R^{12}$, $R^{16}$, $R^{17}$, $R^{18}$, $R^{19}$, $R^{20}$, $R^{21}$, $R^{22}$ and $R^{23}$, m, n, and p are defined as above;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

The expression prodrug is defined as a pharmacological substance, a drug, which is administered in an inactive or significantly less active form. Once administered, the prodrug is metabolized in the body *in vivo* into the active compound.

The expression tautomer is defined as an organic compound that is interconvertible by a chemical reaction called tautomerization. Tautomerization can be catalyzed preferably by bases or acids or other suitable compounds.

Preferred are compounds of general formula (I), wherein B is

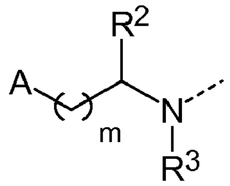

and enantiomers, stereoisomeric forms, mixtures of enantiomers, mixtures of diastereomers, prodrugs, tautomers, hydrates, solvates and racemates of the above mentioned compounds and pharmaceutically acceptable salts thereof.

Thus, a preferred embodiment of this invention is directed to compounds of general formula **(II)**

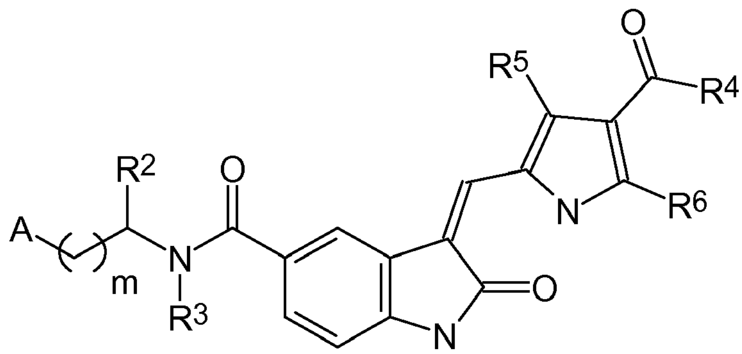

**(II)**

wherein A, m, and $R^2$-$R^6$ are defined as above and enantiomers, stereoisomeric forms, mixtures of enantiomers, mixtures of diastereomers, prodrugs, tautomers, hydrates, solvates and racemates of the above mentioned compounds and pharmaceutically acceptable salts thereof.

Preferred compounds of the present invention are compounds of general formula **(III)**,

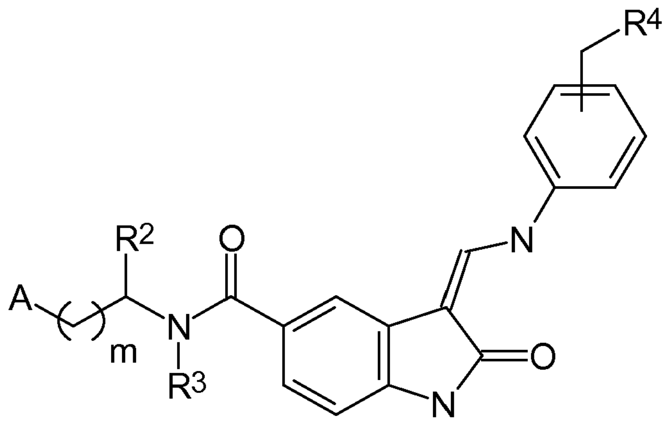

**(III)**

wherein A, m, $R^2$, $R^3$ and $R^4$ are defined as above and enantiomers, stereoisomeric forms, mixtures of enantiomers, mixtures of diastereomers, prodrugs, tautomers, hydrates, solvates and racemates of the above mentioned compounds and pharmaceutically acceptable salts thereof.

Another aspect of the present invention is related to compounds of general formula **(I)**

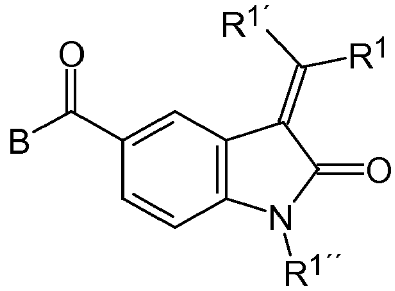

**(I)**

wherein both residues $R^1$ and $R^{1'}$ are different of -H and enantiomers, stereoisomeric forms, mixtures of enantiomers, mixtures of diastereomers, prodrugs, tautomers, hydrates, solvates and racemates of the above mentioned compounds and pharmaceutically acceptable salts thereof. Preferably, $R^{1'}$ is -Ph and $R^1$ is different from -Ph and -H.

Another preferred embodiment of the present invention is directed to compounds of general formula (I) wherein the residue $R^{1'}$ is selected from -Ph or -H and $R^1$ is selected from

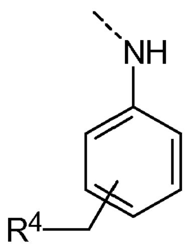

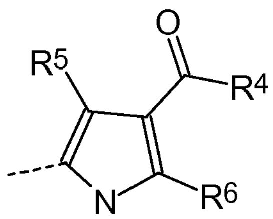

and

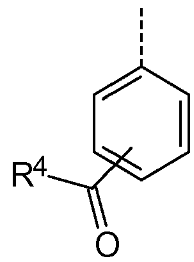

,

and more preferentially $R^1$ is

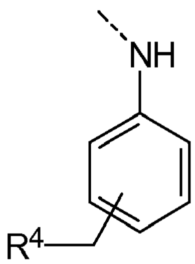

or

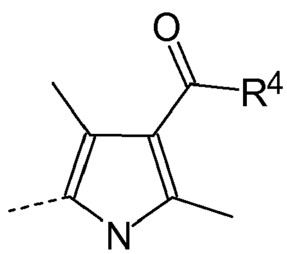

.

Yet preferred compounds, are compounds of genera formula (I), wherein $R^1$ is -H or -Ph, and $R^{1'}$ is selected from

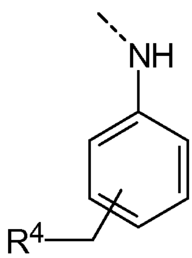

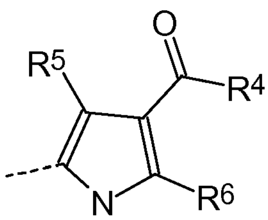

and

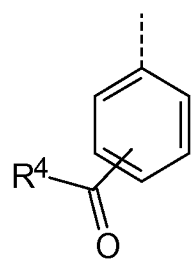

,

and more preferentially $R^{1'}$ is

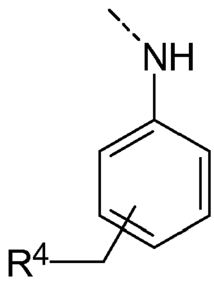

or

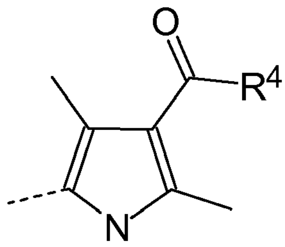

.

Preferably the residue A is:

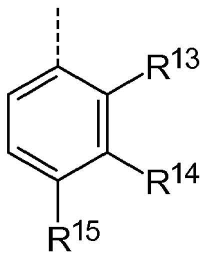

and m = 0; thus, preferred are compounds of general formula (I), **(II)** and **(III)** wherein A represents

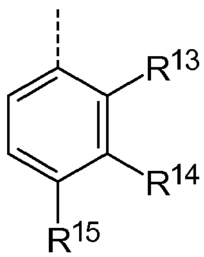

and m=0.

A preferred embodiment of the present application is directed to compounds of general formula (I), wherein B is $OR^{16}$ and $R^{16}$ is selected from: -H, $-CH_3$, $-C_2H_5$, $-C_3H_7$, $-C_4H_9$, and $-C_5H_{11}$. Preferentially, $R^{16}$ reperesents -H, $-CH_3$, $-C_2H_5$, and more preferentially $R^{16}$ reperesents -H.

Further preferred compounds of this application are compounds wherein $R^2$ is selected from: $-CH_3$ and $-C_2H_5$.

In yet another preferred embodiment of the present invention, the compound according to the general formula (I) is selected from the group of compounds depicted in the following Table 1.

**Table 1:**

| No. | Name |
|---|---|
| | (3Z)-3-{[3,5-dimethyl-4-(piperidin-1-ylcarbonyl)-1H-pyrrol-2-yl]methylene}-2-oxoindoline-5-carboxylic acid |
| 2 | ((3Z)-3-({3,5-dimethyl-4-[(2-pyrrolidin-1-ylethyl)carbamoyl]-1H-pyrrol-2-yl}methylene)-2-oxoindoline-5-carboxylic acid |
| 3 | (3Z)-3-[(4-{[2-(diethylamino)ethyl]carbamoyl}-3,5-dimethyl-1H-pyrrol-2-yl)methylene]-2-oxoindoline-5-carboxylic acid |
| 4 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxylic acid |
| 5 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxylic acid |
| 6 | (3Z)-3-({3,5-dimethyl-4-[(2-pyrrolidin-1-ylethyl)carbamoyl]-1H-pyrrol-2-yl}methylene)-2-oxo-N-[(1R)-1-phenylpropyl]indoline-5-carboxamide |

(continued)

| No. | Name |
|---|---|
| 7 | (3Z)-3-({3,5-dimethyl-4-[(4-methylpiperazin-1-yl)carbonyl]-1H-pyrrol-2-yl}methylene)-2-oxo-N-[(1R)-1-phenylpropyl]indoline-5-carboxamide |
| 8 | (3Z)-N-[1-(3-chlorophenyl)propyl]-3-({3,5-dimethyl-4-[(4-methylpiperazin-1-yl)carbonyl]-1H-pyrrol-2-yl}methylene)-2-oxoindoline-5-carboxamide |
| 9 | (3Z)-N-[1-(3,4-difluorophenyl)propyl]-3-({3,5-dimethyl-4-[(4-methylpiperazin-1-yl)carbonyl]-1H-pyrrol-2-yl}methylene)-2-oxoindoline-5-carboxamide |
| 10 | (3Z)-N-[1-(3,4-difluorophenyl)propyl]-3-({3,5-dimethyl-4-[(2-pyrrolidin-1-ylethyl)carbamoyl]-1H-pyrrol-2-yl}methylene)-2-oxoindoline-5-carboxamide |
| 11 | (3Z)-N-[1-(3-chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide |
| 12 | (3Z)-N-[1-(4-chlorophenyl)propyl]-3-({3,5-dimethyl-4-[(4-methylpiperazin-1-yl)carbonyl]-1H-pyrrol-2-yl}methylene)-2-oxoindoline-5-carboxamide |
| 13 | (3Z)-N-[1-(4-chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide |
| 14 | (3Z)-3-{[3,5-dimethyl-4-(piperidin-1-ylcarbonyl)-1H-pyrrol-2-yl]methylene}-2-oxo-N-[(1R)-1-phenylethyl]indoline-5-carboxamide |
| 15 | (3Z)-3-({3,5-dimethyl-4-[(4-methylpiperazin-1-yl)carbonyl]-1H-pyrrol-2-yl}methylene)-2-oxo-N-[(1R)-1-phenylethyl]indoline-5-carboxamide |
| 16 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1R)-1-phenylethyl]indoline-5-carboxamide |
| 17 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1R)-1-phenylethyl]indoline-5-carboxamide |
| 18 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1S)-1-phenylethyl]indoline-5-carboxamide |
| 19 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1S)-1-phenylethyl]indoline-5-carboxamide |
| 20 | (3Z)-3-({3,5-dimethyl-4-[(4-methylpiperazin-1-yl)carbonyl]-1H-pyrrol-2-yl}methylene)-N-[(1S)-1-(4-methoxyphenyl)ethyl]-2-oxoindoline-5-carboxamide |
| 21 | (3Z)-3-({3,5-dimethyl-4-[(4-methylpiperazin-1-yl)carbonyl]-1H-pyrrol-2-yl}methylene)-N-[(1R)-1-(4-methoxyphenyl)ethyl]-2-oxoindoline-5-carboxamide |
| 22 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(4-methoxyphenyl)ethyl]-2-oxoindoline-5-carboxamide |
| 23 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(3-methoxyphenyl)ethyl]-2-oxoindoline-5-carboxamide |
| 24 | (3Z)-N-[(1R)-1-cyclohexylethyl]-3-({3,5-dimethyl-4-[(4-methylpiperazin-1-yl)carbonyl]-1H-pyrrol-2-yl}methylene)-2-oxoindoline-5-carboxamide |
| 25 | (3Z)-N-[(1R)-1-(3-chlorophenyl)ethyl]-3-({3,5-dimethyl-4-[(4-methylpiperazin-1-yl)carbonyl]-1H-pyrrol-2-yl}methylene)-2-oxoindoline-5-carboxamide |
| 26 | (3Z)-N-[(1R)-1-(3-chlorophenyl)ethyl]-3-[(3,5-dimethyl-4-{[(2R)-2-(pyrrolidin-1-ylmethyl)pyrrolidin-1-yl]carbonyl}-1 H-pyrrol-2-yl)methylene]-2-oxoindoline-5-carboxamide |
| 27 | (3Z)-3-({3,5-dimethyl-4-[(4-methylpiperazin-1-yl)carbonyl]-1H-pyrrol-2-yl}methylene)-N-[(1R)-1-(2-naphthyl)ethyl]-2-oxoindoline-5-carboxamide |
| 28 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1R)-1-(2-naphthyl)ethyl]-2-oxoindoline-5-carboxamide |
| 29 | (32)-N-[(1S)-2,3-dihydro-1H-inden-1-yl]-3-[(3,5-dimethyl-4-{[(2R)-2-(pyrrolidin-1-ylmethyl)pyrrolidin-1-yl]carbonyl}-1 H-pyrrol-2-yl)methylene]-2-oxoindoline-5-carboxamide |
| 30 | (3Z)-N-[(1R)-2,3-dihydro-1H-inden-1-yl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide |
| 31 | (3Z)-3-({3,5-dimethyl-4-[(4-methylpiperazin-1-yl)carbonyl]-1H-pyrrol-2-yl}methylene)-N-methyl-2-oxo-N-[(1R)-1-phenylethyl]indoline-5-carboxamide |
| 32 | (3Z)-3-({3,5-dimethyl-4-[(4-methylpiperazin-1-yl)carbonyl]-1H-pyrrol-2-yl}methylene)-N-[(1R)-1-(4-methylphenyl)ethyl]-2-oxoindoline-5-carboxamide |

(continued)

| No. | Name |
|---|---|
| 33 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(4-methylphenyl)ethyl]-2-oxoindoline-5-carboxamide |
| 34 | (3Z)-3-({3,5-dimethyl-4-[(4-methylpiperazin-1-yl)carbonyl]-1H-pyrrol-2-yl}methylene)-N-[(1R)-1-(4-methylphenyl)ethyl]-2-oxoindoline-5-carboxamide |
| 35 | (3Z)-N-[(11R)-1-(4-chlorophenyl)ethyl]-3-({3,5-dimethyl-4-[(4-methylpiperazin-1-yl)carbonyl]-1H-pyrrol-2-yl}methylene)-2-oxoindoline-5-carboxamide |
| 36 | (32)-N-[(1S)-1-(4-chlorophenyl)ethyl]-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxamide |
| 37 | (3Z)-N-[(1R)-1-(4-chlorophenyl)ethyl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide |
| 38 | (3Z)-N-[(1R)-1-(3-chlorophenyl)ethyl]-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxamide |
| 39 | (3Z)-N-[(1R)-1-(3-bromophenyl)ethyl]-3-({3,5-dimethyl-4-[(4-methylpiperazin-1-yl)carbonyl]-1H-pyrrol-2-yl}methylene)-2-oxoindoline-5-carboxamide |
| 40 | (32)-N-[(S)-1-(3-chlorophenyl)ethyl]-3-({3,5-dimethyl-4-[(4-methylpiperazin-1-yl)carbonyl]-1H-pyrrol-2-yl}methylene)-2-oxoindoline-5-carboxamide |
| 41 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1S)-1-(4-fluorophenyl)ethyl]-2-oxoindoline-5-carboxamide |
| 42 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1S)-1-(4-fluorophenyl)ethyl]-2-oxoindoline-5-carboxamide |
| 43 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1R)-1-(4-fluorophenyl)ethyl]-2-oxoindoline-5-carboxamide |
| 44 | (3Z)-3-[(3,5-dimethyl-4-{[4-(4-methylpiperazin-1-yl)piperidin-1-yl]carbonyl}-1H-pyrrol-2-yl)methylene]-2-oxo-N-[(1R)-1-phenylethyl]indoline-5-carboxamide |
| 45 | (3Z)-3-({3,5-dimethyl-4-[(4-methylpiperazin-1-yl)carbonyl]-1H-pyrrol-2-yl}methylene)-2-oxo-N-[(1S)-1-phenylpropyl]indoline-5-carboxamide |
| 46 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1R)-1-phenylpropyl]indoline-5-carboxamide |
| 47 | (3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1S)-1-phenylpropyl]indoline-5-carboxamide |
| 48 | (3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1R)-1-phenylpropyl]indoline-5-carboxamide |
| 49 | (32)-N-[(1S)-1-(3-bromophenyl)ethyl]-3-({3,5-dimethyl-4-[(4-methylpiperazin-1-yl)carbonyl]-1H-pyrrol-2-yl}methylene)-2-oxoindoline-5-carboxamide |
| 50 | (3Z)-3-{[3,5-dimethyl-4-(pyrrolidin-1-ylcarbonyl)-1H-pyrrol-2-yl]methylene}-N-[(1S)-1-(4-methoxyphenyl)ethyl]-2-oxoindoline-5-carboxamide |
| 51 | (3Z)-3-({3,5-dimethyl-4-[(4-methylpiperazin-1-yl)carbonyl]-1H-pyrrol-2-yl}methylene)-N-[1-(3-methylphenyl)ethyl]-2-oxoindoline-5-carboxamide |
| 52 | (3Z)3-[(3,5-dimethyl-4-{[4-(4-methylpiperazin-1-yl)piperidin-1-yl]carbonyl}-1H-pyrrol-2-yl)methylene]-2-oxo-N-[(1S)-1-phenylpropyl]indoline-5-carboxamide |
| 53 | (3Z)-3-[(3,5-dimethyl-4-{[4-(4-methylpiperazin-1-yl)piperidin-1-yl]carbonyl}-1H-pyrrol-2-yl)methylene]-2-oxo-N-[(1R)-1-phenylpropyl]indoline-5-carboxamide |
| 54 | (3Z)-3-{[3,5-dimethyl-4-(piperidin-1-ylcarbonyl)-1H-pyrrol-2-yl]methylene}-2-oxo-N-[(1S)-1-phenylethyl]indoline-5-carboxamide |
| 55 | (3Z)-N-[(1R)-1-(3-chlorophenyl)ethyl]-3-{[3,5-dimethyl-4-(piperidin-1-ylcarbonyl)-1H-pyrrol-2-yl]methylene}-2-oxoindoline-5-carboxamide |
| 56 | (3Z)-N-[(1S)-1-(3-chlorophenyl)ethyl]-3-{[3,5-dimethyl-4-(piperidin-1-ylcarbonyl)-1H-pyrrol-2-yl]methylene}-2-oxoindoline-5-carboxamide |
| 57 | (3Z)-3-{[3,5-dimethyl-4-(piperidin-1-ylcarbonyl)-1H-pyrrol-2-yl]methylene}-2-oxo-N-[(1S)-1-phenylpropyl]indoline-5-carboxamide |
| 58 | (3Z)-3-{4-[(4-methylpiperazin-1-yl)carbonyl]benzylidene}-2-oxo-N-[(1S)-1-phenylpropyl]indoline-5-carboxamide |

(continued)

| No. | Name |
|---|---|
| 59 | (3Z)-3-[(4-{[2-(diethylamino)ethyl]carbamoyl}-3,5-dimethyl-1H-pyrrol-2-yl)methylene]-2-oxo-N-[(1R)-1-phenylethyl]indoline-5-carboxamide |
| 60 | (3Z)-3-[(4-{[2-(diethylamino)ethyl]carbamoyl}-3,5-dimethyl-1H-pyrrol-2-yl)methylene]-2-oxo-N-[(1S)-1-phenylethyl]indoline-5-carboxamide |
| 61 | (3Z)-3-[(4-{[2-(diethylamino)ethyl]carbamoyl}-3,5-dimethyl-1H-pyrrol-2-yl)methylene]-2-oxo-N-[(1R)-1-phenylpropyl]indoline-5-carboxamide |
| 62 | (3Z)-3-[(4-{[2-(diethylamino)ethyl]carbamoyl}-3,5-dimethyl-1H-pyrrol-2-yl)methylene]-2-oxo-N-[(1S)-1-phenylpropyl]indoline-5-carboxamide |
| 63 | (3Z)-3-{[3,5-dimethyl-4-(piperidin-1-ylcarbonyl)-1H-pyrrol-2-yl]methylene}-2-oxo-N-[(1R)-1-phenylpropyl]indoline-5-carboxamide |
| 64 | (3Z)-3-{[3,5-dimethyl-4-(piperidin-1-ylcarbonyl)-1H-pyrrol-2-yl]methylene}-N-[(1S)-1-(4-fluorophenyl)ethyl]-2-oxoindoline-5-carboxamide |
| 65 | (3Z)-3-{[3,5-dimethyl-4-(piperidin-1-ylcarbonyl)-1H-pyrrol-2-yl]methylene}-N-[(1S)-1-(4-methoxyphenyl)ethyl]-2-oxoindoline-5-carboxamide |
| 66 | (3Z)-N-[(1R)-1-(4-chlorophenyl)ethyl]-3-{[3,5-dimethyl-4-(piperidin-1-ylcarbonyl)-1H-pyrrol-2-yl]methylene}-2-oxoindoline-5-carboxamide |
| 67 | (3Z)-N-[(1R)-1-(4-chlorophenyl)ethyl]-3-{[3,5-dimethyl-4-(pyrrolidin-1-ylcarbonyl)-1H-pyrrol-2-yl]methylene}-2-oxoindoline-5-carboxamide |
| 68 | (3Z)-2-oxo-3-({[4-(piperidin-1-ylmethyl)phenyl]amino}methylene)indoline-5-carboxylic acid |
| 69 | (3Z)-2-oxo-N-[(1R)-1-phenylpropyl]-3-({[4-(piperidin-1-ylmethyl)phenyl]amino}methylene)- indoline-5-carboxamide |
| 70 | (3Z)-N-[(1R)-1-(4-methoxyphenyl)ethyl]-2-oxo-3-({[4-(piperidin-1-ylmethyl)phenyl]amino}methylene)indoline-5-carboxamide |
| 71 | (3Z)-N-methyl-2-oxo-N-[(1S)-1-phenylethyl]-3-({[4-(piperidin-1-ylmethyl)phenyl]amino}methylene)indoline-5-carboxamide |
| 72 | (3Z)-N-[(1R)-1-(4-methylphenyl)ethyl]-2-oxo-3-({[4-(piperidin-1-ylmethyl)phenyl]amino}methylene)indoline-5-carboxamide |
| 73 | (3Z)-N-[(1R)-1-(4-chlorophenyl)ethyl]-2-oxo-3-({[4-(piperidin-1-ylmethyl)phenyl]amino}methylene)indoline-5-carboxamide |
| 74 | (3Z)-2-oxo-N-[(1S)-1-phenylpropyl]-3-({[4-(piperidin-1-ylmethyl)phenyl]amino}methylene)indoline-5-carboxamide |
| 75 | (3Z)-2-oxo-N-[(1S)-1-phenylethyl]-3-({[4-(piperidin-1-ylmethyl)phenyl]amino}methylene)indoline-5-carboxamide |
| 76 | (3Z)-2-oxo-3-({[4-(piperidin-1-ylmethyl)phenyl]aminolmethylene)-N-[(1S)-1,2,3,4-tetrahydronaphthalen-1-yl]indoline-5-carboxamide |
| 77 | (3Z)-N-[(1R)-1-cyclohexylethyl]-2-oxo-3-({[4-(piperidin-1-ylmethyl)phenyl]amino}methylene)indoline-5-carboxamide |
| 78 | (3Z)-2-oxo-N-[(1R)-1-phenylethyl]-3-({[4-(piperidin-1-ylmethyl)phenyl] amino}methylene)indoline-5-carboxamide |
| 79 | (3Z)-N-[(1R)-1-(3-methoxyphenyl)ethyl]-2-oxo-3-({[4-(piperidin-1-ylmethyl)phenyl]amino}methylene)indoline-5-carboxamide |
| 80 | (3Z)-N-[(1R)-1-(3-chlorophenyl)ethyl]-2-oxo-3-({[4-(piperidin-1-ylmethyl)phenyl]amino}methylene)indoline-5-carboxamide |
| 81 | (3Z)-2-oxo-N-[(1R)-1-phenylethyl]-3-(phenyl{[3-(piperidin-1-ylmethyl)phenyl]amino}methylene)indoline-5-carboxamide |
| 82 | (3Z)-N-[(1S)-1-(4-fluorophenyl)ethyl]-2-oxo-3-(phenyl{[3-(piperidin-1-ylmethyl)phenyl]amino}methylene)indoline-5-carboxamide |
| 83 | (3Z)-N-[(1R)-1-(4-fluorophenyl)ethyl]-2-oxo-3-(phenyl{[3-(piperidin-1-ylmethyl)phenyl]amino}methylene)indoline-5-carboxamide |
| 84 | (3Z)-2-oxo-3-(phenyl{[3-(piperidin-1-ylmethyl)phenyl]amino}methylene)-N-[(1 R)-1-phenylpropyl]indoline-5-carboxamide |

(continued)

| No. | Name |
|---|---|
| 85 | (3Z)-N-[(1S)-1-(4-chlorophenyl)ethyl]-2-oxo-3-(phenyl{[3-(piperidin-1-ylmethyl)phenyl]amino}methylene)indoline-5-carboxamide |
| 86 | (3Z)-N-[(1R)-1-(4-chlorophenyl)ethyl]-2-oxo-3-(phenyl{[3-(piperidin-1-ylmethyl)phenyl]amino}methylene)indoline-5-carboxamide |
| 87 | (3Z)-2-oxo-N-[(1S)-1-phenylethyl]-3-(phenyl{[3-(piperidin-1-ylmethyl)phenyl]amino}methylene)indoline-5-carboxamide |
| 88 | (3Z)-2-oxo-N-[(1R)-1-phenylethyl]-3-(phenyl{[3-(piperidin-1-ylmethyl)phenyl]amino}methylene)indoline-5-carboxamide |
| 89 | (3Z)-2-oxo-3-(phenyl{[3-(piperidin-1-ylmethyl)phenyl]amino}methylene)-N-[(1 S)-1-phenylpropyl]indoline-5-carboxamide |
| 90 | (3Z)-2-oxo-3-(phenyl{[3-(piperidin-1-ylmethyl)phenyl]amino}methylene)-N-[(1 R)-1-phenylpropyl]indoline-5-carboxamide |
| 91 | (3Z)-N-[(1R)-1-(4-methoxyphenyl)ethyl]-2-oxo-3-({[4-(piperidin-1-ylmethyl)phenyl]amino}methylene)indoline-5-carboxamide |
| 92 | (3Z)-N-[(1S)-1-(4-fluorophenyl)ethyl]-2-oxo-3-({[4-(piperidin-1-ylmethyl)phenyl]amino}methylene)indoline-5-carboxamide |
| 93 | (3Z)-N-[(1R)-1-(3-methoxyphenyl)ethyl]-2-oxo-3-({[4-(piperidin-1-ylmethyl)phenyl]amino}methylene)indoline-5-carboxamide |
| 94 | (3Z)-N-[(1R)-1-(4-fluorophenyl)ethyl]-2-oxo-3-({[4-(piperidin-1-ylmethyl)phenyl]amino}methylene)indoline-5-carboxamide |
| 95 | (3Z)-N-[(1R)-1-(3-chlorophenyl)ethyl]-2-oxo-3-({[4-(piperidin-1-ylmethyl)phenyl]amino}methylene)indoline-5-carboxamide |
| 96 | (3Z)-3-({[3-(morpholin-4-ylmethyl)phenyl]amino}methylene)-2-oxoindoline-5-carboxylic acid |
| 97 | (3Z)-3-({[3-(morpholin-4-ylmethyl)phenyl]amino}methylene)-2-oxo-N-[(1R)-1-phenylpropyl]indoline-5-carboxamide |
| 98 | (3Z)-3-({[3-(morpholin-4-ylmethyl)phenyl]amino}methylene)-2-oxo-N-[(1S)-1-phenylpropyl]indoline-5-carboxamide |
| 99 | (3Z)-3-({[3-(morpholin-4-ylmethyl)phenyl]amino}methylene)-2-oxo-N-[(1S)-1-phenylethyl]indoline-5-carboxamide |
| 100 | (3Z)-3-({[3-(morpholin-4-ylmethyl)phenyl]amino}methylene)-2-oxo-N-[(1R)-1-phenylethyl]indoline-5-carboxamide |
| 101 | (3Z)-N-[(1R)-1-(4-fluorophenyl)ethyl]-3-({[3-(morpholin-4-ylmethyl)phenyl]amino}methylene)-2-oxoindoline-5-carboxamide |
| 102 | (3Z)-N-[(1S)-1-(4-fluorophenyl)ethyl]-3-({[3-(morpholin-4-ylmethyl)phenyl]amino}methylene)-2-oxoindoline-5-carboxamide |
| 103 | (3Z)-N-[(1S)-1-(4-methoxyphenyl)ethyl]-3-({[3-(morpholin-4-ylmethyl)phenyl]amino}methylene)-2-oxoindoline-5-carboxamide |
| 104 | 3Z)-N-[(1R)-1-(4-methoxyphenyl)ethyl]-3-({[3-(morpholin-4-ylmethyl)phenyl]amino}methylene)-2-oxoindoline-5-carboxamide |
| 105 | (3Z)-2-oxo-3-({[4-(piperidin-1-ylmethyl)phenyl]amino}methylene)indoline-6-carboxylic acid |
| 106 | (3Z)-2-oxo-N-[(1R)-1-phenylethyl]-3-({[4-(piperidin-1-ylmethyl)phenyl]amino}methylene)indoline-6-carboxamide |
| 107 | (3Z)-2-oxo-N-[(1S)-1-phenylethyl]-3-({[4-(piperidin-1-ylmethyl)phenyl]amino}methylene)indoline-6-carboxamide |
| 108 | (3Z)-2-oxo-N-[(1S)-1-phenylpropyl]-3-({[4-(piperidin-1-ylmethyl)phenyl]amino}methylene)indoline-6-carboxamide |
| 109 | (3Z)-2-oxo-N-[(1R)-1-phenylpropyl]-3-({[4-(piperidin-1-ylmethyl)phenyl]amino}methylene)indoline-6-carboxamide |
| 110 | (3Z)-N-[(1R)-1-(4-fluorophenyl)ethyl]-2-oxo-3-({[4-(piperidin-1-ylmethyl)phenyl]amino}methylene)indoline-6-carboxamide |
| 111 | (3Z)-N-[(1S)-1-(4-fluorophenyl)ethyl]-2-oxo-3-({[4-(piperidin-1-ylmethyl)phenyl]amino}methylene)indoline-6-carboxamide |

(continued)

| No. | Name |
|---|---|
| 112 | (3Z)-N-[(1S)-1-(3-methoxyphenyl)ethyl]-2-oxo-3-({[4-(piperidin-1-ylmethyl)phenyl]amino}methylene)indoline-6-carboxamide |
| 113 | (3Z)-N-[(1R)-1-(3-methoxyphenyl)ethyl]-2-oxo-3-({[4-(piperidin-1-ylmethyl)phenyl]amino}methylene)indoline-6-carboxamide |
| 114 | (3Z)-3-({3,5-dimethyl-4-[(2-pyrrolidin-1-ylethyl)carbamoyl]-1H-pyrrol-2-yl}methylene)-2-oxo-N-[(1R)-1-phenylethyl]indoline-5-carboxamide |
| 115 | (3Z)-3-({3,5-dimethyl-4-[(2-pyrrolidin-1-ylethyl)carbamoyl]-1H-pyrrol-2-yl}methylene)-2-oxo-N-[(1S)-1-phenylethyl]indoline-5-carboxamide |
| 116 | (3Z)-3-({3,5-dimethyl-4-[(2-pyrrolidin-1-ylethyl)carbamoyl]-1H-pyrrol-2-yl}methylene)-N-[(1S)-1-(4-fluorophenyl)ethyl]-2-oxoindoline-5-carboxamide |
| 117 | (3Z)-3-({3,5-dimethyl-4-[(2-pyrrolidin-1-ylethyl)carbamoyl]-1H-pyrrol-2-yl}methylene)-N-[(1R)-1-(4-fluorophenyl)ethyl]-2-oxoindoline-5-carboxamide |
| 118 | (3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-5-carboxamide |
| 119 | (3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-5-carboxamid |
| 120 | (3Z)-3-({3,5-dimethyl-4-[(2-pyrrolidin-1-ylethyl)carbamoyl]-1H-pyrrol-2-yl}methylene)-N-[(1S)-1-(3-methoxyphenyl)ethyl]-2-oxoindoline-5-carboxamide |
| 121 | (3Z)-3-({3,5-dimethyl-4-[(2-pyrrolidin-1-ylethyl)carbamoyl]-1H-pyrrol-2-yl}methylene)-N-[(1R)-1-(3-methoxyphenyl)ethyl]-2-oxoindoline-5-carboxamide |
| 122 | (3Z)-3-({3,5-dimethyl-4-[(2-pyrrolidin-1-ylethyl)carbamoyl]-1H-pyrrol-2-yl}methylene)-2-oxo-N-[(1R)-1-phenylethyl]indoline-6-carboxamide |
| 123 | (3Z)-3-({3,5-dimethyl-4-[(2-pyrrolidin-1-ylethyl)carbamoyl]-1H-pyrrol-2-yl}methylene)-2-oxo-N-[(1S)-1-phenylethyl]indoline-6-carboxamide |
| 124 | (3Z)-3-({3,5-dimethyl-4-[(2-pyrrolidin-1-ylethyl)carbamoyl]-1H-pyrrol-2-yl}methylene)-2-oxo-N-[(1S)-1-phenylpropyl]indoline-6-carboxamide |
| 125 | (3Z)-3-({3,5-dimethyl-4-[(2-pyrrolidin-1-ylethyl)carbamoyl]-1H-pyrrol-2-yl}methylene)-2-oxo-N-[(1R)-1-phenylpropyl]indoline-6-carboxamide |
| 126 | (3Z)-3-({3,5-dimethyl-4-[(2-pyrrolidin-1-ylethyl)carbamoyl]-1H-pyrrol-2-yl}methylene)-N-[(1R)-1-(4-fluorophenyl)ethyl]-2-oxoindoline-6-carboxamide |
| 127 | (3Z)-3-({3,5-dimethyl-4-[(2-pyrrolidin-1-ylethyl)carbamoyl]-1H-pyrrol-2-yl}methylene)-N-[(1S)-1-(4-fluorophenyl)ethyl]-2-oxoindoline-6-carboxamide |
| 128 | (3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-6-carboxamide |
| 129 | (3Z)-3-({3,5-dimethyl-4-[(2-pyrrolidin-1-ylethyl)carbamoyl]-1H-pyrrol-2-yl}methylene)-N-[(1R)-1-(4-methoxyphenyl)ethyl]-2-oxoindoline-6-carboxamide |
| 130 | (3Z)-3-({3,5-dimethyl-4-[(1-methylpiperidin-4-yl)carbamoyl]-1H-pyrrol-2-yl}methylene)-2-oxo-N-[(1R)-1-phenylpropyl]indoline-5-carboxamide |
| 131 | (3Z)-3-{[3,5-dimethyl-4-(piperidin-4-ylcarbamoyl)-1H-pyrrol-2-yl]methylene}-2-oxo-N-[(1R)-1-phenylpropyl]indoline-5-carboxamide |
| 132 | (3Z)-3-[(3,5-dimethyl-4-{[(3S)-1-methylpiperidin-3-yl]carbamoyl}-1H-pyrrol-2-yl)methylene]-2-oxo-N-[(1R)-1-phenylpropyl]indoline-5-carboxamide |
| 133 | (3Z)-3-{[4-({(2S)-2-[(diethylamino)methyl]pyrrolidin-1-yl}carbonyl)-3,5-dimethyl-1 H-pyrrol-2-yl]methylene}-2-oxo-N-[(1R)-1-phenylpropyl]indoline-5-carboxamide |
| 134 | (3Z)-3-{[4-({(2S)-2-[(dimethylamino)methyl]pyrrolidin-1-yl}carbonyl)-3,5-dimethyl-1 H-pyrrol-2-yl]methylene}-2-oxo-N-[(1R)-1-phenylpropyl]indoline-5-carboxamide |
| 135 | (3Z)-3-({3,5-dimethyl-4-[(3S)-piperidin-3-ylcarbamoyl]-1H-pyrrol-2-yl}methylene)-2-oxo-N-[(1R)-1-phenylpropyl]indoline-5-carboxamide |
| 136 | (3Z)-3-{[4-({(2R)-2-[(diethylamino)methyl]pyrrolidin-1-yl}carbonyl)-3,5-dimethyl-1 H-pyrrol-2-yl]methylene}-2-oxo-N-[(1R)-1-phenylpropyl]indoline-5-carboxamide |
| 137 | (3Z)-3-({3,5-dimethyl-4-[(2-piperidin-1-ylethyl)carbamoyl]-1H-pyrrol-2-yl}methylene)-2-oxo-N-[(1R)-1-phenylpropyl]indoline-5-carboxamide |

(continued)

| No. | Name |
|---|---|
| 138 | (3Z)-3-[(3,5-dimethyl-4-{[2-(4-methylpiperazin-1-yl)ethyl]carbamoyl}-1H-pyrrol-2-yl)methylene]-2-oxo-N-[(R)-1-phenylpropyl]indoline-5-carboxamide |
| 139 | (3Z)-3-({3,5-dimethyl-4-[(2-morpholin-4-ylethyl)carbamoyl]-1H-pyrrol-2-yl}methylene)-2-oxo-N-[(1R)-1-phenylpropyl]indoline-5-carboxamide |

## Chemical synthesis

The inventive compound **(I)** according to the present invention can be prepared by methods known to one skilled in the art. The synthesis is preferably carried out according to the general synthetic routes, shown in schemes 1 and 2.

The compounds of general formula **(I),** wherein $R^1$ is a pyrrole derivative and $R^{1'}$ and $R^{1''}$ are hydrogen can be accessed starting from pyrrole 2-carboxaldehyde **3*,** 2-oxoindoline-5-carboxylic acid **2*** and optionally amine **1*** *via* a two steps procedure involving aldol reaction and amide bond formation or esterification. Thus, compounds of general formula **(I)** with $R^1$ is a pyrrole derivative and the B moiety connected *via* an amide bond can be synthesized either from precursor **4*** though aldol reaction, or from carboxylic acid **5*** *via* amide bond formation. Thus, the amide bond can be constructed either at the beginning of the synthesis by reacting commercially available carboxylic acid **2*** with amines **1*,** or at the end of the synthesis by reacting carboxylic acid **5*** with amine **1*.** Conveniently, many amines of general formula **1*** are commercially available or can be easily prepared by a person skilled in the art following known synthetic procedures that are disclosed in patent or non-patent literature. Amide bond formation involves activation of the carboxylic group, followed by nucleophile attack of the amino group. Activation of the carboxylic acid includes conversion of the carboxylic acid to the corresponding active ester, carboxyl chloride or bromide, or anhydride. For facility reasons, the activation of the carboxylic acid performed *in situ* by treatment of the mixture of carboxylic acid and amine with an activating agent. Activating agents include carbodiimides such as DCC (*N,N'*-dicyclohexylcarbodiimide), DIC (*N,N'*-diisopropylcarbodiimide) and EDCI (N-(3-dimethylaminopropyl)-N'-ethylcarbodiimide hydrochloride), phosphonium salts such as BOP (benzotriazol-1-yloxy)tris(dimethylamino)phosphonium hexafluorophosphate, PyBOP (benzotriazol-1-yloxy)tripyrrolidinophosphonium hexafluorophosphate, PyAOP ((7-azabenzotriazol-1-yloxy)tripyrrolidinophosphonium hexafluorophosphate), BroP (bromotris(dimethylamino)phosphonium hexafluorophosphate), PyCloP (chlorotripyrrolidinophosphonium hexafluorophosphate), DEPBT (3-(diethoxyphosphoryloxy)-1,2,3-benzotriazin-4(3H)-one), uronium and guanidinium salts such as HBTU (O-(benzotriazol-1-yl)-N,N,N',N'-tetramethyluronium hexafluorophosphate), TBTU (O-(Benzotriazol-1-yl)-N,N,N',N'-tetramethyluronium tetrafluoroborate), HATU (O-(7-azabenzotriazol-1-yl)-N,N,N',N'-tetramethyluronium hexafluorophosphate), HBPyU (O-(benzotriazol-1-yl)-N,N,N',N'-bis(tetramethylene)uranium hexafluorophosphate), HCTU (O-(6-chlorobenzotriazol-1-yl)-N,N,N',N'- 8 tetramethyluronium hexafluorophosphate). Amide bond formation is conducted preferably in presence of a base, such as tertiary amines including TEA (triethylamine) and (DIPEA) diisopropylethyl amine. Thus, the amide bond formation can be carried out using reagent systems such as HATU/DIPEA, HBTU/TEA or EDCI/HOBT/TEA.

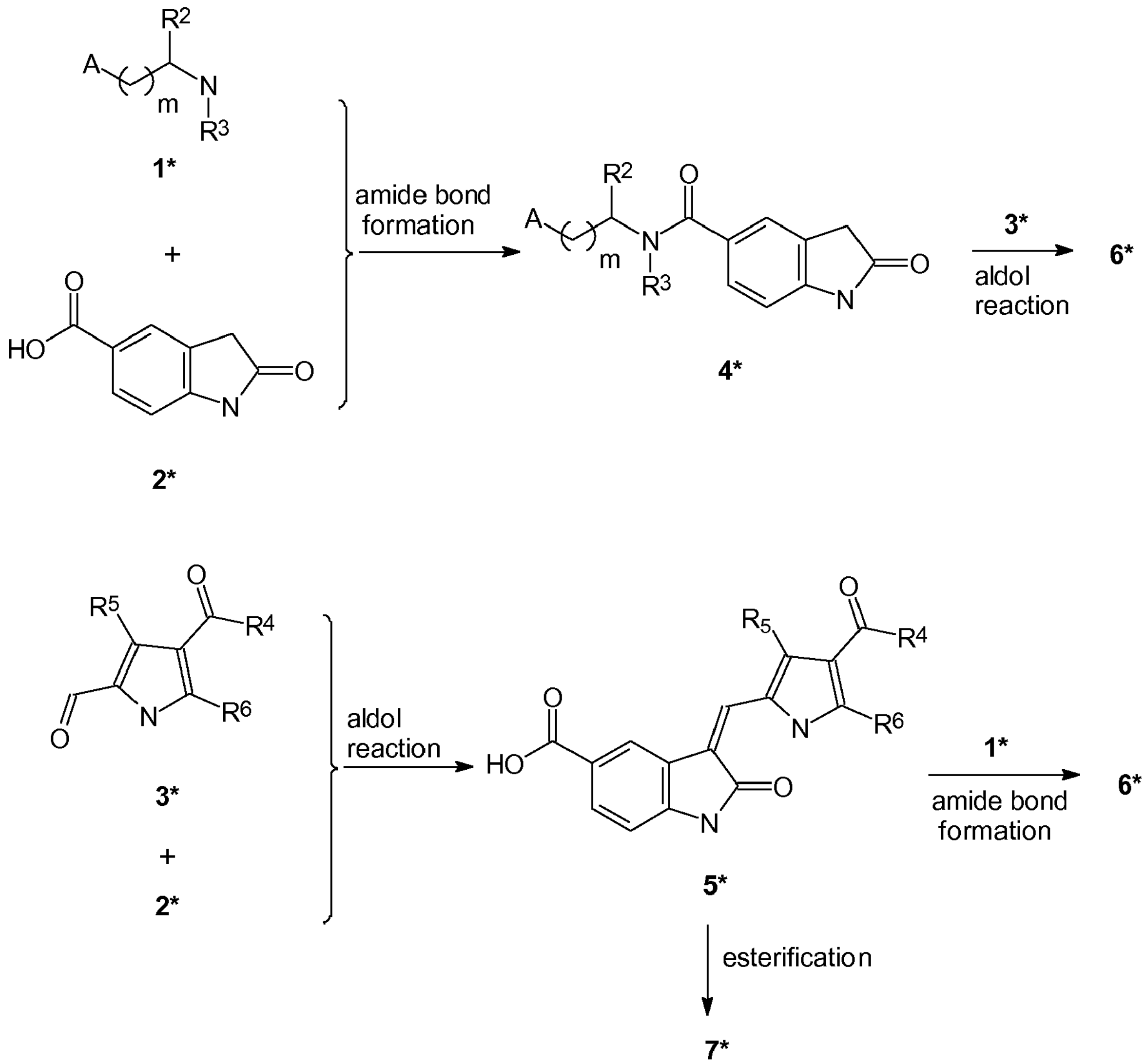


**Scheme 1**: Synthesis of the pyrrole derivatives.

The aldol reaction allows the introduction of the pyrrole moiety on the molecule and the formation of α,β-unsaturated compounds **6*** and **5*.** As in the case of the amide bond formation, the aldol reaction can be performed at the end of the synthesis by reaction of intermediate **4*** with pyrrole 2-carboxaldehyde **3*** or at beginning of the synthesis by reaction of pyrrole 2-carboxaldehyde **3*** with 2-oxoindoline-5-carboxylic acid **2*.** The aldol reaction is conducted by heating the reaction mixture containing aldehyde **3*** and ketone **4*** or **2*** in presence of a base, such as piperidine. Conveniently, many pyrrole 2-carboxaldehyde of general formula **3*** are commercially available or can be easily prepared by a person skilled in the art following known synthetic procedures that are disclosed in patent or non-patent literature Aldol reaction of compounds **2*** and **4*** provides compounds of general formula **(I)** with $R^1$ being a pyrrole derivative and the B moiety connected *via* an amide bond, while aldol reaction of compounds **2*** and **3*** furnishes compounds of general formula **(I)** with $R^1$ being a pyrrole derivative and the B moiety beings a hydroxyl group i.e. B moiety is $OR^{16}$, where $R^{16}$ is hydrogen. Such compounds can be further submitted to esterification to give compounds of general formula **(I)** with $R^1$ being a pyrrole derivative and the B moiety being $OR^{16}$, wherein $R^{16}$ is different of hydrogen, or can be submitted to amide bond formation as previously described to furnish compounds of general formula **(I)** with $R^1$ being a pyrrole derivative and the B moiety being connected *via* an amide bond. Esterification can be performed by simple treatment of the carboxylic acid with a suitable alcohol in presence of a catalytic amount of acid. Suitable alcohols include methanol, ethanol, *n*-propanol, *iso*-propanol, *n*-butanol, *iso*-butanol, *tert*-butanol, 1-pentanol, 2-pentanol, 3-pentanol, 2-methyl-1-butanol, 3-methyl-1-butanol, 3-methyl-2-butanol, 2-methyl-2-butanol, 2,2-dimethyl-1-propanol.

The compounds of general formula **(I),** wherein $R^1$ or $R^{1'}$ is an aniline derivative and $R^{1''}$ is hydrogen or phenyl can be obtained from the same commercially available substrate: 2-oxoindoline-5-carboxylic acid **2*.** First, oxoindoline **2*** is reacted with $ZC(OT)_3$ (Z = H or Ph; T = Me or Et) at reflux in presence of acetic anhydride to provide α,β-unsaturated

vinyl ethers **8*a, 8*b, 8*c** and **8*d.** Then, the vinyl ethers **8*a, 8*b, 8*c** and **8*d** are treated with aniline derivatives of general formula **9*** to append the aniline moiety on the molecule. Many amines of general formula **9*** are commercially available or can be easily prepared by a person skilled in the art following known synthetic procedures that are disclosed in patent or non-patent literature. Subsequent treatment with NaOMe/MeOH or $NH_3$/MeOH results in cleavage of the acetamide and provides intermediates **10*a, 10*b, 10*c** and 10*d corresponding to the compounds of general formula **(I),** wherein $R^1$ or $R^{1'}$ is an aniline derivative and B is a hydroxyl group. As previously, intermediates **10*a, 10*b, 10*c** and **10*d** can be involved in amide bond formation to afford compounds of general formula **(I),** wherein $R^1$or $R^{1'}$ is an aniline derivative and B is connected *via* an amide bond, or can be submitted to esterification to give compounds of general formula **(I),** wherein $R^1$ or $R^{1'}$ is a an aniline derivative and B is a $OR^{16}$, with $R^{16}$ different of hydrogen.

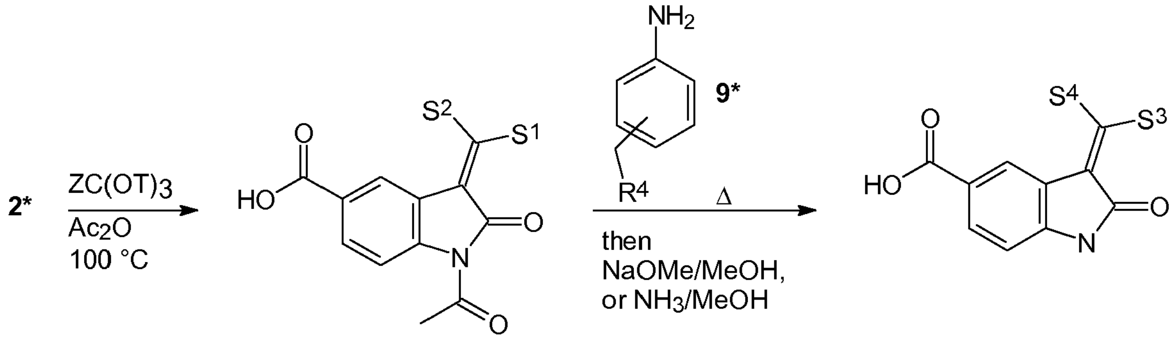


**8*a** S1 = OT, S2 = H
**8*b** S1 = H, S2 = OT
**8*c** S1 = OT, S2 = Ph
**8*d** S1 = Ph, S2 = OT

**10*a** S3 = S, S4 = H
**10*b** S4 = H, S4 = S
**10*c** S3 = S, S4 = Ph
**10*d** S3 = Ph, S4 = S

Z = H or Ph
T = Me or Et

S =

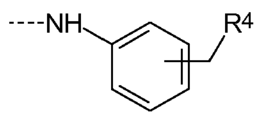

**Scheme 2**: Synthesis of the aniline derivatives.

**Indications**

In a further aspect of the present invention, the novel compounds according to the general formula (I) are used as pharmaceutically active agent applicable in medicine.

Surprisingly it was found that the above-mentioned compounds of general formula **(I),** as well as the pharmaceutical compositions comprising said compounds of general formula **(I)** are acting as protein kinases inhibitors and more specifically as inhibitor of GRK5 kinase and are useful for the treatment and prophylaxis of metabolic diseases.

The term "protein kinase inhibitors" refers to compounds that are acting as enzyme inhibitors, which specifically block the action of one or more protein kinases. The term "enzyme inhibitor" refers to compounds, which bind to enzymes and decrease their activity. The binding of an inhibitor can stop a substrate from entering the enzymes active site, compete with the substrate for the binding site, or hinder the enzyme from catalyzing its reaction. Inhibitor binding can be reversible or irreversible.

GRK5 as used herein refers to the protein having accession number P34947 with UniProtKB/Swiss-Prot. Alternatives names of this protein are: G protein-coupled receptor kinase 5, EC = 2.7.11.16 and G protein-coupled receptor kinase GRK5.G protein-coupled receptor kinase 5. As GRK5 modulates the insulin release in a significant manner (WO 2012/028335 A1), it became a preferred target for the therapy of metabolic diseases, preferably for the therapy of diabetes, obesity and impaired adipogenesis. Thus in a preferred embodiment of this invention, the compounds of

general formula **(I)** and/or pharmaceutical acceptable salts thereof are useful for or can be used for the treatment or prophylaxis of diabetes, obesity and impaired adipogenesis.

Furthermore, it was found that the inhibition of GRK5 is correlated to a significant increase in insulin release. Consequently, GRK5 represents a preferable target for the therapy of diabetes mellitus type 2. Thus, another aspect of the present invention is directed to the use of compound of general formula **(I)** and/or pharmaceutically acceptable salts thereof for the treatment or prophylaxis of diabetes mellitus type 2.

Moreover the compounds of the present application influence the glucose dependent regulation of insulin production and/or release and/or the increase of glucose uptake. Therefore, another aspect of the present invention is directed to the use of compounds of general formula **(I)** for glucose dependent regulation of insulin production and/or release of insulin, as well as for increasing glucose uptake.

Furthermore, the compounds of the present application can be used for the treatment or prophylaxis of cancer, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

Another preferred embodiment of the present application refers to pharmaceutical composition comprising at least one compound described herein together with at least one pharmaceutically acceptable carrier, solvent or excipient. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as

acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must

be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

### <u>Part A: Chemical Syntheis</u>

### General chemical procedures

All reagents were commercial grade and were used as received without further purification, unless otherwise specified. Reagent grade solvents were used in all other cases, unless otherwise specified, respectively. Silica gel generally used for column chromatography was Fisher silica gel (60A, 35-70 micron). Thin layer chromatography was carried out using pre-coated silica gel F-254 plates (thickness 0.25 mm). $^{1}$H-NMR spectra were recorded on a Bruker AM-300 (300 MHz) spectrometer. The chemical shifts are expressed in ppm using the residual solvent as internal standard. Splitting patterns are designated as s (singlet), d (doublet), dd (double-doublet), t (triplet), q (quartet), quint (quintet), m (multiplet), and bs (broad singlet). Electrospray MS spectra were obtained on a Waters LC-MS system Acquity SQD detector, Waters 2795 Alliance HPLC equipped with Waters 996 PDA Detector (HPLC column: Waters XBridge, RP C18, 3.5 $\mu$m, 4.6 mm x 50 mm; gradient MeCN/H2O, containing 0.1% HCOOH: 5% MeCN (0.5 min), 5% to 95% MeCN (5 min), 95% MeCN (0.5 min); flow rate: 2.0 ml/min.

**<u>Example 1:</u>** (3Z)-3-[(4-{[2-(diethylamino)ethyl]carbamoyl}-3,5-dimethyl-1H-pyrrol-2-yl)methylene]-2-oxoindoline-5-carboxylic acid (**1**)

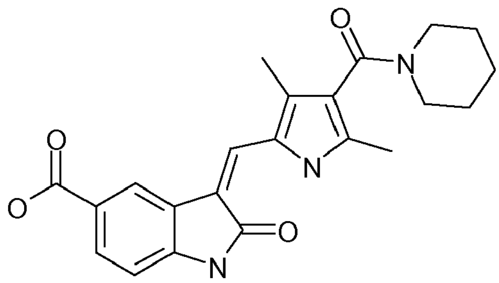

**1**

A mixture of 2-oxoindoline-5-carboxylic acid (0.708 g, 4 mmol), 3,5-dimethyl-4-(piperidin-1-ylcarbonyl)-1*H*-pyrrole-2-carbaldehyde (1.030 g, 4.4 mmol) and piperidine (0.2 mL) in EtOH (75 mL) was refluxed for 8 h. The mixture was cooled down to room temperature to provide a precipitate which was filtered and washed with EtOH to provide the target compound 1 (0.980 g, 62.25%)
NMR (300 MHz, DMSO-$d_6$) δ ppm: 13.52 (s,1H), 12.5 (bs,1H), 11.19 (s,1H), 8.37 (s,1H), 7.78 (s,1H), 7.77 (d,1H), 6.96 (d,1H), 3.43 (m,4H), 2.29 (s,6H), 1.60 (bs,2H), 1.47 (bs,4H);
$t_R$: 3.18 min, MS (ESI): *m/z* $(M+H)^+$ 394; $(M+H)^-$392.

The following compounds were prepared according to the procedure described by **example 1.**

((3Z)-3-({3,5-Dimethyl-4-[(2-pyrrolidin-1-ylethyl)carbamoyl]-1*H*-pyrrol-2-yl}methylene)-2-oxoindoline-5-carboxylic acid :

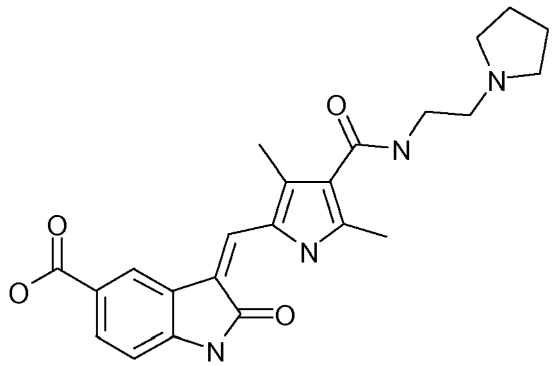

**2**

(970 mg, 57%);
NMR (300 MHz, DMSO-$d_6$) δ ppm: 13.44 (s,1H), 11.09 (s,1H), 8.26 (s,1H), 7.74 (d,1H), 7.68 (s,1H), 7.58 (bs,1H), 6.92 (d,1H), 3.37 (m,2H), 2.66 (bs,2H), 2.60 (bs,4H), 2.43 (s,3H), 2.40 (s,3H), 1.72 (bs,4H);
$t_R$: 0.45 min, 1.97 min, 2.22 min min; MS (ESI): m/z $(M+H)^+$423; $(M+H)^-$421. (3*Z*)-3-[(4-{[2-(Diethylamino)ethyl]carbamoyl}-3,5-dimethyl-1*H*-pyrrol-2-yl)methylene]-2-oxoindoline-5-carboxylic acid:

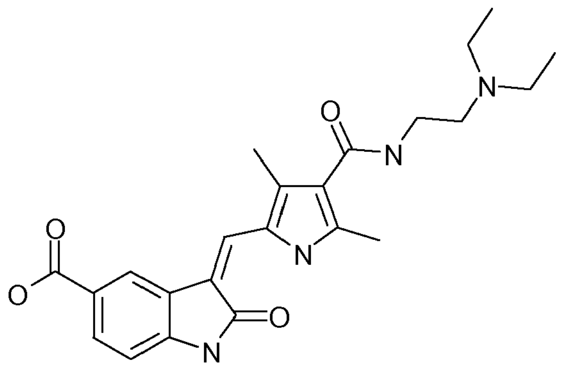

3

(940 mg, 55%);
NMR (300 MHz, DMSO-$d_6$) δ ppm: 13.53 (s,1H), 11.16 (bs,1H), 8.34 (s,1 H), 7.76 (d,1H), 7.75 (s,1H), 7.47 (t,1H), 6.94 (d,1H), 3.27 (m,2H), 2.65 (m, 6H), 2.44 (s,6H), 0.99 (t,6H);
$t_R$: 0.46 min,1.99 min, 2.31 min; MS (ESI): m/z $(M+H)^+$425, $(M+H)^-$423.

(3Z)-3-[(3,5-Dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid

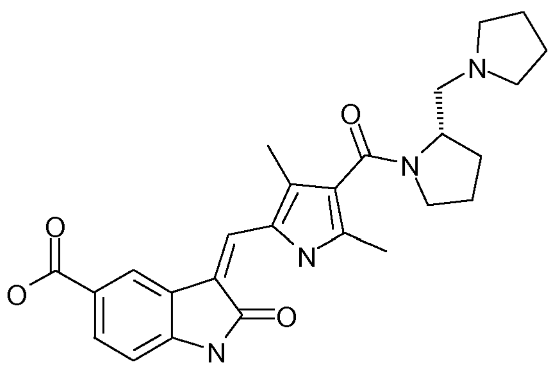

4

(1.04 g, 56%);
NMR (300 MHz, DMSO-$d_6$) δ ppm: 13.52 (bs,1H), 12.2 (bs,1H), 11.24 (s,1H), 8.36 (d,1H), 7.77 (s,1H), 7.76 (d,1H), 6.87 (d,1H), 4.30 (m,1H), 2.90 (bs,6H), 2.31 (s,6H), 1.86(m,10H);
$t_R$: 0.46 min, 2.13 min, 2.41 min; MS (ESI): *m*/*z* $(M+H)^+$ 463; $(M+H)^-$461. (3Z)-3-[(3,5-Dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid

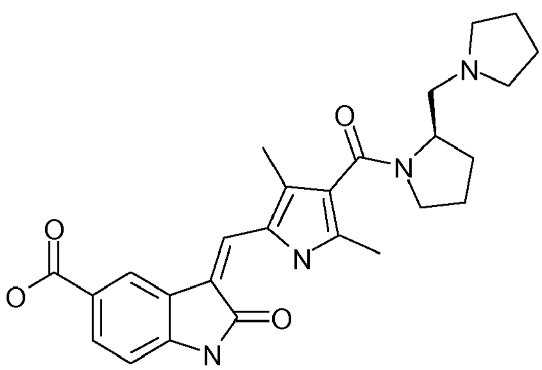

5

(940 mg, 51 %)
NMR (300 MHz, DMSO-$d_6$) δ ppm: 13.53 (bs,1H), 11.24 (s,1H), 8.37 (s,1H), 7.79 (s,1H), 7.80 (d,1H), 6.98 (d,1H), 2.32 (s,6H), 3.05 (bs,7H), 1.90 (m,8H);
$t_R$: 0.44 min, 2.21 min, 2.41 min; MS (ESI): m/z $(M+H)^+$ 463; $(M+H)^-$461.

**Example 2:** (3Z)-3-({3,5-Dimethyl-4-[(2-pyrrolidin-1-ylethyl)carbamoyl]-1H-pyrrol-2-yl}methylene)-2-oxo-N-[(1*R*)-1-phenylpropyl]indoline-5-carboxamide **(6) :**

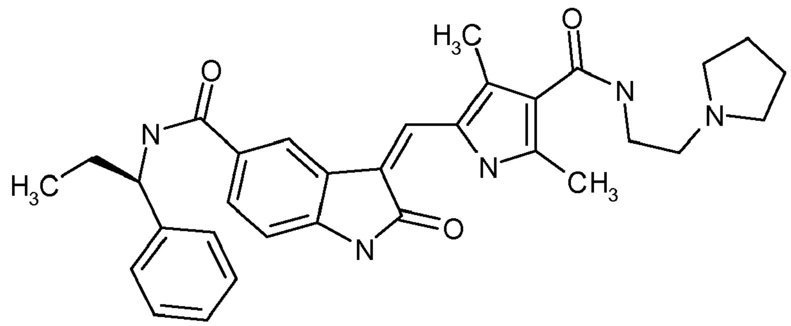

**6**

Step 1: Synthesis of 2-oxo-*N*-[(1*R*)-1-phenylpropyl]indoline-5-carboxamide

A mixture of 2-oxoindoline-5-carboxylic acid (300 mg, 1.69 mmol), (1*R*)-1-phenylpropan-1-amine (228 mg, 1.88 mmol), HATU (740 mg), 0.16 mL DIPEA in dry DMF (7 mL) was stirred overnight, then concentrated under reduced pressure. The resulting residue was diluted with AcOEt and washed with a saturated aq. solution of $K_2CO_3$. The organic layer was dried and concentrated in vacuo. Purification by flash chromatography (Kieselgel, $CHCl_3$:MeOH 15:1) provided intermediate 2-oxo-*N*-[(1*R*)-1-phenylpropyl]indoline-5-carboxamide: (340 mg, 68%):

NMR (300 MHz, DMSO-$d_6$) δ ppm: 10.59 (s,1H), 8.53 (d,1H), 7.78 (s,1H), 7.76 (d,1H), 7.33 (m,4H), 7.22 (t,1H), 6.85 (d,1H), 4.85 (m,1H), 3.53 (s,2H), 1.79 (m,2H), 0.89 (t,3H);
$t_R$: 3.09 min; MS (ESI): *m/z* $(M+H)^+$ 295; $(M+H)^-$293.

Step 2:

A mixture of 2-oxo-*N*-[(1*R*)-1-phenylpropyl]indoline-5-carboxamide (150 mg, 0.51 mmol) obtained at step 1, 5-formyl-2,4-dimethyl-*N*-(2-pyrrolidin-1-ylethyl)-1H-pyrrole-3-carboxamide (139 mg, 0.53 mmol) and piperidine (two drops) in abs. EtOH (5 mL) was refluxed for 4 h. The mixture was cooled down to room temperature and the resulting precipitate was filtered and washed with EtOH to provide compound **6** (160 mg, 58%)
NMR (300 MHz, DMSO-d6) δ ppm: 13.59 (s,1H), 11.10 (bs,1H), 8.51 (d,1H), 8.22 (s,1H), 7.69 (m,2H), 7.52 (s,1H), 7.40 (m,2H), 7.33 (m,2H), 7.22 (s,1H), 6.94 (d,1H), 4.80 (m,1H), 2.56 (m,4H), 2.43 (s,10H), 1.85 (m,2H), 1.68 (s,4H), 0.93 (t,3H);
$t_R$: 3.00 min; MS (ESI): m/z $(M+H)^+$ 540; $(M+H)^-$ 538.

The following compounds were prepared according to the procedure described by **example 2.**

(3*Z*)-3-({3,5-Dimethyl-4-[(4-methylpiperazin-1-yl)carbonyl]-1H-pyrrol-2-yl}methylene)-2-oxo-*N*-[(1*R*)-1-phenylpropyl]indoline-5-carboxamide

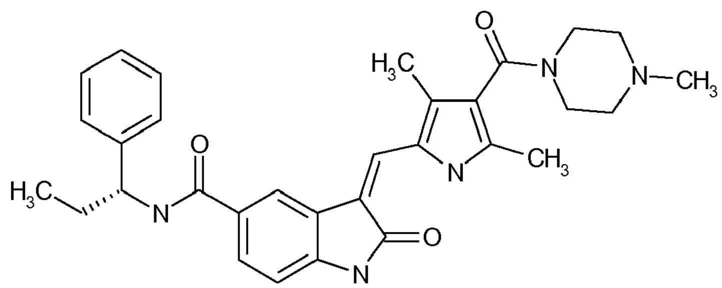

**7**

Starting from: 2-oxo-*N*-[(1*R*)-1-phenylpropyl]indoline-5-carboxamide and 3,5-dimethyl-4-[(4-methylpiperazin-1-yl)carbonyl]-1H-pyrrole-2-carbaldehyde compound **7** was obtained (110 mg, 41 %):

NMR (300 MHz, DMSO-d6) δ ppm: 13.54 (s,1H), 11.10 (s,1H), 8.52 (d,1H), 8.21 (s,1H), 7.71 (m,2H), 7.40 (m,2H), 7.32 (m,2H), 7.23 (m,1H), 6.93 (d,1H), 4.93 (m,1H), 3.37 (bs,4H), 2.19 (s,3H), 1.89 (m,2H), 0.92 (t,3H); $t_R$: 2.90 min; MS (ESI): m/z $(M+H)^+$ 526; $(M+H)^-$ 524.

(3*Z*)-N-[1-(3-Chlorophenyl)propyl]-3-({3,5-dimethyl-4-[(4-methylpiperazin-1-yl)carbonyl]-1H-pyrrol-2-yl}methylene)-2-oxoindoline-5-carboxamide

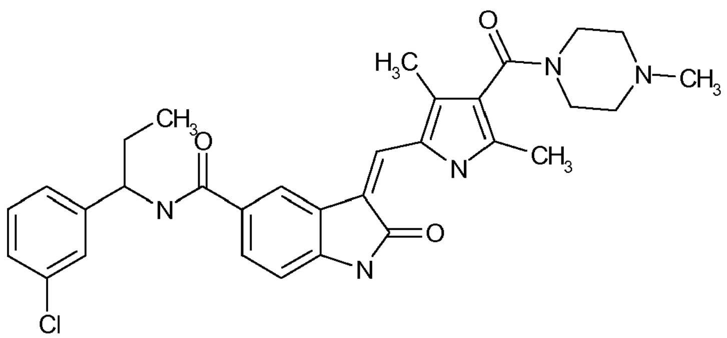

**8**

Starting from *N*-[1-(3-chlorophenyl)propyl]-2-oxoindoline-5-carboxamide and 3,5-dimethyl-4-[(4-methylpiperazin-1-yl)carbonyl]-1H-pyrrole-2-carbaldehyde compound **8** was obtained (110 mg, 38%):

NMR (300 MHz, DMSO-d6) δ ppm: 13.55 (s,1H), 11.12 (s,1H), 8.59 (d,1H), 8.20 (s,1H), 7.69 (m,2H), 7.47 (s,1H), 7.36 (m,2H), 7.28 (m,1H), 6.93 (d,1H), 4.92 (m,1 H), 3.46 (bs,4H), 2.29 (s,10H), 1.82 (m,2H), 0.92 (t,3H); $t_R$: 2.84, 3.09 min; MS (ESI): *m*/z $(M+H)^+$ 560, $(M+H)^-$ 558.

(3Z)-N-[1-(3,4-Difluorophenyl)propyl]-3-({3,5-dimethyl-4-[(4-methylpiperazin-1-yl)carbonyl]-1H-pyrrol-2-yl}methylene)-2-oxoindoline-5-carboxamide

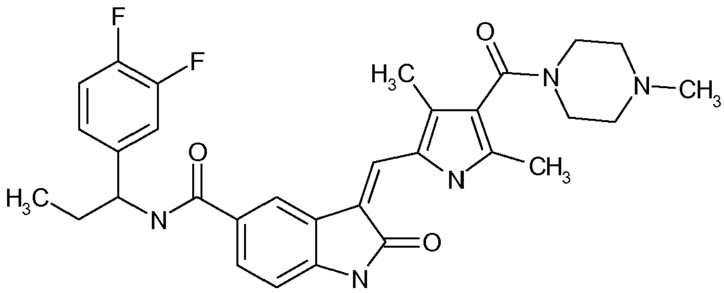

**9**

Starting from *N*-[1-(3,4-difluorophenyl)propyl]-2-oxoindoline-5-carboxamide and 3,5-dimethyl-4-[(4-methylpiperazin-1-yl)carbonyl]-1H-pyrrole-2-carbaldehyde compound **9** was obtained (76 mg, 26%)
NMR (300 MHz, DMSO-d6) δ ppm: 13.55 (s,1 H), 11.11 (s,1H), 8.56 (d,1H), 8.20 (s,1H), 7.70 (bs,2H), 7.43 (m,2H), 7.25 (s,1H), 6.94 (d,1H), 4.93 (m,1H), 3.46 (bs,4H), 2.28 (s,10H), 2.19 (s,3H), 1.84 (m,2H), 0.92(t,3H);
$t_R$: 0.44, 2.91, 3.04 min; MS (ESI): m/z $(M+H)^+$ 562, $(M+H)^-$ 560.

(3*Z*)-N-[1-(3,4-difluorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

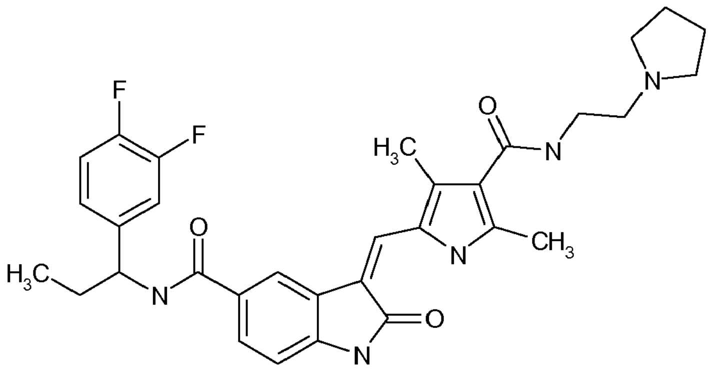

**10**

Starting from *N*-[1-(3,4-difluorophenyl)propyl]-2-oxoindoline-5-carboxamide and 5-formyl-2,4-dimethyl-*N*-(2-pyrrolidin-1-ylethyl)-1H-pyrrole-3-carboxamide compound 10 was obtained (80 mg, 27%):

NMR (300 MHz, DMSO-d6) δ ppm: 13.58 (s,1 H), 11.11 (s,1H), 8.56 (d,1H), 8.21 (s,1H), 7.69 (m,2H), 7.40 (m,3H), 7.25 (s,1H), 6.94 (d,1H), 4.92 (m,1H), 2.57 (t,4H), 2.43 (s,10H), 1.82 (m,2H), 1.69 (s,4H), 0.91 (t,3H);
$t_R$: 3.13 min; MS (ESI): m/z $(M+H)^+$ 576, $(M+H)^-$ 574.

(3Z)-*N*-[1-(3-Chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

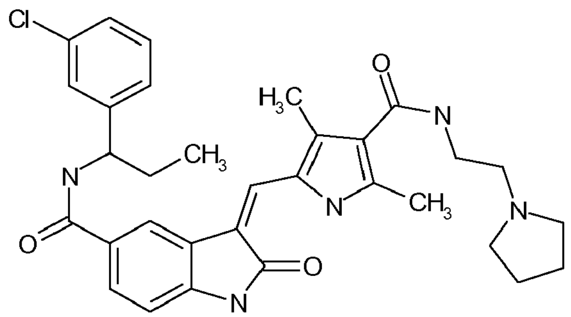

**11**

Starting from *N*-[1-(3-chlorophenyl)propyl]-2-oxoindoline-5-carboxamide and 5-formyl-2,4-dimethyl-*N*-(2-pyrrolidin-1-ylethyl)-1H-pyrrole-3-carboxamide compound **11** was obtained (120 mg, 41 %):

NMR (300 MHz, DMSO-d6) δ ppm: 13.56(s,1H), 11.11(s,1H), 8.50(d,1H), 8.18(s,1H), 7.46(m,7H), 6.87(d,1H), 4.80(m,1H), 2.48(bs,14H), 1.81(m,2H), 1.68(s,4H), 0.93(t,3H);
$t_R$: 2.98 min, 3.28 min; MS (ESI): *m/z* $(M+H)^+$ 574, $(M+H)^-$ 572.

(3Z)-N-[1-(4-Chlorophenyl)propyl]-3-({3,5-dimethyl-4-[(4-methylpiperazin-1-yl)carbonyl]-1H-pyrrol-2-yl}methylene)-2-oxoindoline-5-carboxamide

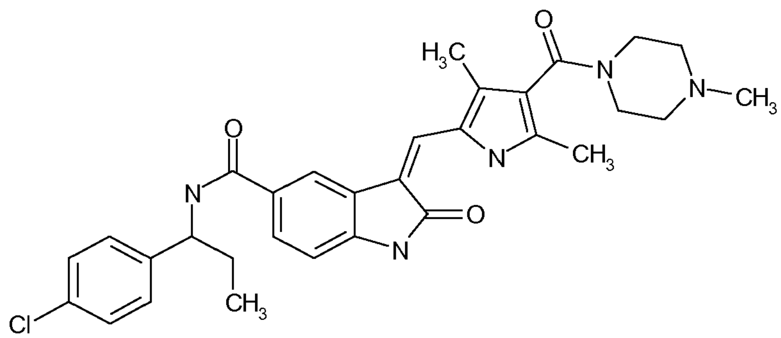

**12**

Starting from *N*-[1-(4-chlorophenyl)propyl]-2-oxoindoline-5-carboxamidec and 3,5-dimethyl-4-[(4-methylpiperazin-1-yl)carbonyl]-1H-pyrrole-2-carbaldehyde compound **12** was obtained (120 mg, 42%):

NMR (300 MHz, DMSO-d6) δ ppm: 13.55 (s,1 H), 11.11 (s,1H), 8.57 (d,1H), 8.20 (s,1H), 7.69(m,2H), 7.40 (dd,4H), 6.93 (d,1H), 4.92 (m,1H), 3.46 (bs,4H), 2.29 (s,10H), 2.19 (s,3H), 1.88 (m,2H),0.92 (t,3H);
$t_R$: 3.14 min; MS (ESI): *m/z* $(M+H)^+$ 560, $(M+H)^-$ 558.

(3Z)-N-[1-(4-Chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

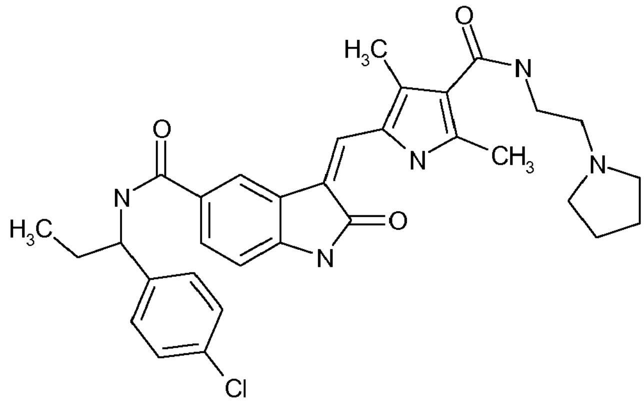

**13**

Starting from *N*-[1-(4-chlorophenyl)propyl]-2-oxoindoline-5-carboxamidec and 5-formyl-2,4-dimethyl-*N*-(2-pyrrolidin-1-ylethyl)-1H-pyrrole-3-carboxamide compound **13** was obtained (90 mg, 31 %):

NMR (300 MHz, DMSO-d6) δ ppm: 13.60 (s,1 H), 11.11 (s,1H), 8.55 (d,1H), 8.21 (s,1H), 7.70 (m,2H), 7.53 (s,1H), 7.40 (dd,4H), 6.94 (d,1H), 4.91 (m,1H), 2.59 (t,4H), 2.43 (s,10H), 1.83 (m,2H), 1.69 (s,4H), 0.92 (t,3H);
$t_R$: 3.25 min; MS (ESI): *m*/z $(M+H)^+$ 574, $(M+H)^-$572.

**<u>Example 3:</u>** (3Z)-3-{[3,5-Dimethyl-4-(piperidin-1-ylcarbonyl)-1*H*-pyrrol-2-yl]methylene}-2-oxo-*N*-[(1*R*)-1-phenylethyl]indoline-5-carboxamide **(14)**

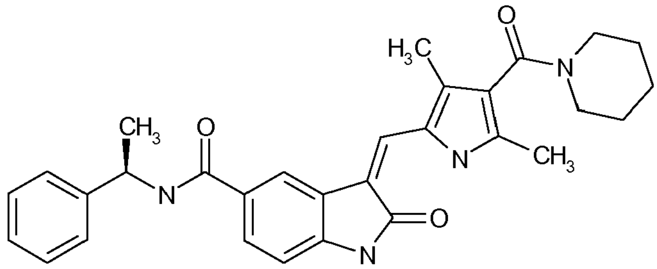

**14**

A mixture of (3Z)-3-{[3,5-dimethyl-4-(piperidin-1-ylcarbonyl)-1*H*-pyrrol-2-yl]methylene}-2-oxoindoline-5-carboxylic acid (1) (0.200 g, 0.51 mmol), EDCI (0.107 g, 0.56 mmol), HOBt (0.151 g, 1.5 mmol), TEA and (1R)-1-phenylethanamine (0.075 g, 0.62 mmol) in dry DMF was stirred for 1 day. The reaction mixture was poured on ice-water and extracted with AcOEt. The combined organic layers were washed with brine, dried on $MgSO_4$, filtered and concentrated *in vacuo.* Purication on silica gel (eluent $CHCl_3$ : MeOH: TEA 10:1:0.1) gave compound **14** (70 mg, 27%):

NMR (300 MHz, DMSO-$d_6$) δ ppm: 13.52 (s,1H), 11.10 (s,1 H), 8.60 (d,1H), 8.23 (s,1 H), 7.71 (d,1 H), 7.67 (s,1 H), 7.41 (m,2H), 7.33 (m,2H), 7.22 (t,1 H), 6.94 (d,1 H), 5.20 (m,1H), 3.43 (bs,4H), 2.29 (s,3H), 2.28 (s,3H), 1.60 (bs,2H), 1.51 (bs,4H), 1.50 (d,3H);
$t_R$: 3.92 min; MS (ESI): *m*/*z* $(M+H)^+$497; $(M+H)^-$ 495.

The following compounds were prepared according to the procedure described by **<u>example 3</u>**.

(3*Z*)-3-({3,5-dimethyl-4-[(4-methylpiperazin-1-yl)carbonyl]-1*H*-pyrrol-2-yl}methylene)-2-oxo-*N*-[(1*R*)-1-phenylethyl]indoline-5-carboxamide

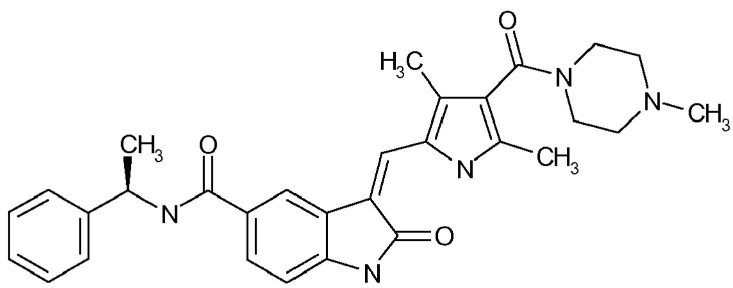

**15**

Starting from (3Z)-3-({3,5-dimethyl-4-[(4-methylpiperazin-1-yl)carbonyl]-1*H*-pyrrol-2-yl}methylene)-2-oxoindoline-5-carboxylic acid and (1*R*)-1-phenylethanamine, compound 15 was obtained (130 mg, 50%):

NMR (300 MHz, DMSO-$d_6$) δ ppm: 13.55 (s,1H), 11.11 (s,1H), 8.60 (d,1H), 8.23 (s,1H), 7.70 (d,1H), 7.68 (d,1H), 7.70 (d,1H), 7.68 (s,1H), 7.41 (d,1H), 7.33 (t,2H), 7.20 (t,1 H), 5.16 (m,1H), 3.55 (bs,4H), 2.28 (bs,10H), 2.20 (s,3H), 1.51 (s,3H); $t_R$: 0.46, 2.36, 2.54 min; MS (ESI): *m*/*z* $(M+H)^+$ 512, $(M+H)^-$510;

(3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxo-*N*-[(1*R*)-1-phenylethyl]indoline-5-carboxamide

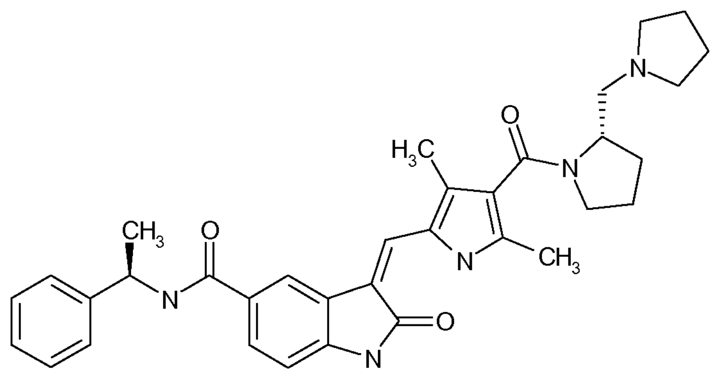

**16**

Compound **16** (50 mg, 17%) was obtained starting from: (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-phenylethanamine:

NMR (300 MHz, DMSO-$d_6$) δ ppm: 13.50 (bs,1 H), 11.10 (s,1 H), 8.58 (d,1 H), 8.23 (s,1 H), 7.71 (t,1 H), 7.67 (s,1 H), 7.41 (m,2H), 7.33 (m,2H), 7.22 (t,1 H), 6.94 (d,1 H), 5.20 (m,1H), 2.52 (bs,8H), 2.29 (s,6H), 1.91 (m,5H), 1.66 (bs,4H), 1.51 (d,3H);
$t_R$: 3.01min; MS (ESI): *m*/*z* $(M+H)^-$564; $(M+H)^+$566.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-2-oxo-*N*-[(1*R*)-1-phenylethyl]indoline-5-carboxamide

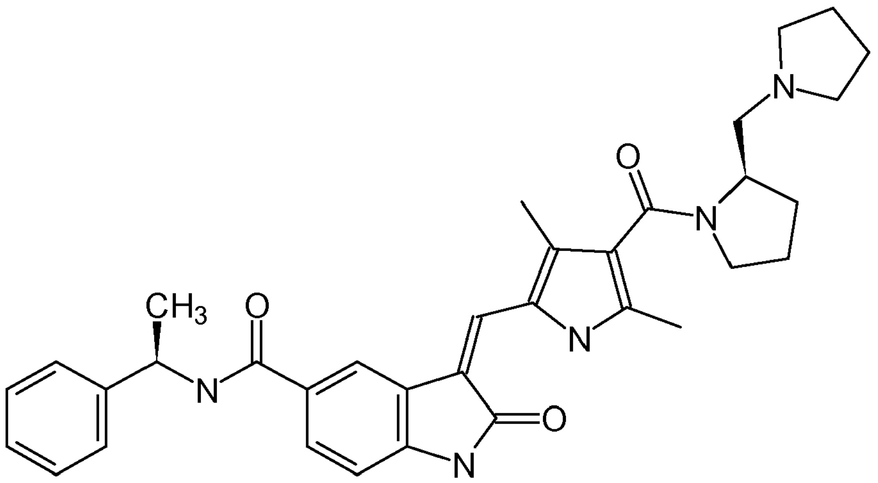

**17**

Compound **17** (74 mg, 25%) was obtained starting from: (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-phenylethanamine:

NMR (300 MHz, DMSO-$d_6$) δ ppm: 13.50 (bs,1 H), 11.10 (s,1 H), 8.59 (d,1 H), 8.23 (s,1H), 7.89 (m,2H), 7.41 (m,2H), 7.33 (m,2H), 7.22 (t,1H), 6.94 (d,1H), 2.50 (bs,8H), 2.30 (s,6H), 1.93 (bs,5H), 1.67 (bs,4H), 1.51 (d,3H), $t_R$: 3.18 min; MS (ESI): m/z $(M+H)^-$ 579, $(M+H)^+$ 581.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-2-oxo-*N*-[(1*S*)-1-phenylethyl]indoline-5-carboxamide

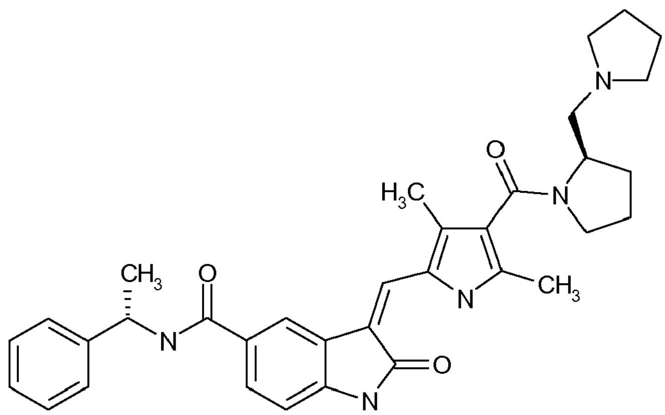

**18**

Compound **18** (86 mg, 30%) was obtained starting from: (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-phenylethanamine:

NMR (300 MHz, DMSO-$d_6$) δ ppm: 13.50 (bs,1 H), 11.10 (s,1 H), 8.59 (d,1 H), 8.22 (s,1H), 7.69 (m,2H), 7.41 (m,2H), 7.33 (m,2H), 7.20 (t,1H), 6.94 (d,1H), 5.20 (m,1H), 2.50 (bs,8H), 2.30 (s,6H), 1.94 (bs,5H), 1.67 (bs,4H), 1.51 (d,3H); $t_R$: 3.02 min; MS (ESI): *m/z* $(M+H)^-$564; $(M+H)^+$ 566.

(3Z)-3-[(3,5-Dimethyl-4-{[(2*S*)-2-(pyrrolidin-1-ylmethyl)pyrrolidin-1-yl]carbonyl}-1*H*-pyrrol-2-yl)methylene]-2-oxo-*N*-[(1*S*)-1-phenylethyl]indoline-5-carboxamide

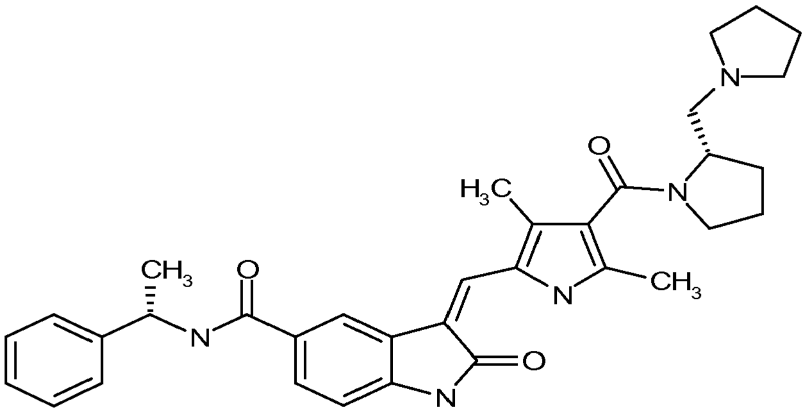

**19**

Compound **19** (106 mg, 37%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-phenylethanamine. NMR (300 MHz, DMSO-$d_6$) δ ppm: 13.51 (s,1H), 11.10 (s,1 H), 8.89 (d,1H), 8.22 (s,1H), 7.71 (d,1H), 7.67 (s,1H), 7.41 (m,2H), 7.33 (t,2H), 7.22 (t,1H), 6.94 (d,1H), 5.20 (m,1H), 4.25 (bs,1H), 3.45 (bs,2H), 2.80 (bs,4H), 2.30 (s,6H), 1.98 (bs,6H), 1.67 (bs,4H), 1.51 (d,3H);
$t_R$: 3.02 min; MS (ESI): *m*/*z* $(M+H)^+$ 566, $(M+H)^-$ 564.

(3*Z*)-3-({3,5-Dimethyl-4-[(4-methylpiperazin-1-yl)carbonyl]-1*H*-pyrrol-2-yl}methylene)-*N*-[(1*S*)-1-(4-methoxyphenyl)ethyl]-2-oxoindoline-5-carboxamide

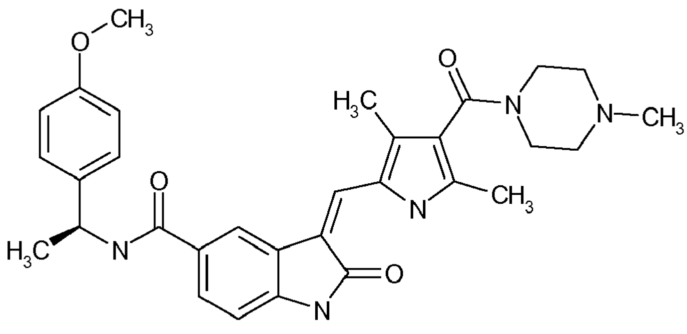

**20**

Compound **20** (120 mg, 43%) was obtained starting from (3*Z*)-3-({3,5-Dimethyl-4-[(4-methylpiperazin-1-yl)carbonyl]-1*H*-pyrrol-2-yl}methylene)-2-oxoindoline-5-carboxylic acid and (1*S*)-1-(4-methoxyphenyl)ethanamine:

NMR (300 MHz, DMSO-$d_6$) δ ppm: 13.54 (s,1H), 11.10 (s,1 H), 8.51 (d.1 H), 8.22 (s,1 H), 7.69 (d,1 H), 7.67 (s,1 H), 7.33 (d,2H), 6.93 (d,1 H), 6.89 (d,2H), 5.15 (m,1 H), 3.72 (s,3H), 3.46 (bs,4H), 2.29 (s,10H), 2.19 (s,3H), 1.48 (d,3H);
$t_R$: 0.45, 2.37, 2.55 min; MS (ESI): *m*/*z* $(M+H)^+$ 542; $(M+H)^-$540.

(3Z)-3-({3,5-Dimethyl-4-[(4-methylpiperazin-1-yl)carbonyl]-1*H*-pyrrol-2-yl}methylene)-*N*-[(1*R*)-1-(4-methoxyphenyl)ethyl]-2-oxoindoline-5-carboxamide

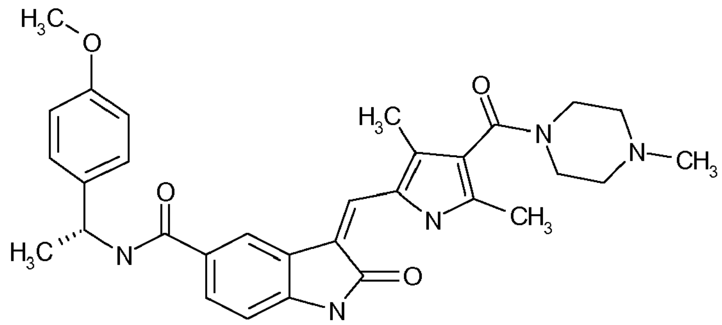

**21**

Compound **21** (130 mg, 47%) was obtained starting (3Z)-3-({3,5-dimethyl-4-[(4-methylpiperazin-1-yl)carbonyl]-1*H*-pyrrol-2-yl}methylene)-2-oxoindoline-5-carboxylic acid and(1R)-1-(4-methoxyphenyl)-ethanamine:

NMR (300 MHz, DMSO-$d_6$) δ ppm: 11.10 (s,1H), 11.10 (s,1 H), 8.50 (d,1H), 8.21 (s,1H), 7.72 (d,1H), 7.67 (s,1H), 7.33 (d,2H), 6.93 (d,1H) 6.89 (d,2H), 5.16 (m,1H), 3.73 (s,3H), 3.46 (bs,4H), 2.29 (s,10H), 2.19 (s,3H), 1.48 (d,3H), $t_R$: 0.45, 2.42, 2.55 min; MS (ESI): *m/z* $(M+H)^+$ 542; $(M+H)^-$540.

(3Z)-3-[(3,5-Dimethyl-4-{[(2S)-2-(pyrrolidin-1-ylmethyl)pyrrolidin-1-yl]carbonyl]-1H-pyrrol-2-yl)methylene]-N-[(1R)-1-(4-methoxyphenyl)ethyl]-2-oxoindoline-5-carboxamide

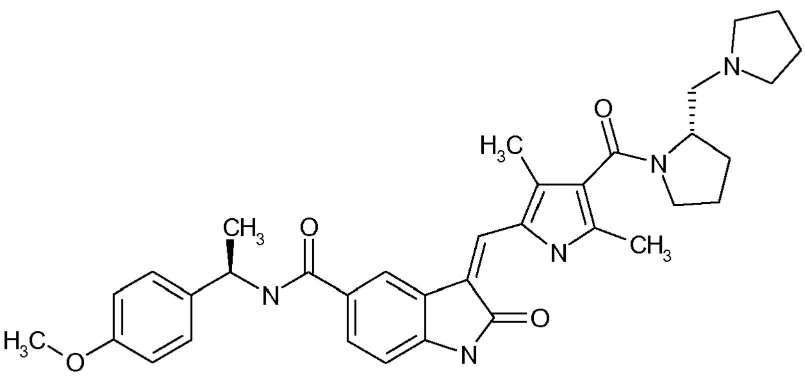

**22**

Compound **22** (41 mg, 13%) wa obtained starting from (3*Z*)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(4-methoxyphenyl)-ethanamine:

NMR (300 MHz, DMSO-$d_6$) δ ppm: 13.56 (s,1H), 11.14 (s,1H), 10.06 (bs,1H), 8.59 (d,1H), 8.30 (s,1H), 7.71 (m,2H), 7.34 (m,2H), 6.91 (m,3H), 5.15 (m,1H), 4.45 (m,1H), 3.72 (s,3H), 3.32 (bs,5H), 3.13 (bs,2H), 2.85 (bs,1H), 2.35 (s,6H), 19.2 (bs,8H), 1.49 (d,3H);
$t_R$: 3.02 min; (ESI): *m/z* $(M+H)^+$ 596; $(M+H)^-$ 594.

(3*Z*)-3-[(3,5-Dimethyl-4-{[(2*S*)-2-(pyrrolidin-1-ylmethyl)pyrrolidin-1-yl]carbonyl}-1*H*-pyrrol-2-yl)methylene]-*N*-[(1*R*)-1-(3-methoxyphenyl)ethyl]-2-oxoindoline-5-carboxamide

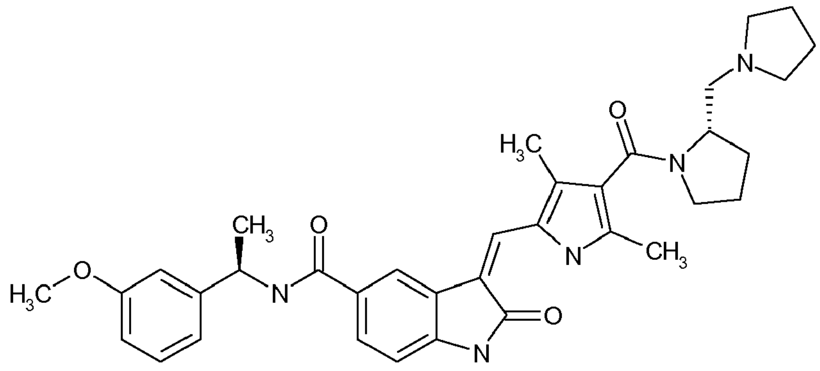

**23**

Compound **23** (68 mg, 22%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(3-methoxyphenyl)-ethanamine:

NMR (300 MHz, DMSO-$d_6$) δ ppm: 13.50 (bs,1 H), 11.10 (s,1 H), 8.57 (d,1 H), 8.22 (s,1H), 7.70 (m,2H), 7.24 (t,1H), 6.95 (m,3H), 6.80 (d,1H), 5.14 (m,1H), 4.30 (bs,1H), 3.74 (s,3H), 3.50 (bs,2H), 2.55 (bs,4H), 2.30 (s,6H), 1.96 (bs,6H), 1.68 (bs,4H), 1.49 (d,3H);
$t_R$: 2.59min, 3.01 min; MS (ESI): m/z $(M+H)^+$ 596; $(M+H)^-$ 594.

(3Z)-*N*-[(1*R*)-1-Cyclohexylethyl]-3-({3,5-dimethyl-4-[(4-methylpiperazin-1-yl)carbonyl]-1*H*-pyrrol-2-yl}methylene)-2-oxoindoline-5-carboxamide

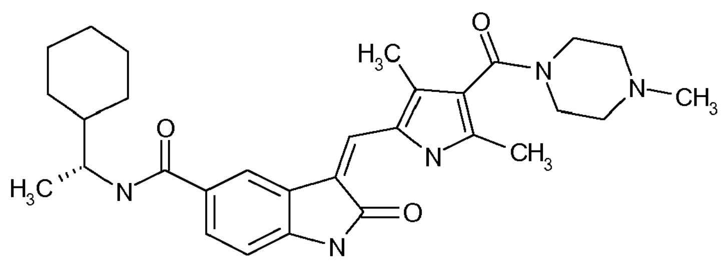

**24**

Compound **24** (153 mg, 58%) was obtained starting from (3Z)-3-({3,5-dimethyl-4-[(4-methylpiperazin-1-yl)carbonyl]-1*H*-pyrrol-2-yl}methylene)-2-oxoindoline-5-carboxylic acid and (1*R*)-1-cyclohexylethanamine:

NMR (300 MHz, DMSO-$d_6$) δ ppm: 13.55 (s,1H), 11.10 (s,1H), 8.18 (s,1H), 7.89 (d,1H), 7.67 (s,1H), 7.66 (d,1H), 6.91 (d,1H), 3.87 (m,1H), 3.46 (bs,4H), 2.29 (s,10H), 2.18 (s,3H), 1.74 (m,6H), 1.43 (m,1H), 1.18 (m,2H), 1.13 (d,3H), 0.98 (m,2H);
$t_R$: 0.45, 2.78 min; MS (ESI): *m*/*z*$(M+H)^+$518; $(M+H)^-$516.

(3Z)-*N*-[(1*R*)-1-(3-Chlorophenyl)ethyl]-3-({3,5-dimethyl-4-[(4-methylpiperazin-1-yl)carbonyl]-1*H*-pyrrol-2-yl}methylene)-2-oxoindoline-5-carboxamide

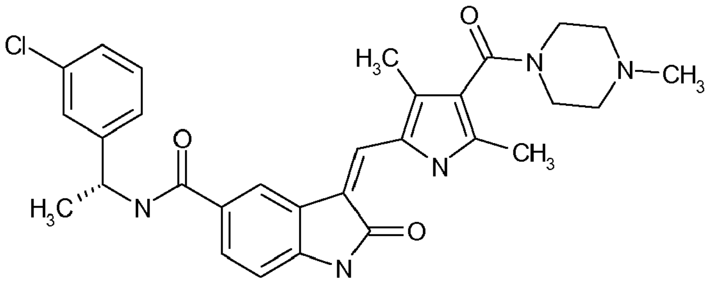

**25**

Compound **25** (153 mg, 55%) was obtained tarting from: (3*Z*)-3-({3,5-Dimethyl-4-[(4-methylpiperazin-1-yl)carbonyl]-1*H*-pyrrol-2-yl}methylene)-2-oxoindoline-5-carboxylic acid and (1*R*)-1-(3-chlorophenyl)-ethanamine:

NMR (300 MHz, DMSO-$d_6$) δ ppm: 13.54 (s,1H), 11.10 (s,1 H), 8.55 (d,1H), 8.23 (s,1H), 7.70 (d,1H), 7.69 (s,1H), 7.46 (s,1H), 7.37 (m,2H), 7.30 (m,1H), 6.95 (d,1 H), 5.15 (m,1 H), 3.55 (bs,4H), 2.29 (s,10H), 2.19 (s,3H), 1.50 (d,3H); $t_R$: 0.45,2.60, 2.75 min; MS (ESI): *m*/*z* $(M+H)^+$ 546; $(M+H)^-$544.

(3Z)-*N*-[(1*R*)-1-(3-Chlorophenyl)ethyl]-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxamide

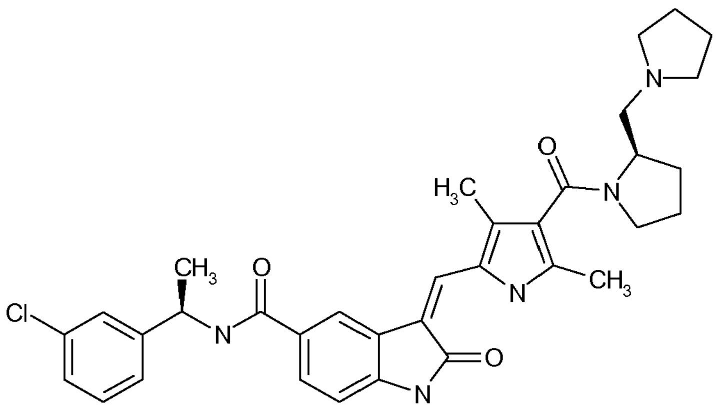

**26**

Compound **26** (78 mg, 25%) was obtained starting from (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(3-chlorophenyl)-ethanamine:

NMR (300 MHz, DMSO-$d_6$) δ ppm: 13.50 (bs,1 H), 11.10 (s,1 H), 8.65 (d,1 H), 8.22 (s,1H), 7.72 (bs,1H), 7.68 (s,1H), 7.46 (s,1H), 7.36 (m,2H), 7.29 (s,1H), 6.95 (d,1H), 5.17 (m,1H), 2.51 (bs,8H), 2.30 (s,6H), 1.83 (bs,5H), 1.67 (bs,4H), 1.51 (d,3H);
$t_R$: 3.22 min; MS (ESI): m/z $(M+H)^-$ 598; $(M+H)^+$ 600.

(3*Z*)-3-({3,5-Dimethyl-4-[(4-methylpiperazin-1-yl)carbonyl]-1*H*-pyrrol-2-yl}methylene)-*N*-[(1*R*)-1-(2-naphthyl)ethyl]-2-oxoindoline-5-carboxamide

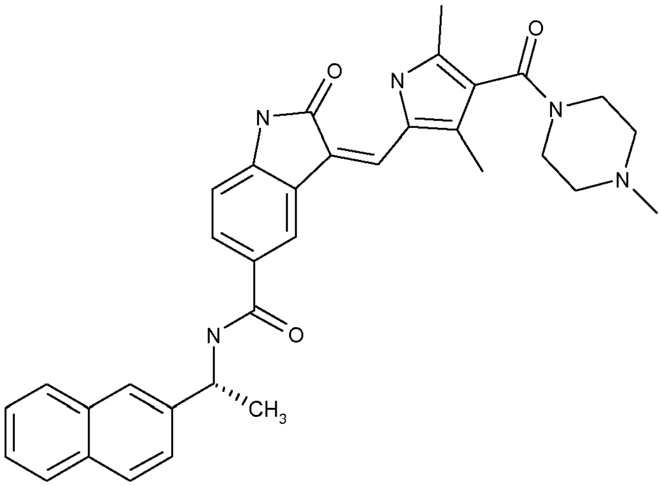

**27**

Compound **27** (133 mg, 46%) was synthesized starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*R*)-1-(2-naphthyl)ethanamine:

NMR (300 MHz, DMSO-$d_6$) δ ppm: 13.54 (s,1H), 11.12 (s,1H), 8.70 (d,1H), 8.25 (s,1H), 7.88 (m,4H), 7.74 (d,1H), 7.68 (s,1H), 7.61 (d,1H), 7.48 (m,2H), 6.95 (d,1 H), 5.35 (m,1 H), 3.46 (bs,4H), 2.28 (s,10H), 2.19 (s,3H), 1.61 (d,3H); $t_R$: 0.45, 2.73, 2.86 min; MS (ESI): *m*/*z* $(M+H)^+$ 562; $(M+H)^-$560.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-*N*-[(1*R*)-1-(2-naphthyl)ethyl]-2-oxoindoline-5-carboxamide

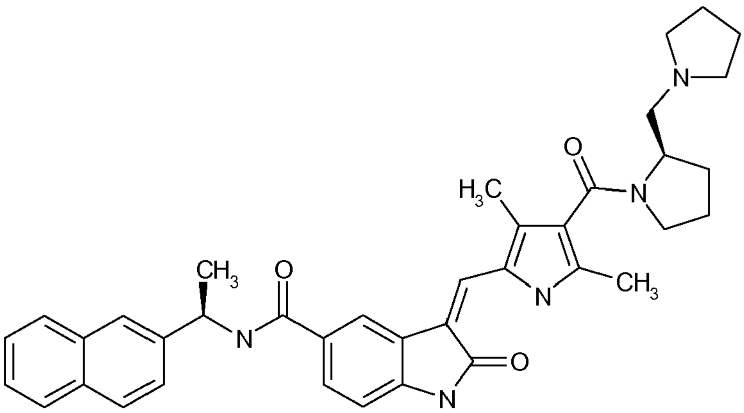

**28**

Compound **28** (81 mg, 26%) was obtained starting from (3*Z*)-3-[(3,5-dimethyl-4-{[(2R)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(2-naphthyl)ethanamine:

NMR (300 MHz, DMSO-$d_6$) δ ppm: 13.50 (s,1H), 11.11 (s,1H), 8.70 (d,1H), 8.25 (s,1H), 7.89 (m,4H), 7.73 (d,1H), 7.67 (s,1H), 7.61 (d,1H), 7.48 (m,2H), 6.94 (d,1H), 5.37 (m,1H), 4.30 (bs,1H), 3.50 (bs,4H), 2.29 (s,6H), 1.96 (bs,6H), 1.62 (bs,6H), 1.60 (d,3H);
$t_R$: 3.04 min, 3.32 min; MS (ESI): *m*/*z* $(M+H)^+$ 616; $(M+H)^-$614.

(3Z)-*N*-[(1*S*)-2,3-Dihydro-1*H*-inden-1-yl]-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxamide

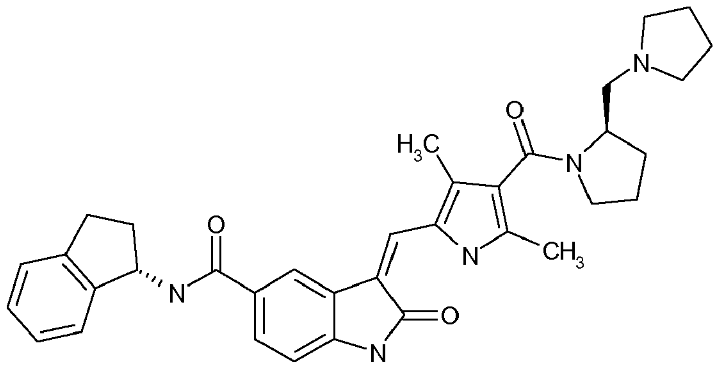

**29**

Compound **29** (48 mg, 16%) was obtained starting from (3*Z*)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-indan-1-amine:

NMR (300 MHz, DMSO-$d_6$) δ ppm: 13.50 (bs.1 H), 11.11 (s,1H), 8.54 (d,1H), 8.28 (s,1H), 7.76 (d,1H), 7.64 (s,1H), 7.22 (m,4H), 6.94 (d,1H), 5.60 (m,1H), 4.30 (bs,1H), 3.44 (bs,2H), 3.00 (m,2H), 2.90 (m,2H), 2.70 (bs,5H), 2.30 (s,3H), 2.27 (s,3H), 1.98 (bs,6H), 1.60 (bs,4H);
$t_R$: 2.84, 3.13 min; MS (ESI): *m*/*z*$(M+H)^+$578, $(M+H)^-$576.

(3Z)-*N*-[(1*R*)-2,3-Dihydro-1*H*-inden-1-yl]-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxamide

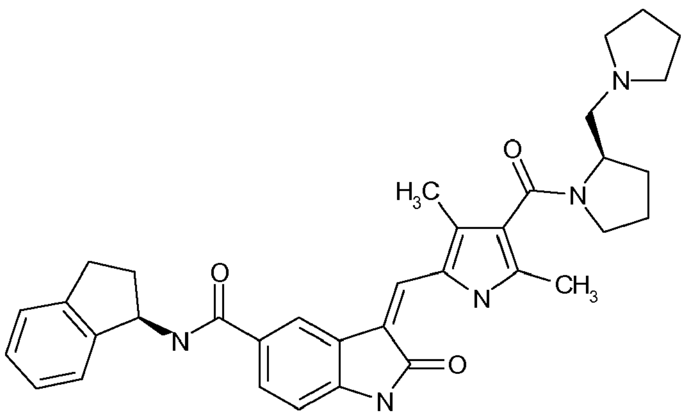

**30**

Compound **30** (52 mg, 18%) was obtained starting from (3*Z*)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-indan-1-amine:

NMR (300 MHz, DMSO-$d_6$) δ ppm: 13.50 (bs,1H), 11.11 (s,1H), 8.55 (d,1H), 8.28 (s,1H), 7.76 (d,1H), 7.64 (s,1H), 7.24 (m,4H), 6.94 (d,1H), 5.61 (m,1H), 4.30 (bs,1H), 3,44 (bs,2H), 3.00 (m,1H), 2.87 (m,1H), 2.61 (bs,5H), 2.01 (bs,6H), 1.67 (bs,4H), 1.50 (bs,2H), 0.96 (t,3H);
$t_R$: 2.78, 3.06 min; MS (ESI): m/z $(M+H)^+$578, $(M+H)^-$576.

(3*Z*)-3-({3,5-Dimethyl-4-[(4-methylpiperazin-1-yl)carbonyl]-1*H*-pyrrol-2-yl}methylene)-*N*-methyl-2-oxo-*N*-[(1*R*)-1-phenylethyl]indoline-5-carboxamide

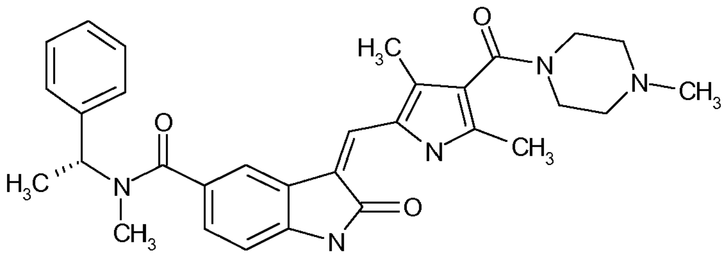

**31**

Compound **31** (118 mg, 44%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*R*)-*N*-methyl-1-phenylethanamine:

NMR (300 MHz, DMSO-$d_6$) δ ppm: 13.57 (s,1H), 11.02 (s,1H), 7.94 (s,1H), 7.73 (bs,1H), 7.35 (m,5H), 7.20 (d,1H), 6.92 (d,1H), 5.62 (bs,1H), 3.45 (bs,4H), 2.66 (s,3H), 2.28 (s,10H), 2.19 (s,3H), 1.58 (d,3H);
$t_R$: 0.44, 2.51, 2.64 min; MS (ESI): *m*/*z*$(M+H)^+$526, $(M+H)^-$524.

(3*Z*)-3-({3,5-Dimethyl-4-[(4-methylpiperazin-1-yl)carbonyl]-1*H*-pyrrol-2-yl}methylene)-*N*-[(1R)-1-(4-methylphenyl)ethyl]-2-oxoindoline-5-carboxamide

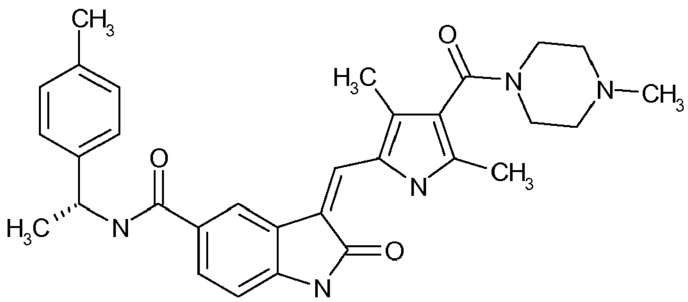

**32**

Compound **32** (143 mg, 53%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*R*)-1-(4-methylphenyl)-ethanamine:

NMR (300 MHz, DMSO-$d_6$) δ ppm: 13.54 (s,1H), 11.10 (s,1 H), 8.53 (d,1H), 8.22 (s,1H), 7.70 (d,1H), 7.29 (d,2H), 7.13 (d,2H), 6.93 (d,1H), 5.16 (m,1H), 3.47 (bs,4H), 2.28 (s,13H), 2.19 (s,3H), 1.48 (d,3H);#
$t_R$: 0.44, 2.55, 2.70 min; MS (ESI): *m*/*z* $(M+H)^+$ 526, $(M+H)^-$524.

(3*Z*)-3-[(3,5-Dimethyl-4-{[(2*S*)-2-(pyrrolidin-1-ylmethyl)pyrrolidin-1-yl]carbonyl}-1*H*-pyrrol-2-yl)methylene]-*N*-[(1*R*)-1-(4-methylphenyl)ethyl]-2-oxoindoline-5-carboxamide

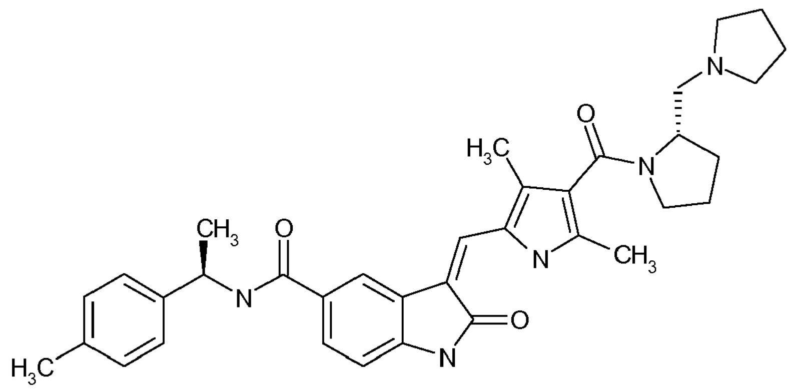

**33**

Compound **33** (60 mg, 20%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(4-methylphenyl)-ethanamine:

NMR (300 MHz, DMSO-$d_6$) δ ppm: 13.50 (bs,1 H), 11.10 (s,1 H), 8.53 (d,1 H), 8.21 (s,1H), 7.69 (m,2H), 7.29 (d,2H), 7.14 (d,2H), 6.93 (d,1H), 5.16 (m,1H), 2.52 (bs,8H), 2.28 (s,9H), 1.82 (bs,5H), 1.67 (bs,4H), 1.48 (d,3H); $t_R$: 3.17 min; MS (ESI): *m/z* $(M+H)^-$ 578, $(M+H)^+$ 580.

(3*Z*)-3-({3,5-Dimethyl-4-[(4-methylpiperazin-1-yl)carbonyl]-1*H*-pyrrol-2-yl}methylene)-*N*-[(1R)-1-(4-methylphenyl)ethyl]-2-oxoindoline-5-carboxamide

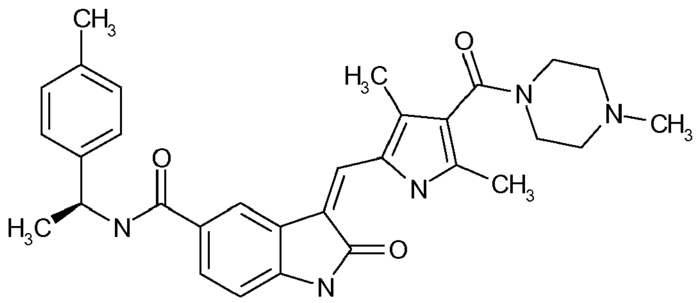

**34**

Compound **34** (130 mg, 49%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*S*)-1-(4-methylphenyl)-ethanamine:

NMR (300 MHz, DMSO-$d_6$) δ ppm: 13.54 (s,1H), 11.10 (s,1 H), 8.53 (d,1H), 8.22 (s,1H), 7.70 (d,1H), 7.29 (d,2H), 7.13 (d,2H), 6.93 (d,1H), 5.16 (m,1H), 3.47 (bs,4H), 2.28 (s,13H), 2.19 (s,3H), 1.48 (d,3H); $t_R$: 0.44, 2.57, 2.69 min; MS (ESI): *m/z* $(M+H)^+$ 526, $(M+H)^-$524.

(3Z)-*N*-[(1*R*)-1-(4-Chlorophenyl)ethyl]-3-({3,5-dimethyl-4-[(4-methylpiperazin-1-yl)carbonyl]-1*H*-pyrrol-2-yl}methylene)-2-oxoindoline-5-carboxamide

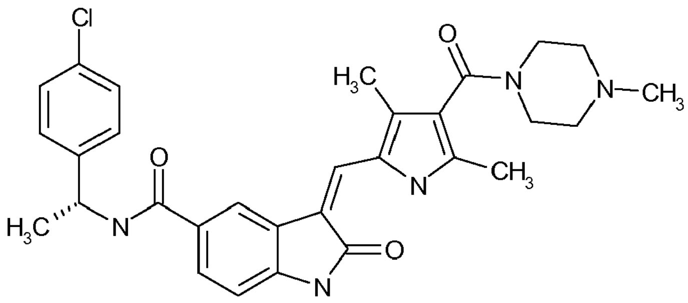

**35**

Compound **35** (189 mg, 69%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1 R)-1-(4-chlorophenyl)-ethanamine:

NMR (300 MHz, DMSO-$d_6$) δ ppm: 13.54 (s,1H), 11.10 (s,1 H), 8.65 (d,1H), 8.22 (s,1 H), 7.70 (d,1 H), 7.68 (s,1 H), 7.43 (d,2H), 7.38 (d,2H), 6.94 (d,1 H), 5.18 (m,1 H), 2.29 (s,10H), 2.19 (s,3H), 1.49 (d,3H);
$t_R$: 0.45, 2.62, 2.77 min; MS (ESI): *m/z* $(M+H)^+$ 546, $(M+H)^-$544.

(3*Z*)-*N*-[(1*S*)-1-(4-Chlorophenyl)ethyl]-3-[(3,5-dlmethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxamide

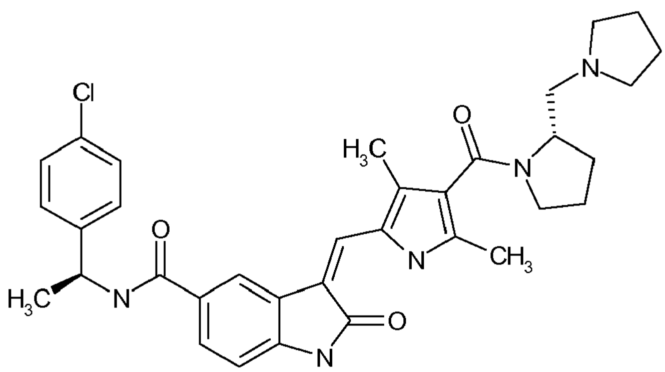

**36**

Compound **36** (72 mg, 24%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-(4-chlorophenyl)-ethanamine:

NMR (300 MHz, DMSO-$d_6$) δ ppm: 13.53 (bs,1H), 11.12 (s,1H), 8.64 (d,1H), 8.24 (s,1H), 7.71 (s,1H), 7.68 (s.1H), 7.41 (dd,4H), 6.94 (d,1H), 5.17 (m,1H), 2.52 (bs,8H), 2.31 (s,6H), 1.86 (bs,9H), 1.49(d,3H);
$t_R$: 3.27 min; MS (ESI): *m/z* $(M+H)^-$ 598, $(M+H)^+$ 600.

(3Z)-*N*-[(1*R*)-1-(4-Chlorophenyl)ethyl]-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxamide

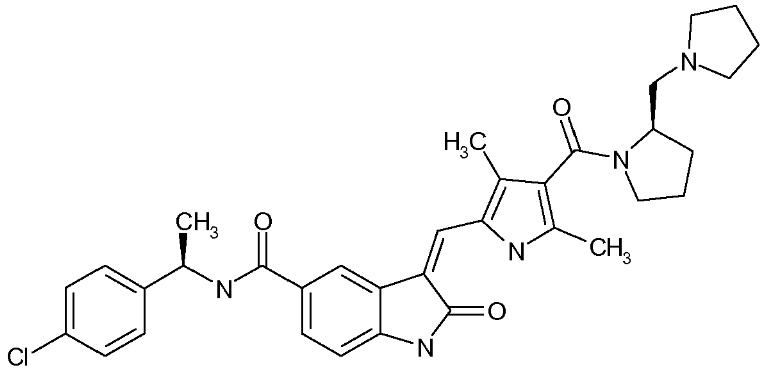

**37**

Compound **37** (65 mg, 21 %) was obtained starting from (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-(4-chlorophenyl)-ethanamine:

NMR (300 MHz, DMSO-$d_6$) δ ppm: 13.50 (s,1H), 11.11 (s,1H), 8.63 (d,1H), 8.22(s,1H), 7.70(m,2H), 7.41(dd,4H), 6.94(d,1H), 5.17(m,1H), 4.30(bs,1H), 3.45(bs,2 H), 2.80(bs,4H), 2.30(s,6H), 1.96(bs,6H), 1.71 (bs,4H), 1.49(d,3H), $t_R$: 2.97min, 3.23min, MS (ESI): *m*/*z* $(M+H)^+$ 600, $(M+H)^-$ 598.

(3Z)-*N*-[(1*R*)-1-(3-Chlorophenyl)ethyl]-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxamide

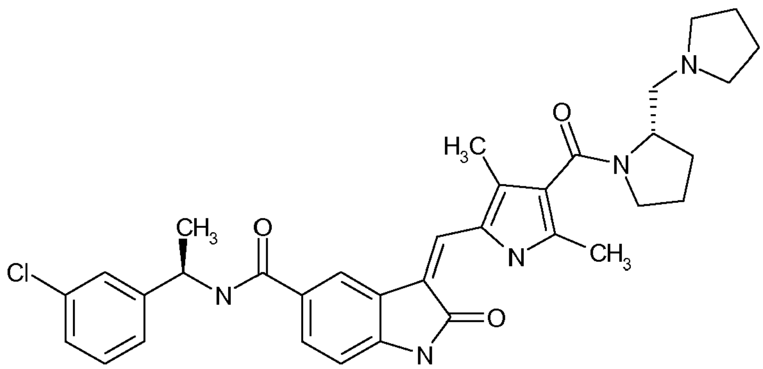

**38**

Compound **38** (66 mg, 22%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1R)-1-(3-chlorophenyl)-ethanamine:

NMR (300 MHz, DMSO-$d_6$) δ ppm: 13.51 (s,1H), 11.12 (s,1H), 8.65 (d,1H), 8.23 (s,1H), 7.70 (m,2H), 7.46 (s,1H), 7.36 (m,2H), 7.28 (m,1H), 6.95 (d,1H), 5.17 (m,1H), 4.30 (bs.1H), 3.45 (bs,2H), 2.60 (bs,4H), 2.30 (s,6H), 1.96 (bs,2H), 1.86 (bs,4H), 1.70 (bs,4H), 1.50 (d,3H);
$t_R$: 3.20 min, MS (ESI): *m*/z $(M+H)^+$ 600, $(M+H)^-$598.

(3Z)-*N*- [(1*R*)-1-(3-bromophenyl)ethyl]-3-({3,5-dimethyl-4-[(4-methylpiperazin-1-yl)carbonyl]-1*H*-pyrrol-2-yl}methylene)-2-oxoindoline-5-carboxamide

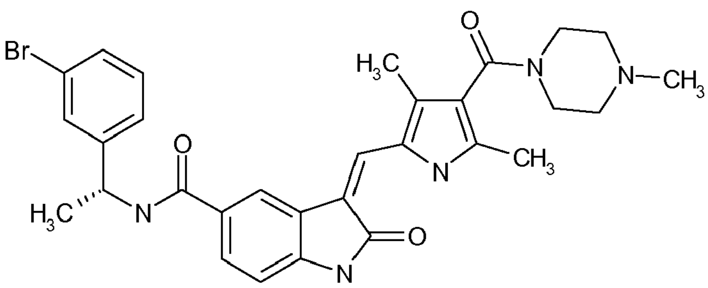

**39**

Compound **39** (244 mg, 83%) was obtained starting from: (3*Z*)-3-({3,5-Dimethyl-4-[(4-methylpiperazin-1-yl)carbonyl]-1*H*-pyrrol-2-yl}methylene)-2-oxoindoline-5-carboxylic acid and (1*R*)-1-(3-bromophenyl)-ethanamine:

NMR (300 MHz, DMSO-$d_6$) δ ppm: 13.54 (s,1H), 11.12 (s,1H), 8.65 (d,1H), 8.22 (s,1H), 7.70 (t,1H), 7.69 (s,1H), 7.60 (s,1H), 7.70 (d,1H), 7.40 (s,1H), 7.31 (d,1H), 6.95 (d,1 H), 5.19 (m,1 H), 3.46 (bs,4H), 2.29(s,10H), 2.19 (s,3H), 1.50 (d,3H);
$t_R$: 0.45, 2.65, 2.78 min; MS (ESI): *m*/*z* $(M+H)^+$ 590, $(M+H)^-$588.

(3*Z*)-*N*-[(1*S*)-1-(3-Chlorophenyl)ethyl]-3-({3,5-dimethyl-4-[(4-methylpiperazin-1-yl)carbonyl]-1*H*-pyrrol-2-yl}methylene)-2-oxoindoline-5-carboxamide

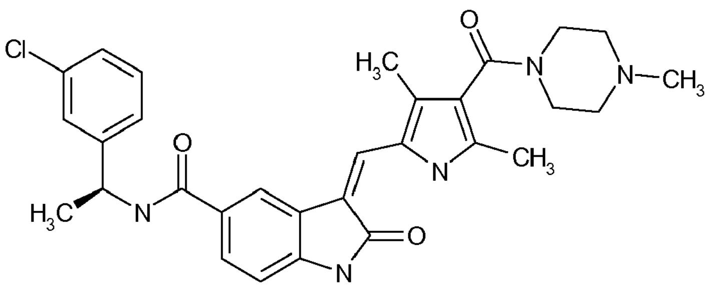

**40**

Compound **40** (110 mg, 39%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*S*)-1-(3-chlorophenyl)-ethanamine:

NMR (300 MHz, DMSO-$d_6$) δ ppm: 13.53 (s,1H), 11.11 (s,1H), 8.65 (d,1H), 8.23 (s,1 H), 7.69 (d,1 H), 7.68 (s,1 H), 7.45 (s,1 H), 7.35 (m,2H), 7.28 (t,1 H), 5.16 (m,1 H), 3.43 (bs,2H), 3.02 (bs,2H), 2.28 (s,10H), 2.20 (s,3H), 1.59 (d,3H);
$t_R$: 0.44, 2.63, 2.73 min; MS (ESI): *m*/*z* $(M+H)^+$ 546, $(M+H)^-$ 544.

(3*Z*)-3-[(3,5-Dimethyl-4-{[(2*S*)-2-(pyrrolidin-1-ylmethyl)pyrrolidin-1-yl]carbonyl}-1*H*-pyrrol-2-yl)methylene]-*N*-[(1*S*)-1-(4-fluorophenyl)ethyl]-2-oxoindoline-5-carboxamide

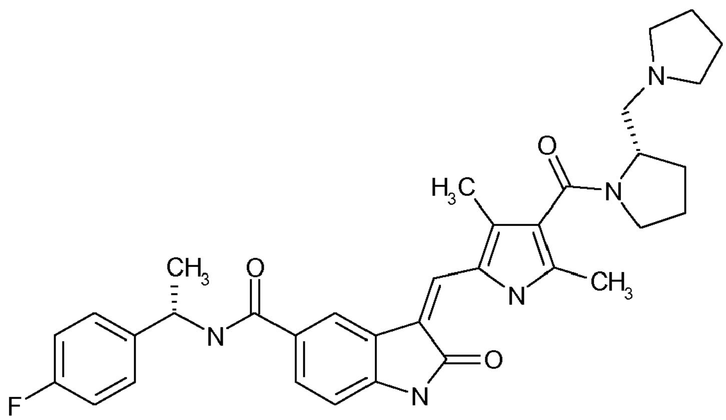

**41**

Compound **41** (103 mg, 34%) was obtained starting from (3*Z*)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-(4-fluorophenyl)-ethanamine:

NMR (300 MHz, DMSO-$d_6$) δ ppm: 13.50 (bs,1 H), 11.10 (s,1 H), 8.60 (d,1 H), 8.22 (s,1H), 7.69 (m,2H), 7.44 (m,2H), 7.14 (m,2H), 6.94 (m,1H), 5.20 (m,1H), 2.52 (bs,8H), 2.30 (s,6H), 1.90 (bs,5H), 1.67 (bs,4H), 1.50 (d,3H);
$t_R$: 3.09 min; MS (ESI): m/z $(M+H)^-$ 582, $(M+H)^+$ 584.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-*N*-[(1*S*)-1-(4-fluorophenyl)ethyl]-2-oxoindoline-5-carboxamide

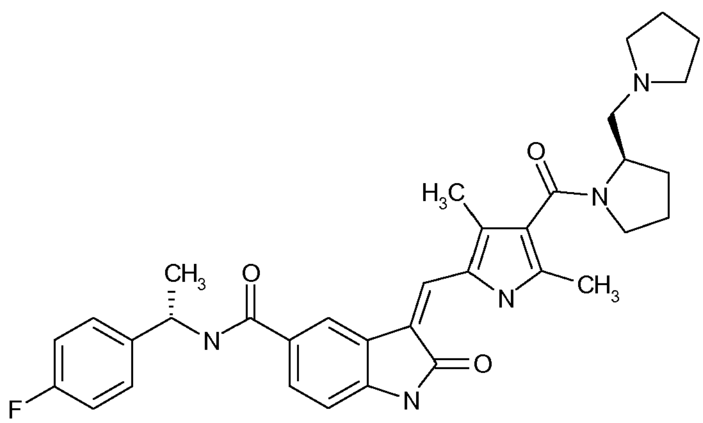

**42**

Compound **42** (85 mg, 28%) was obtained starting from (3*Z*)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-(4-fluorophenyl)-ethanamine:

NMR (300 MHz, DMSO-$d_6$) δ ppm: 13.5 (bs,1H), 11.10 (s,1 H), 8.60 (d,1H), 8.22 (s,1H), 7.71 (bs,1H), 7.67 (s,1H), 7.44 (m,2H), 7.14 (m,2H), 6.94 (d,1H), 5.19 (m,1H), 2.52 (bas,8H), 2.29 (s,6H), 1.91 (bs,5H), 1.67 (bs,4H), 1.50 (d,3H);
$t_R$: 3.07 min; MS (ESI): *m*/*z* $(M+H)^-$582, $(M+H)^+$ 584.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-*N*-[(1*R*)-1-(4-fluorophenyl)ethyl]-2-oxoindoline-5-carboxamide:

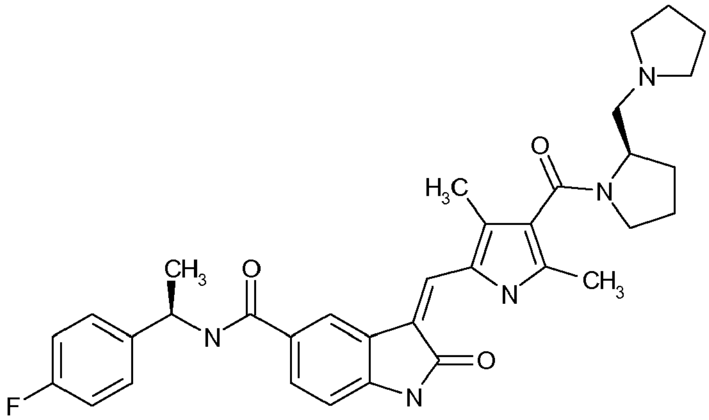

**43**

Compound 43 (123 mg, 41%) was obtained starting from (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1R)-1-(4-fluorophenyl)-ethanamine:

NMR (300 MHz, DMSO-$d_6$) δ ppm: 13.51 (bs,1 H), 11.10 (s,1 H), 8.60 (d,1 H), 8.22 (s,1H), 7.71 (bs,1H), 7.67 (s,1H), 7.44 (m,2H), 7.15 (m,2H), 6.94 (d,1H), 5.19 (m,1H), 2.52 (bs,8H), 2.30 (s,6H), 1.92 (bs,5H), 1.68 (bs,4H), 1.50 (d,3H); $t_R$: 3.06 min; MS (ESI): *m/z* $(M+H)^-$ 582, $(M+H)^+$ 584.

(3*Z*)-3-[(3,5-Dimethyl-4-{[4-(4-methylpiperazin-1-yl)piperidin-1-yl]carbonyl}-1*H*-pyrrol-2-yl)methylene]-2-oxo-*N*-[(1*R*)-1-phenylethyl]indoline-5-carboxamide

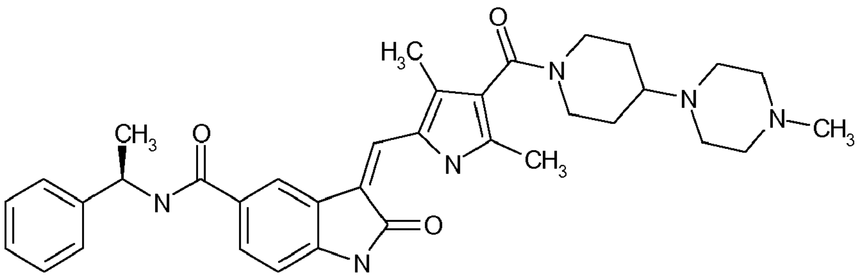

**44**

Compound 44 (85 mg, 27%) was obtained starting from (3Z)-3-[(3,5-dimethyl-4-{[4-(4-methylpiperazin-1-yl)piperidin-1-yl]carbonyl}-1*H*-pyrrol-2-yl)methylene]-2-oxoindoline-5-carboxylic acid and (1*R*)-1-phenylethanamine:

NMR (300 MHz, DMSO-$d_6$) δ ppm: 13.50 (s,1H), 11.08 (s,1H), 8.57 (d,1H), 8.22 (s,1H), 7.68 (m,2H), 7.40-7.20 (m,5H), 6.92 (d,1H), 5.20 (m,1H), 4.0 (bs,2H), 2.90 (bs,2H), 2.43 (bs,8H), 2.22 (s,6H), 3.19 (s,3H), 1.86 (bs,5H), 2.49 (d,3H);
$t_R$: 2.50 min; MS (ESI): *m/z* $(M+H)^+$ 595, $(M+H)^-$ 593.

(3*Z*)-3-({3,5-Dimethyl-4-[(4-methylpiperazin-1-yl)carbonyl]-1*H*-pyrrol-2-yl}methylene)-2-oxo-N-[(1*S*)-1-phenylpropyl]indoline-5-carboxamide

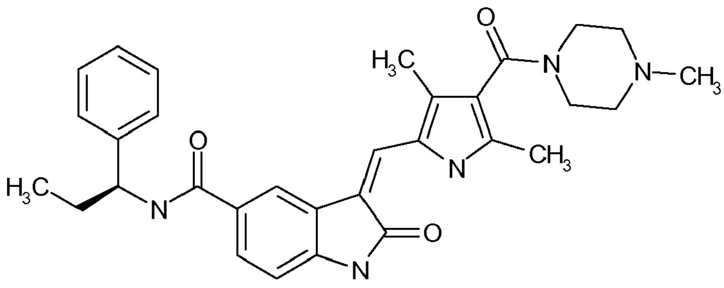

**45**

Compound 45 (105 mg, 39%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1*S*)-1-phenylpropan-1-amine:

NMR (300 MHz, DMSO-$d_6$) δ ppm: 13.54 (s,1H), 11.10 (s,1 H), 8.53 (d,1H), 8.21 (s,1H), 7.70 (d,1H), 7.68 (s,1 H), 7.41 (d,2H), 7.32 (t,2H), 7.22 (t,1H), 6.94 (d,1H), 4.94 (m,1H), 3.46 (bs,4H), 2.29 (s,10H), 2.19 (s,3H), 1.83 (m,2H), 0.90 (t,3H);
$t_R$: 0.46, 2.54, 2.67 min; MS (ESI): m/z $(M+H)^+$ 526, $(M+H)^-$ 524.

(3*Z*)-3-[(3,5-Dimethyl-4-{[(2*S*)-2-(pyrrolidin-1-ylmethyl)pyrrolidin-1-yl]carbonyl}-1*H*-pyrrol-2-yl)methylene]-2-oxo-N-[(1*R*)-1-phenylpropyl]indoline-5-carboxamide

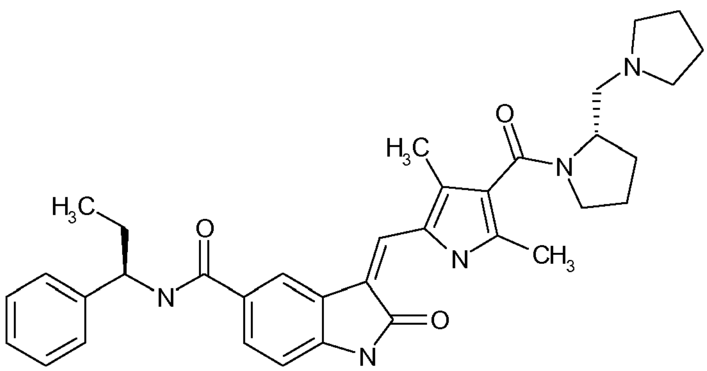

**46**

Compound 46 (64 mg, 21 %) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-phenylpropan-1-amine:

NMR (300 MHz, DMSO-$d_6$) δ ppm: 13.51 (bs,1 H), 11.10 (s,1 H), 8.53 (d,1 H), 8.20 (s,1H), 7.69 (m,2H), 7.42-7.30 (m,4H), 7.22 (t,1H), 6.94 (d,1H), 2.52 (bs,8H), 2.29 (s,6H), 1.84 (bs,7H), 1.67 (bs,4H), 0.92 (t,3H);
$t_R$: 3.16 min; MS (ESI): m/z $(M+H)^-$ 578, $(M+H)^+$ 580.

(3*Z*)-3-[(3,5-Dimethyl-4-{[(2*S*)-2-(pyrrolidin-1-ylmethyl)pyrrolidin-1-yl]carbonyl}-1*H*-pyrrol-2-yl)methylene]-2-oxo-*N*-[(1*S*)-1-phenylpropyl]indoline-5-carboxamide

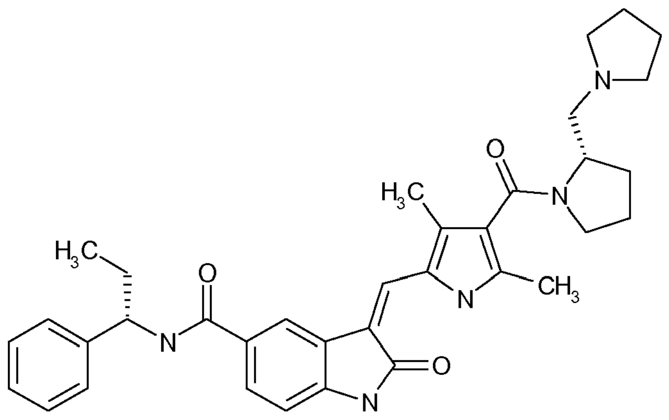

**47**

Compound 47 (95 mg, 32%) was obtained starting from (3Z)-3-[(3,5-dimethyl-4-{[(2*S*)-2-(pyrrolidin-1-ylmethyl)pyrrolidin-1-yl]carbonyl}-1*H*-pyrrol-2-yl)methylene]-2-oxoindoline-5-carboxylic acid and (1*S*)-1-phenylpropan-1-amine:

NMR (300 MHz, DMSO-$d_6$) $\delta$ ppm: 13.51 (bs,1 H), 11.10 (s.1 H), 8.53 (d,1 H), 8.21 (s,1H), 7.70 (m,2H), 7.41 (m,2H), 7.32 (t,2H), 7.22 (m,1H), 6.94 (d,1H), 4.95 (m,1H), 4.26 (bs.1H), 3.45 (bs,2H), 2.60 (bs,4H), 2.30 (s,6H), 1.86 (bs,6H), 1.59 (bs,6H), 0.92 (t,3H);
$t_R$: 2.86, 3.06 min; MS (ESI): *m*/*z* $(M+H)^+$ 580, $(M+H)^-$ 578.

(3*Z*)-3-[(3,5-Dimethyl-4-{[(2*R*)-2-(pyrrolidin-1-ylmethyl)pyrrolidin-1-yl]carbonyl}-1*H*-pyrrol-2-yl)methylene]-2-oxo-*N*-[(1*R*)-1-phenylpropyl]indoline-5-carboxamide

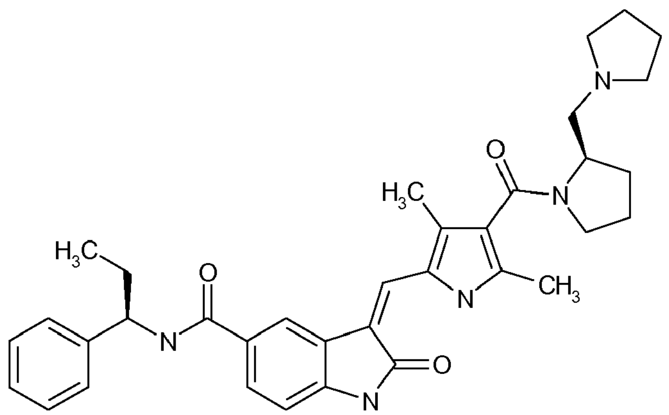

**48**

Compound 48 (120 mg, 41%) was obtained starting from (3Z)-3-[(3,5-dimethyl-4-{[(2*R*)-2-(pyrrolidin-1-ylmethyl)pyrrolidin-1-yl]carbonyl}-1*H*-pyrrol-2-yl)methylene]-2-oxoindoline-5-carboxylic acid and (1*R*)-1-phenylpropan-1-amine:

NMR (300 MHz, DMSO-$d_6$) $\delta$ ppm: 13.51 (bs,1 H), 11.10 (s,1 H), 8.53 (d,1 H), 8.20 (s,1H), 7.71 (bs,1H), 7.67 (s,1H), 7.41 (m,2H), 7.32 (m,2H), 7.23 (m,1H), 6.94 (d,1H), 4.94 (m,1H), 2.50 (bs,8H), 2.30 (s,2H), 1.87 (bs,7H), 1.67 (bs,4H), 0.93 (t,3H);
$t_R$: 3.02 min; MS (ESI): *m*/*z* $(M+H)^-$ 578, $(M+H)^-$ 580.

(3*Z*)-*N*-[(1*S*)-1-(3-Bromophenyl)ethyl]-3-({3,5-dimethyl-4-[(4-methylpiperazin-1-yl)carbonyl]-1*H*-pyrrol-2-yl}methylene)-2-oxoindoline-5-carboxamide

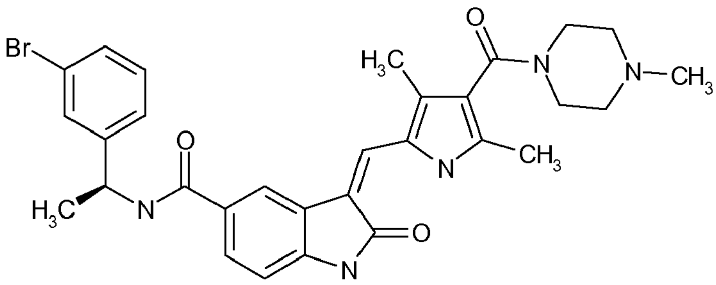

**49**

Compound 49 (102 mg, 33%) was obtained starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and (1 R)-1-(3-bromophenyl)-ethanamine:

NMR (300 MHz, DMSO-$d_6$) δ ppm: 13.53 (s,1H), 11.10 (s,1 H), 8.60 (d,1H), 8.21 (s,1 H), 7.69 (m,2H), 7.58 (s,1 H), 7.41 (m,2H), 7.28 (t,1 H), 6.93 (d,1 H), 5.18(m,1H), 3.45 (bs,4H), 2.27 (s,10H), 2.18 (s,3H), 1.48 (d,3H);
$t_R$: 0.44, 2.76, 2.91 min; MS (ESI): *m*/*z* $(M+H)^+$ 590, $(M+H)^-$ 588.

(3*Z*)-3-{[3,5-Dimethyl-4-(pyrrolidin-1-ylcarbonyl)-1*H*-pyrrol-2-yl]methylene}-*N*-[(1*S*)-1-(4-methoxyphenyl)ethyl]-2-oxoindoline-5-carboxamide

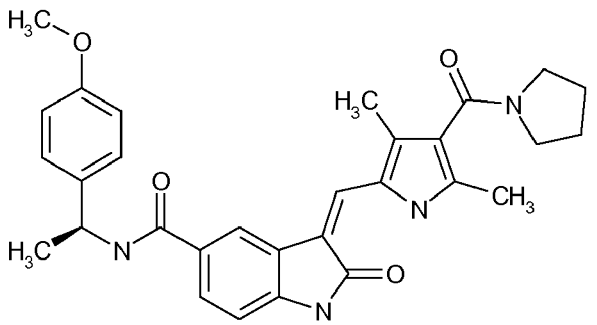

**50**

Compound 50 (111 mg, 43%) was obtained starting from ((3*Z*)-3-{[3,5-dimethyl-4-(pyrrolidin-1-ylcarbonyl)-1*H*-pyrrol-2-yl]methylene}-2-oxoindoline-5-carboxylic acid and (1*S*)-1-(4-methoxyphenyl)-ethanamine:

NMR (300 MHz, DMSO-$d_6$) δ ppm: 13.50 (s,1H), 11.10 (s,1 H), 8.55 (d,1H), 8.22 (s,1 H), 7.70 (d,1 H), 7.66 (s,1 H), 7.33 (d,2H), 6.92 (d,1 H), 6.89 (d,2H), 5.20 (m,1 H), 3.72 (s,3H), 3.40 (bs,2H), 3.28 (bs,2H), 2.30 (s,6H), 2.80 (bs,4H), 1.48 (d,3H);
$t_R$: 3.58 min; MS (ESI): *m*/*z* $(M+H)^+$ 513, $(M+H)^-$ 511.

(3*Z*)-3-({3,5-Dimethyl-4-[(4-methylpiperazin-1-yl)carbonyl]-1*H*-pyrrol-2-yl}methylene)-*N*-[1-(3-methylphenyl)ethyl]-2-oxoindoline-5-carboxamide

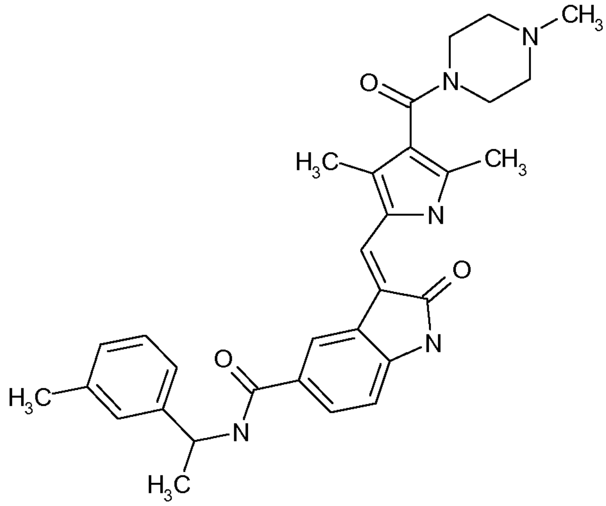

**51**

Compound 51 (95 mg, 35%) was obatined starting from (3*Z*)-3-({3,5-dimethyl-4-[(4-methylpiperazin-1-yl)carbonyl]-1*H*-pyrrol-2-yl}methylene)-2-oxoindoline-5-carboxylic acid and 1-(3-methylphenyl)ethanamine:

NMR (300 MHz, DMSO-$d_6$) δ ppm: 13.54 (s,1H), 11.10 (s,1 H), 8.55 (d,1H), 8.23 (s,1H), 7.68 (m,2H), 7.21 (m,3H), 7.04 (s,1H), 6.93 (d,1H), 5.16 (m,1H), 3.47 (bs,4H), 2.29 (s,13H), 2.19 (s,3H), 1.48 (d,3H);
$t_R$: 0.44, 2.76, 2.91 min; MS (ESI): *m*/*z* $(M+H)^+$ 526, $(M+H)^-$ 524.

(3*Z*)-3-[(3,5-Dimethyl-4-{[4-(4-methylpiperazin-1-yl)piperidin-1-yl]carbonyl}-1*H*-pyrrol-2-yl)methylene]-2-oxo-*N*-[(1*S*)-1-phenylpropyl]indoline-5-carboxamide

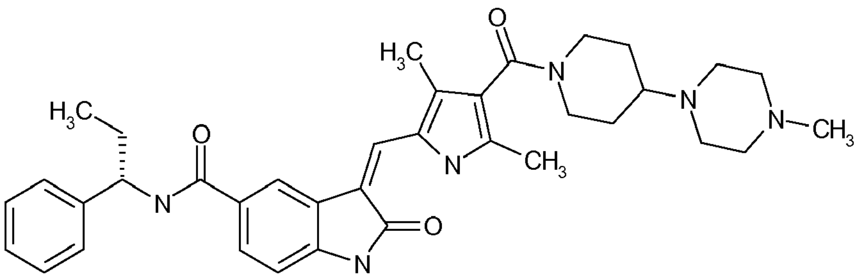

**52**

Compound 52 (102 mg, 32%) was obtained starting from (3Z)-3-[(3,5-dimethyl-4-{[4-(4-methylpiperazin-1-yl)piperidin-1-yl]carbonyl}-1*H*-pyrrol-2-yl)methylene]-2-oxoindoline-5-carboxylic acid and (1*S*)-1-phenylpropan-1-amine:

NMR (300 MHz, DMSO-$d_6$) δ ppm: 13.53 (s,1 H), 11.10 (s,10H), 8.53 (d,1 H), 8.22 (s,1H), 7.71 (m,2H), 7.32 (m,2H), 7.22 (t,1H), 6.94 (d,1H), 4.94 (m,1H), 4.0 (bs,2H), 2.90 (bs,2H), 2.43 (bs,8H), 2.29 (s,6H), 2.20 (s,3H), 1.80 (m,5H), 1.26 (m,2H), 0.92 (t,3H);
$t_R$: 2.66 min; MS (ESI): *m*/*z* $(M+H)^+$ 609, $(M+H)^-$ 607.

(3*Z*)-3-[(3,5-Dimethyl-4-{[4-(4-methylpiperazin-1-yl)piperidin-1-yl]carbonyl}-1*H*-pyrrol-2-yl)methylene]-2-oxo-*N*-[(1*R*)-1-phenylpropyl]indoline-5-carboxamide

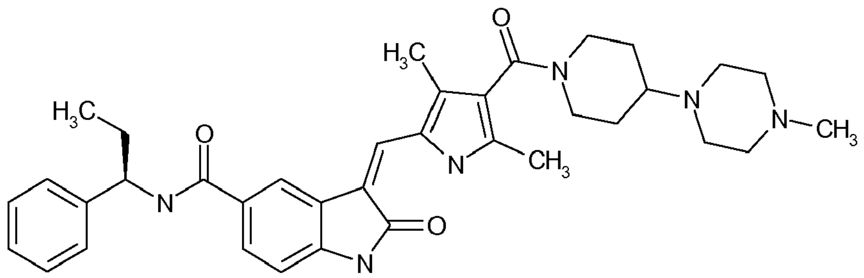

**53**

Compound 53 (136 mg, 44%) was obtained starting from (3Z)-3-[(3,5-dimethyl-4-{[4-(4-methylpiperazin-1-yl)piperidin-1-yl]carbonyl}-1*H*-pyrrol-2-yl)methylene]-2-oxoindoline-5-carboxylic acid and (1*R*)-1-phenylpropan-1-amine:

NMR (300 MHz, DMSO-$d_6$) δ ppm: 13.53 (s,1H), 11.10 (s,1 H), 8.54 (d,1H), 8.22 (s,1H), 7.70 (m,2H), 7.42-7.22 (m,5H), 6.94 (d,1H), 4.0 (bs,2H), 2.90 (bs,2H), 2.42 (bs,8H), 2.28 (s,6H), 2.21 (s,3H), 1.86 (bs,5H), 1.27 (m,2H), 0.92 (t,3H);
$t_R$: 2.76 min; MS (ESI): *m/z* $(M+H)^+$ 609, $(M+H)^-$ 607.

Example 4: (3*Z*)-3-{[3,5-Dimethyl-4-(piperidin-1-ylcarbonyl)-1*H*-pyrrol-2-yl]methylene}-2-oxo-*N*-[(1*S*)-1-phenylethyl]indoline-5-carboxamide

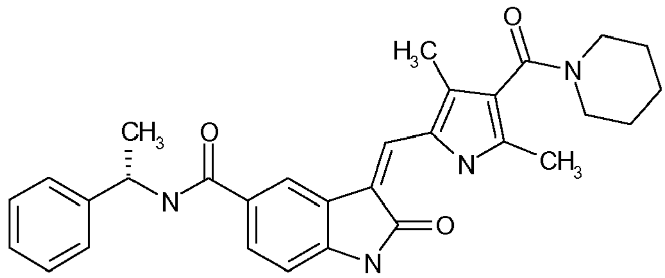

**54**

A mixture of (3*Z*)-3-{[3,5-dimethyl-4-(piperidin-1-ylcarbonyl)-1*H*-pyrrol-2-yl]methylene}-2-oxoindoline-5-carboxylic acid (1) (0.200 g, 0.51 mmol), HBTU (0.227 g, 0.60 mmol) and TEA (0.151 g, 1.5 mmol) in dry DMF (5 mL) was stirred for 0.5 h. After addition of (1*S*)-1-phenylethanamine (0.079 g, 0.65 mmol), the reaction mixture was stirred overnight. The reaction mixture was poured on ice-water and further stirred for 2 h. Resulting solid was collected by filtration, and purified by silica gel chromatography (eluent $CHCl_3$: MeOH : TEA 20 : 1: 0.1→10 : 1 : 0.1) to yield compound **54** (120 mg, 47%):

NMR (300 MHz, DMSO-$d_6$) δ ppm: 13.52 (s,1 H), 11.10 (s,1 H), 8.58 (d,1 H), 8.23 (s,1 H), 7.71 (d,1 H), 7.67 (s,1 H), 7.41 (m,2H), 7.33 (m,2H), 7.22 (t,1 H), 6.94 (d,1 H), 5.20 (m,1H), 3.44 (bs,4H), 2.29 (s,3H), 2.27 (s,3H), 1.60 (bs,2H), 1.51 (bs,4H), 1.50 (d.3H);
$t_R$: 3.90 min; MS (ESI): *m/z* $(M+H)^+$ 497, $(M+H)^-$ 495.

The following compounds were obtained according to the procedure described by **example 4:**

(3*Z*)-*N*-[(1*R*)-1-(3-Chlorophenyl)ethyl]-3-{[3,5-dimethyl-4-(piperidin-1-ylcarbonyl)-1*H*-pyrrol-2-yl]methylene}-2-oxoindoline-5-carboxamide

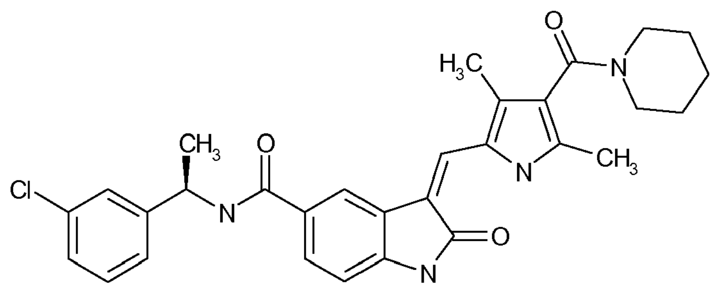

55

Compound 55 (85 mg, 31%) was obtained starting from (3*Z*)-3-{[3,5-dimethyl-4-(piperidin-1-ylcarbonyl)-1*H*-pyrrol-2-yl]methylene}-2-oxoindoline-5-carboxylic acid and (1*R*)-1-(3-chlorophenyl)ethanamine:

NMR (300 MHz, DMSO-$d_6$) δ ppm: 13.52 (s,1H), 11.10 (s,1 H), 8.65 (d,1H), 8.22 (s,1 H), 7.71 (d,1 H), 7.70 (s,1 H), 7.46 (s,1 H), 7.35 (m,2H), 7.29 (t,1 H), 6.95 (d,1 H), 5.17 (m,1H), 3.44 (bs,4H), 2.29 (s,3H), 2.28 (s,3H), 1.60 (bs,2H), 1.51 (bs,4H), 1.49 (d,3H);
$t_R$: 3.90 min; MS (ESI): *m/z* $(M+H)^+$ 531; $(M+H)^-$ 529.

(3*Z*)-*N*-[(1*S*)-1-(3-Chlorophenyl)ethyl]-3-{[3,5-dimethyl-4-(piperidin-1-ylcarbonyl)-1 *H*-pyrrol-2-yl]methylene}-2-oxoindoline-5-carboxamide

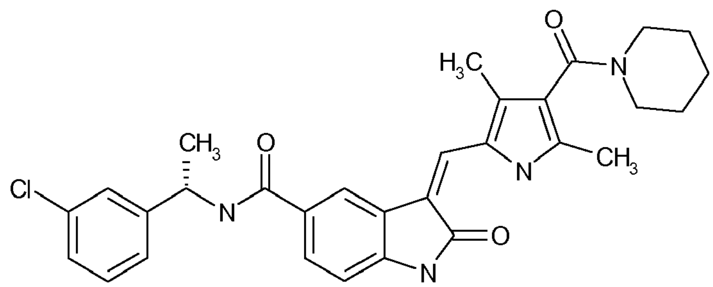

56

Compound **56** (90 mg, 33%) was obtained from (3*Z*)-3-{[3,5-dimethyl-4-(piperidin-1-ylcarbonyl)-1*H*-pyrrol-2-yl]methylene}-2-oxoindoline-5-carboxylic acid and (1*S*)-1-(3-chlorophenyl)ethanamine:

NMR (300 MHz, DMSO-$d_6$) δ ppm: 13.52 (s,1H), 11.12 (s,1H), 8.65 (d,1H), 8.22 (s,1 H), 7.70 (d,1 H), 7.68 (s,1 H), 7.46 (s,1 H), 7.36 (m,2H), 7.29 (t,1 H), 6.95 (d,1 H), 5.17 (m,1H), 3.44 (bs,4H), 2.29 (s,3H), 2.28 (s,3H), 1.60 (bs,2H), 1.51 (bs,4H), 1.50 (d,3H);
$t_R$: 4.17 min; MS (ESI): *m/z*- $(M+H)^+$ 531; $(M+H)^-$ 529.

(3*Z*)-3-{[3,5-Dimethyl-4-(piperidin-1-ylcarbonyl)-1*H*-pyrrol-2-yl]methylene}-2-oxo-*N*-[(1*S*)-1-phenylpropyl]indoline-5-carboxamide

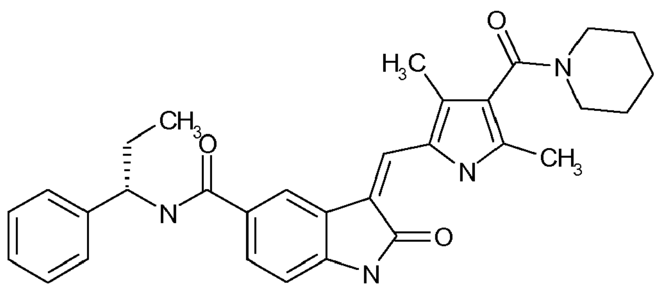

**57**

Compound 57 (95 mg, 36%) was obtained from (3*Z*)-3-{[3,5-dimethyl-4-(piperidin-1-ylcarbonyl)-1*H*-pyrrol-2-yl]methylene}-2-oxoindoline-5-carboxylic acid and (1*S*)-1-phenylpropan-1-amine:

NMR (300 MHz, DMSO-$d_6$) δ ppm: 13.53 (s,1H), 11.10 (s,1 H), 8.53 (d,1H), 8.21 (s,1 H), 7.70 (d,1 H), 7.68 (s,1 H), 7.41 (m,2H), 7.32 (m,2H), 7.22 (t,1 H), 6.94 (d,1 H), 4.94 (m,1H), 3.42 (bs,4H), 2.29 (s,3H), 2.28 (s,3H), 1.84 (m,2H), 1.60 (bs,2H), 1.47 (bs,4H), 0.92 (t,3H);
$t_R$: 4.07 min; MS (ESI): *m/z* $(M+H)^+$ 511; $(M+H)^-$ 509.

(3*Z*)-3-{4-[(4-Methylpiperazin-1-yl)carbonyl]benzylidene}-2-oxo-*N*-[(1*S*)-1-phenylpropyl]indoline-5-carboxamide

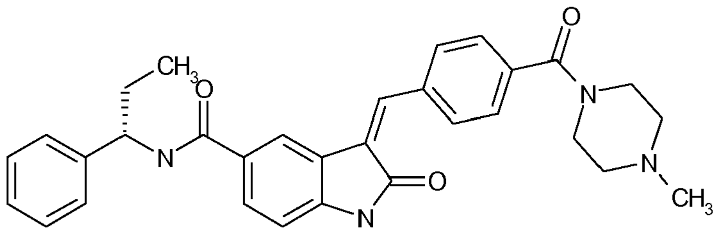

**58**

Compound **58** (120 mg, 46%) was obtained starting from (3*Z*)-3-{4-[(4-methylpiperazin-1-yl)carbonyl]benzylidene}-2-oxoindoline-5-carboxylic acid and (1*S*)-1-phenylpropan-1-amine:

NMR (300 MHz, DMSO-$d_6$) δ ppm: 11.90 (bs,1H), 8.56 (d,1H), 7.84 (d,2H), 8.07 (s,1H), 7.71 (s,1H), 7.31 (m,5H), 6.93 (d,1H), 4.82 (m,1H), 3.55 (bs,2H), 3.45 (bs,2H), 2.30 (bs,4H), 2.18 (s,3H), 2.45 (m,2H), 0.85 (t,3H);
$t_R$: 2.63 min; MS (ESI): *m/z* $(M+H)^+$ 509; $(M+H)^-$ 507.

(3*Z*)-3-[(4-{[2-(Diethylamino)ethyl]carbamoyl}-3,5-dimethyl-1*H*-pyrrol-2-yl)methylene]-2-oxo-*N*-[(1*R*)-1-phenylethyl]indoline-5-carboxamide

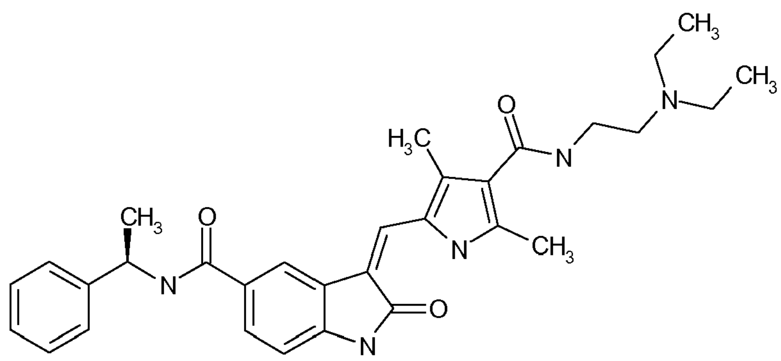

**59**

Compound **59** (95 mg, 35%) was obtained starting from (3*Z*)-3-[(4-{[2-(diethylamino)ethyl]carbamoyl}-3,5-dimethyl-1*H*-pyrrol-2-yl)methylene]-2-oxoindoline-5-carboxylic acid and (1*R*)-1-phenylethanamine:

NMR (300 MHz, DMSO-$d_6$) δ ppm: 13.61 (s,1H), 11.13 (s,1H), 8.60 (d,1H), 8.26 (s,1H), 7.72 (d,1H), 7.71 (s,1H), 7.53 (bs,1H), 7.42 (m,2H), 7.33 (m,2H), 7.22 (t,1H), 6.94 (d,1H), 5.20 (m,1H), 3.32 (bs,4H), 2.67 (bs,4H), 2.46 (s,3H), 3.45 (s,3H), 1.51 (d,3H), 1.04 (t,6H);
$t_R$: 2.91 min; MS (ESI): *m/z* $(M+H)^+$ 528; $(M+H)^-$ 526.

(3*Z*)-3-[(4-{[2-(Diethylamino)ethyl]carbamoyl}-3,5-dimethyl-1*H*-pyrrol-2-yl)methylene]-2-oxo-*N*-[(1*S*)-1-phenylethyl]indoline-5-carboxamide

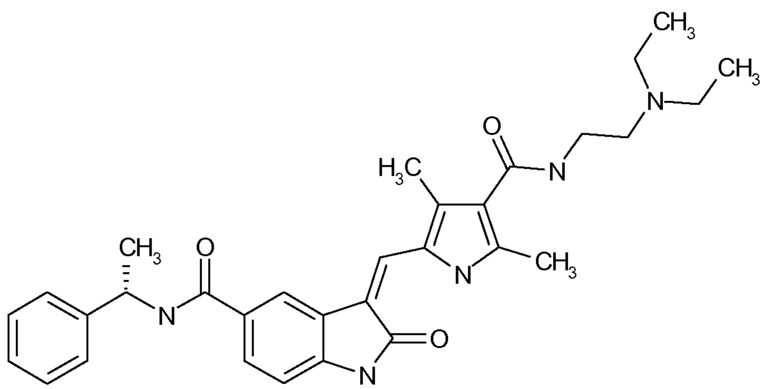

**60**

Compound **60** (75 mg, 28%) was obtained starting from: (3*Z*)-3-[(4-{[2-(diethylamino)ethyl]carbamoyl}-3,5-dimethyl-1*H*-pyrrol-2-yl)methylene]-2-oxoindoline-5-carboxylic acid and (1*S*)-1-phenylethanamine:

NMR (300 MHz, DMSO-$d_6$) δ ppm: 13.59 (s,1H), 11.12 (s,1H), 8.60 (d,1H), 8.25s,1H), 7.71 (d,1H), 7.70 (s,1H), 7.46 (bs,1H), 7.41 (m,2H), 7.33 (m,2H), 7.22 (t,1H), 6.94 (d,1H), 5.20 (m,1H), 3.33 (bs,4H), 2.57 (bs,4H), 2.45 (s,3H), 2.44 (s,3H), 1.51 (d,3H), 1.00 (t,6H);
$t_R$: 2.91 min; MS (ESI): *m/z* $(M+H)^+$ 528; $(M+H)^-$ 526.

(3*Z*)-3-[(4-{[2-(Diethylamino)ethyl]carbamoyl}-3,5-dimethyl-1*H*-pyrrol-2-yl)methylene]-2-oxo-*N*-[(1R)-1-phenylpropyl]indoline-5-carboxamide

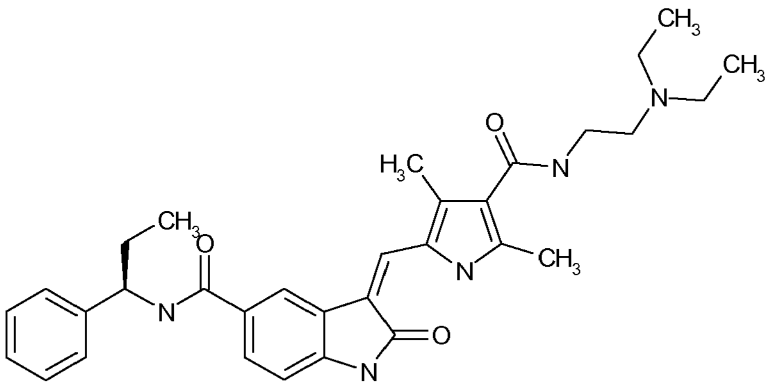

**61**

Compound **61** (100 mg, 36%) was obtained starting from: (3*Z*)-3-[(4-{[2-(diethylamino)ethyl]carbamoyl}-3,5-dimethyl-1*H*-pyrrol-2-yl)methylene]-2-oxoindoline-5-carboxylic acid and (1*R*)-1-phenylpropan-1-amine:

NMR (300 MHz, DMSO-$d_6$) δ ppm: 13.60 (s,1H), 11.13 (s,1 H), 8.55 (d,1H), 8.22 (s,1 H), 7.71 (s,1 H), 7.69 (d,1 H), 7.42 (m,3H), 7.32 (m,2H), 7.22 (t,1 H), 6.94 (d,1 H), 4.94 (m,1H), 3.27 (m,4H), 2.52 (bs,4H), 2.44 (s,6H), 1.84 (m,2H), 0.95 (m,9H);
$t_R$: 3.10 min; MS (ESI): *m*/*z* $(M+H)^+$ 542; $(M+H)^-$ 540.

(3*Z*)-3-[(4-{[2-(Diethylamino)ethyl]carbamoyl}-3,5-dimethyl-1*H*-pyrrol-2-yl)methylene]-2-oxo-*N*-[(1*S*)-1-phenylpropyl]indoline-5-carboxamide

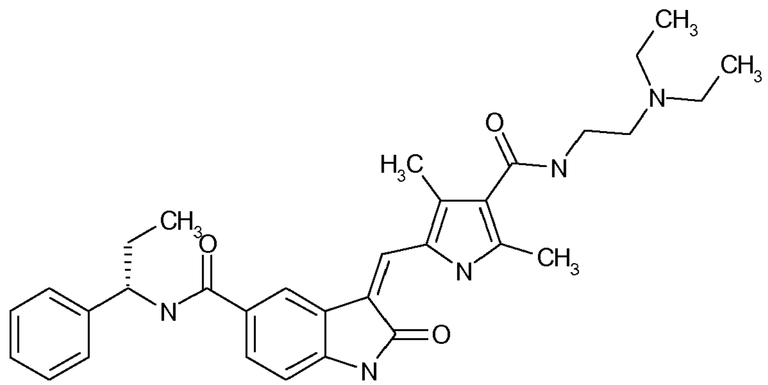

**62**

Compound **62** (130 mg, 47%) was obtained starting from (3*Z*)-3-[(4-{[2-(diethylamino)ethyl]carbamoyl}-3,5-dimethyl-1*H*-pyrrol-2-yl)methylene]-2-oxoindoline-5-carboxylic acid and (1*S*)-1-phenylpropan-1-amine:

NMR (300 MHz, DMSO-$d_6$) δ ppm: 13.61 (s,1H), 11.12 (s,1H), 8.50 (d,1H), 8.25 (s,1H), 7.72 (s,1H), 7.70 (d,1H), 7.51 (bs,1H), 7.42 (m,2H), 7.32 (m,2H), 7.22 (t,1H), 6.94 (d,1H), 4.94 (m,1H), 3.40 (bs,4H), 2.70 (bs,4H), 2.46 (s,6H), 1.86 (m,2H), 1.05 (bs,6H), 0.92 (t,3H);
$t_R$: 3.09 min; MS (ESI): *m*/*z* $(M+H)^+$ 542; $(M+H)^-$ 540.

(3*Z*)-3-{[3,5-Dimethyl-4-(piperidin-1-ylcarbonyl)-1*H*-pyrrol-2-yl]methylene}-2-oxo-*N*-[(1*R*)-1-phenylpropyl]indoline-5-carboxamide

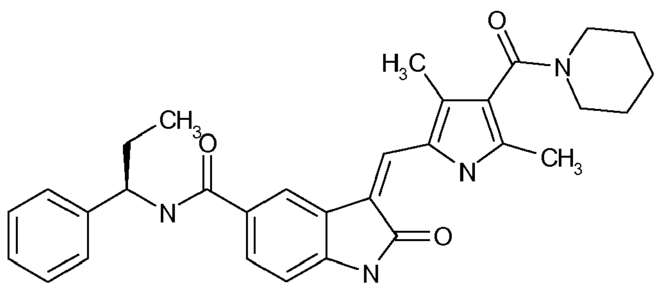

**63**

Compound **63** (100 mg, 38%) was obtained starting from (3*Z*)-3-{[3,5-dimethyl-4-(piperidin-1-ylcarbonyl)-1*H*-pyrrol-2-yl]methylene}-2-oxoindoline-5-carboxylic acid and (1*R*)-1-phenylpropan-1-amine:

NMR (300 MHz, DMSO-$d_6$) δ ppm: 13.53 (s,1H), 11.10 (s,1 H), 8.53 (d,1H), 8.21 (s,1 H), 7.70 (d,1 H), 7.68 (s,1 H), 7.41 (m,2H), 7.32 (m,2H), 7.22 (t,1 H), 6.94 (d,1 H), 3.43 (bs,4H), 2.29 (s,3H), 2.28 (s,3H), 1.84 (m,2H), 1.60 (bs,2H), 1.48 (bs,4H), 0.92 (t,3H);
$t_R$: 4.06 min; MS (ESI): *m/z* $(M+H)^+$ 511; $(M+H)^-$ 509.

(3*Z*)-3-{[3,5-Dimethyl-4-(piperidin-1-ylcarbonyl)-1*H*-pyrrol-2-yl]methylene}-*N*-[(1S)-1-(4-fluorophenyl)ethyl]-2-oxoindoline-5-carboxamide

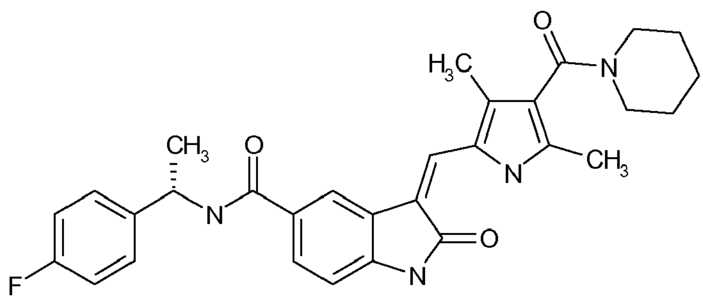

**64**

Compound **64** (171 mg, 65%) was obtaine starting from (3*Z*)-3-{[3,5-dimethyl-4-(piperidin-1-ylcarbonyl)-1*H*-pyrrol-2-yl]methylene}-2-oxoindoline-5-carboxylic acid and (1*S*)-1-(4-fluorophenyl)ethanamine:

NMR (300 MHz, DMSO-$d_6$) δ ppm: 13.52 (s,1H), 11.11 (s,1H), 8.60 (d,1H), 8.22 (s,1H), 7.70 (d,1H), 7.68 (s,1H), 7.44 (m,2H), 7.14 (m,2H), 6.93 (d,1H), 5.19 (m,1H), 3.42 (bs,4H), 2.29 (s,3H), 2.28 (s,3H), 1.60 (bs,2H), 1.51 (bs,4H), 1.49 (d,3H);
$t_R$: 3.94 min; MS (ESI): *m/z* $(M+H)^+$ 515; $(M+H)^-$ 513.

(3*Z*)-3-{[3,5-Dimethyl-4-(piperidin-1-ylcarbonyl)-1*H*-pyrrol-2-yl]methylene}-*N*-[(1*S*)-1-(4-methoxyphenyl)ethyl]-2-oxoindoline-5-carboxamide

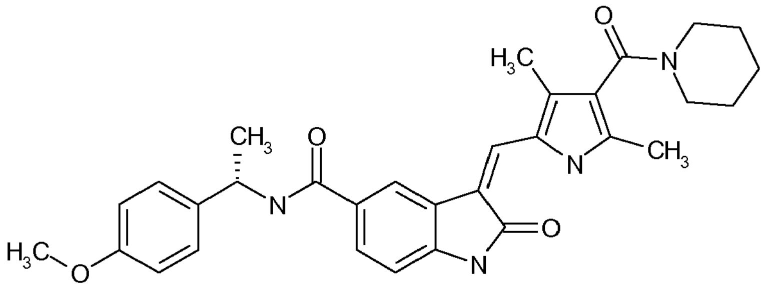

**65**

Compound **65** (85 mg, 32%) was obatined starting from (3*Z*)-3-{[3,5-dimethyl-4-(piperidin-1-ylcarbonyl)-1*H*-pyrrol-2-yl]methylene}-2-oxoindoline-5-carboxylic acid and (1*S*)-1-(4-methoxyphenyl)ethanamine:

NMR (300 MHz, DMSO-$d_6$) δ ppm: 13,52 (s,1H), 11.10 (s,1 H), 8.51 (d,1H), 8.21 (s,1H), 7.70 (d,1H), 7.67 (s,1H), 7.33 (d,2H), 6.91 (t,1H), 6.88 (d,2H), 5.16 (m,1H), 3.72 (s,3H), 3.43 (bs,4H), 2.29 (s,3H), 2.28 (s,3H), 1.60 (bs,2H),1.48 (bs,4H), 1.47 (d,3H);
$t_R$: 3.85 min; MS (ESI): *m/z* $(M+H)^+$ 527; $(M+H)^-$ 525.

(3*Z*)-*N*-[(1*R*)-1-(4-Chlorophenyl)ethyl]-3-{[3,5-dimethyl-4-(piperidin-1-ylcarbonyl)-1*H*-pyrrol-2-yl]methylene}-2-oxoindoline-5-carboxamide

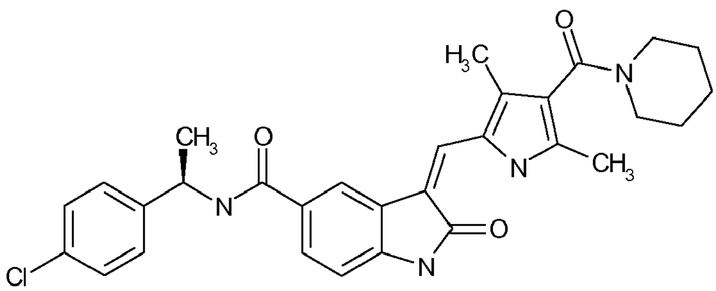

**66**

Compound **66** (114 mg, 43%) was obtained starting from: (3*Z*)-3-{[3,5-dimethyl-4-(piperidin-1-ylcarbonyl)-1*H*-pyrrol-2-yl]methylene}-2-oxoindoline-5-carboxylic acid and (1*R*)-1-(4-chlorophenyl)ethanamine:

NMR (300 MHz, DMSO-$d_6$) δ ppm: 13.52 (s,1 H), 11.11 (s,1H), 8.62 (d,1 H), 8.22 (s,1H), 7.71 (m,2H), 7.39 (dd,4H), 6.94 (d,1H), 5.17 (m,1H), 3.45 (bs,4H), 2.28 (s,6H), 2.08 (s,3H), 1.60 (bs,2H), 1.59 (bs,7H);
$t_R$: 4.17 min; MS (ESI): *m/z* $(M+H)^+$ 531; $(M+H)^-$ 529.
(3*Z*)-*N*-[(1*R*)-1-(4-chlorophenyl)ethyl]-3-{[3,5-dimethyl-4-(pyrrolidin-1-ylcarbonyl)-1*H*-pyrrol-2-yl]methylene}-2-oxoindoline-5-carboxamide

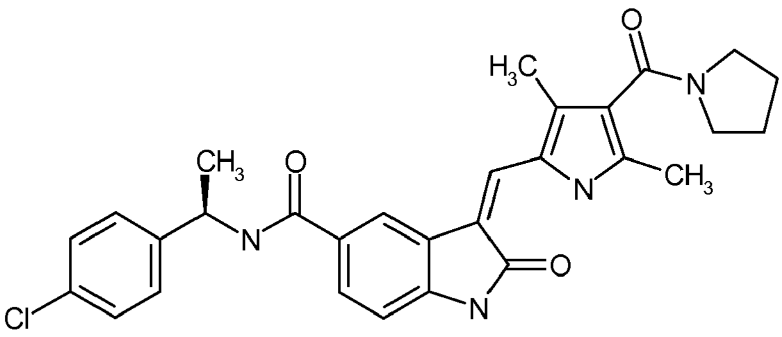

**67**

Compound 67 (105 mg, 40%) was obtained starting from ((3*Z*)-3-{[3,5-dimethyl-4-(pyrrolidin-1-ylcarbonyl)-1*H*-pyrrol-2-yl]methylene}-2-oxoindoline-5-carboxylic acid and (1R)-1-(4-chlorophenyl)-ethanamine:

NMR (300 MHz, DMSO-$d_6$) δ ppm: 13.50(s,1H); 11.10(s,1H); 8.62(d,1H); 8.22(s,1H); 7.70(d,1H); 7.69(s,1H); 7.41 (dd,4H); 6.96(d,1H); 5.18(m,1H); 3.50(bs,2H); 3.25(bs,2H); 2.30(s,6H); 1.84(bs,4H); 1.50(d.3H) $t_R$: 3.93 min; MS (ESI): *m/z* $(M+H)^+$ 517, $(M+H)^-$ 515.

Example 5:

**Step** A: (3*Z*)-1-Acetyl-3-(ethoxymethylene)-2-oxoindoline-5-carboxylic acid

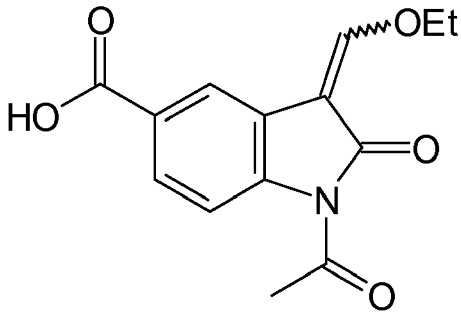

A mixture of 2-oxoindoline-5-carboxylic acid (5.221 g, 29.50 mmol) and triethylorthoformate (13.320 g, 90 mmol) in acetic anhydride (75 mL) were stirred at 100 °C for 8 h. The solvent was removed under reduced pressure. The residue was stirred with i-$Pr_2O$ (250 mL) for 2 h to give a solid, which was filtered and washed with i-$Pr_2O$ to provide (3*Z*)-1-acetyl-3-(ethoxymethylene)-2-oxoindoline-5-carboxylic acid (4.8 g, 59%):

NMR (300 MHz, DMSO-d6) δ ppm: 12.6 (bs,1H), 8.17 (d,1H), 8.15 (s,1H), 8.05 (d,1H), 7.87 (dd,1H), 4.54 (q,2H), 2.62 (s,3H), 1.41 (t,3H);
$t_R$: 3.17 min; MS (ESI): *m/z* $(M+H)^+$ 276; $(M+H)^-$ 274.

**Step B:** (3*Z*)-2-Oxo-3-({[4-(piperidin-1-ylmethyl)phenyl]amino}methylene) indoline-5-carboxylic acid (68)

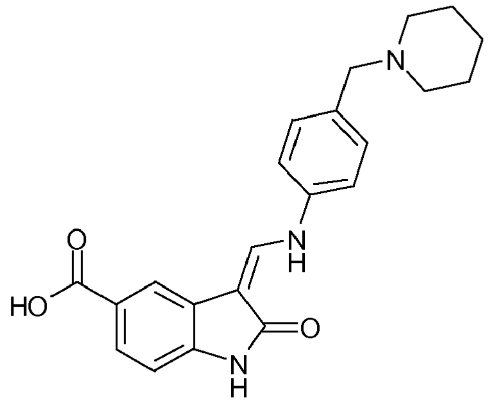

**68**

A mixture of compound (3*Z*)-1-acetyl-3-(ethoxymethylene)-2-oxoindoline-5-carboxylic acid (3.407 g, 12.39 mmol) and 4-(piperidin-1-ylmethyl)aniline (2.47 g, 13 mmol) in dry DMF (10 mL) were stirred at 100 °C for 6 h. After cooling down the reaction mixture, MeOH (70 mL) and NaOMe (30% wt solution in MeOH, 5 mL) were added and the reaction mixture was further stirred overnight. The resulting solid was filtered and washed with cold MeOH to provide target compound 68 (2.40 g, 51 %):

NMR (300 MHz, DMSO-d6) δ ppm: 14.0 (bs,1H), 10.70 (d,1H), 10.44 (s,1H), 8.60 (d,1H), 8.13 (s,1H), 7.628 (d,1H), 7.34 (d,2H), 7.26 (d,2H), 6.71 (d,1H), 3.37 (s,2H), 2.31 (bs,4H), 1.49 (bs,4H), 1.39 (bs,2H);
$t_R$: 0.46, 2.21 min; MS (ESI): *m*/*z* $(M+H)^+$ 378; $(M+H)^-$ 376.

**<u>Step C</u>:** (3*Z*)-2-Oxo-*N*-[(1*R*)-1-phenylpropyl]-3-({[4-(piperidin-1-ylmethyl)phenyl] amino}methylene)-indoline-5-carboxamide (69)

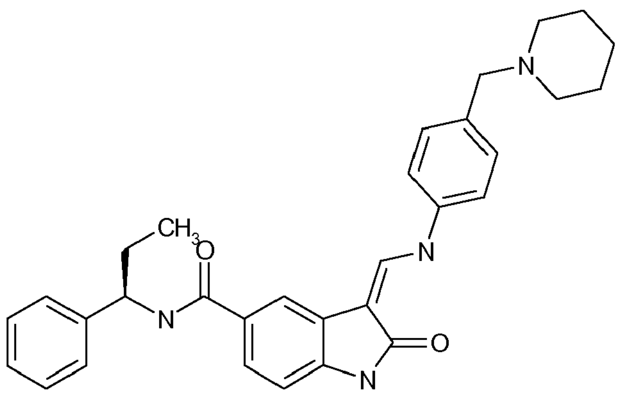

**69**

A solution of carboxylic acid 68 (0.150 g, 0.4 mmol), EDCI (0.107 g, 0.56 mmol), HOBT (0.085 g, 0.56 mmol), DIEA (0.184 g, 1.43 mmol) and (1*R*)-1-phenylpropan-1-amine (54 mg, 0.4 mmol) in dry DMF (5 mL) were stirred overnight. The reaction mixture was poured on ice-water (180 mL) and extracted with EtOAc (3 x 70 mL). The combined organic layers were washed with brine, dried on $MgSO_4$, filtered and concentrated *in vacuo.* Purification by chromatography on silica gel (eluent: $CHCl_3$:MeOH:TEA = 20:1:0.1 trovided the target compound **69** (50 mg, 25%):

NMR (300 MHz, DMSO-$d_6$) δ ppm: 10.35 (s,1H), 9.65 (d,1H), 8.43 (d,1H), 8.10 (s,1H), 7.76 (d,1H), 7.60 (d.1H), 7.43-7.27 (m,8H), 7.22 (t,1H), 6.89 (d,1H), 4.94 (m,1H), 3.39 (s,2H), 2.31 (bs,4H), 1.83 (m,2H), 1.49 (bs,4H), 1.39 (bs,2H), 0.92 (t,3H);
$t_R$: 3.00 min; MS (ESI): *m*/*z* $(M+H)^+$ 495; $(M+H)^-$ 493.

The following compounds were obtained according to the procedure described by **example 5, step C:**

(3*Z*)-*N*-[(1R)-1- (4-Methoxyphenyl)ethyl]-2-oxo-3- ({[4-(piperidin-1-ylmethyl)phenyl]amino}methylene)indoline-5-carboxamide

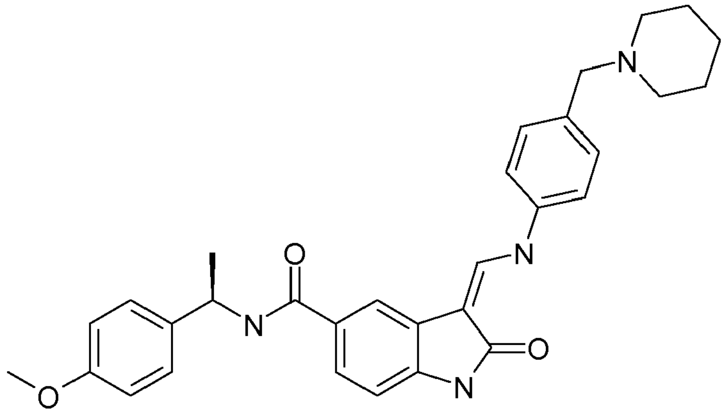

**70**

Compound **70** (55 mg, 28%) was obtained starting from (3*Z*)-2-oxo-3-({[4-(piperidin-1-ylmethyl)phenyl]amino}methylene) indoline-5-carboxylic acid and (1*R*)-1- (4-methoxyphenyl)ethanamine:

NMR (300 MHz, DMSO-$d_6$) δ ppm: 10.74 (d,1H), 10.39 (s,1H), 8.66 (d,1H), 8.45 (d.1H), 8.11 (s,1H), 7.59 (d,1H), 7.33 (m,6H), 6.89 (d,2H), 5.15 (m,1H), 3.72 (s,3H), 3,42 (bs,2H), 2.34 (bs,4H), 1.46 (bs,7H),1.39 (bs,2H); $t_R$: 2.91 min, MS (ESI): *m/z* $(M+H)^+$ 495; $(M+H)^-$ 493.

(3*Z*)-*N*-Methyl-2-oxo-*N*-[(1*S*)-1-phenylethyl]-3-({[4-(piperidin-1-ylmethyl)phenyl]amino}methylene)indoline-5-carboxamide

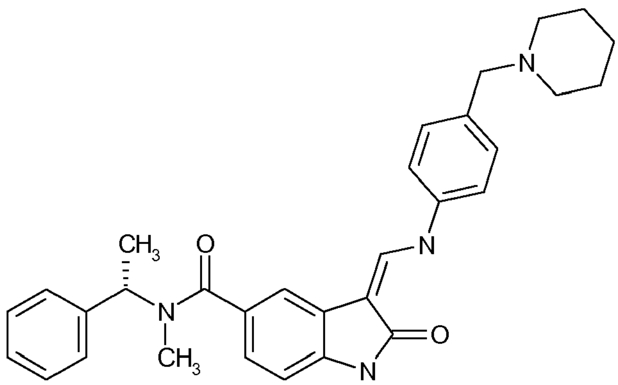

**71**

Compound 71 (80 mg, 40%) was obtained starting from (3*Z*)-2-oxo-3-({[4-(piperidin-1-ylmethyl)phenyl]amino}methylene) indoline-5-carboxylic acid and (1S)-N-methyl-1-phenylethanamine:

NMR (300 MHz, DMSO-$d_6$) δ ppm: 10.74 (d,1H), 10.65 (s,1H), 8.74 (d,1H), 7.77 (s,1H), 7.39 (m,10H), 7.10 (d.1H), 6.91 (d,1H), 5.6 (bs,1H), 3.47 (bs,2H), 2.66 (s,3H), 2.39 (bs,4H), 1.58 (d,3H), 1.52 (bs,4H), 1.41 (bs,2H); $t_R$: 3.02 min; MS (ESI): *m/z* $(M+H)^+$ 495; $(M+H)^-$ 493.

(3*Z*)-*N*-[(1*R*)-1-(4-Methylphenyl)ethyl]-2-oxo-3-({[4-(piperidin-1-ylmethyl)phenyl]amino}methylene)indoline-5-carboxamide

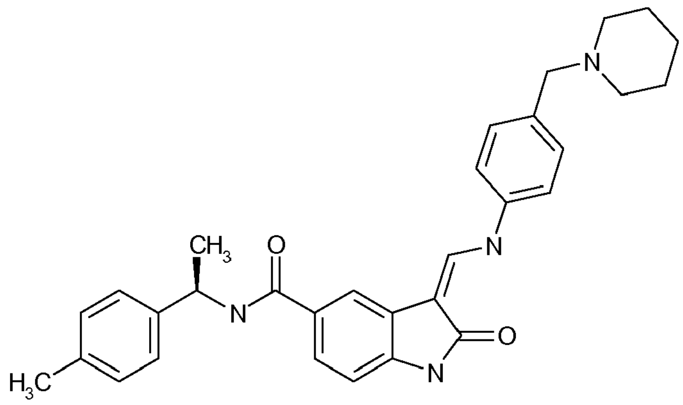

**72**

Compound **72** (53 mg, 27%) was obtained starting from (3*Z*)-2-oxo-3-({[4-(piperidin-1-ylmethyl)phenyl]amino}methylene) indoline-5-carboxylic acid and (1*R*)-1-(4-methylphenyl)ethanamine:

NMR (300 MHz, DMSO-$d_6$) δ ppm: 10.74 (d,1H), 10.72 (s,1H), 8.66 (d,1H), 8.11 (s,1H), 7.60 (d,1H), 7.38 (d,2H), 7.29 (d,4H), 7.13 (d,2H), 6.88 (d,1H), 5.15 (m,1H), 3.41 (bs,2H), 2.34 (bs,4H), 2.27 (s,3H), 1.47 (bs,7H), 1.40 (bs,2H); $t_R$: 3.11 min; MS (ESI): *m/z* $(M+H)^+$ 495, $(M+H)^-$ 493.

(3*Z*)-*N*-[(1*R*)-1-(4-Chlorophenyl)ethyl]-2-oxo-3- ({[4-(piperidin-1-ylmethyl)phenyl]amino}methylene)indoline-5-carboxamide

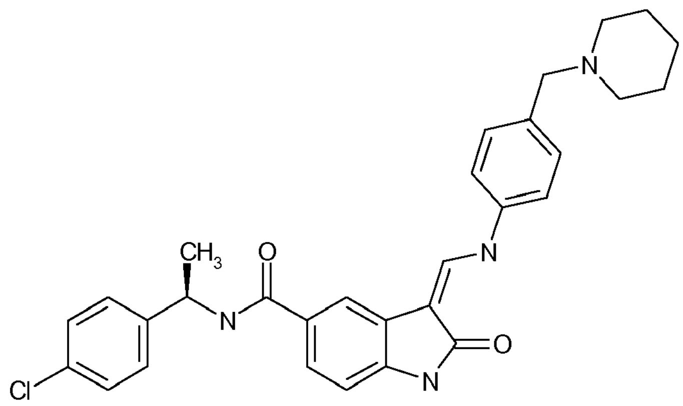

**73**

Compound **73** (70 mg, 34%) was obtained starting from (3*Z*)-2-oxo-3-({[4-(piperidin-1-ylmethyl)phenyl]amino}methylene) indoline-5-carboxylic acid and (1*R*)-1-(4-chlorophenyl)ethanamine:

NMR (300 MHz, DMSO-$d_6$) δ ppm: 10.74 (d,1H), 10.72 (s,1H), 8.65 (d,1H), 8.11 (s,1H), 7.60 (d,1H), 7.45-7.27 (m,8H), 6.89 (d,1H), 5.17 (m,1H), 3.39 (s,2H), 2.32 (bs,4H), 1.48 (bd,7H), 1.39 (bs,2H);
$t_R$: 3.15 min; MS (ESI): *m/z* $(M+H)^+$ 515, $(M+H)^-$ 513.

(3*Z*)-2-Oxo-*N*-[(1*S*)-1-phenylpropyl]-3-({[4-(piperidin-1-ylmethyl)phenyl]amino}methylene)indoline-5-carboxamide

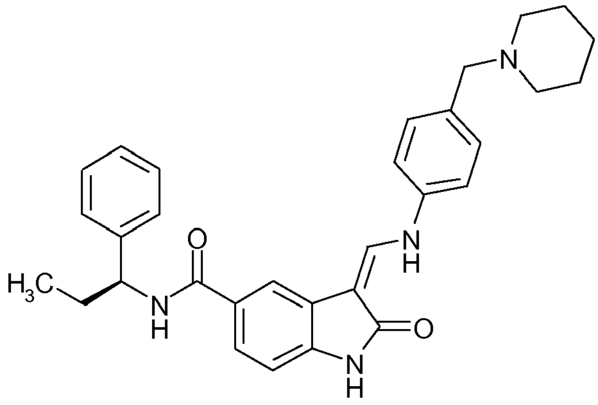

**74**

Compound **74** (70 mg, 35%) was obtained starting from (3*Z*)-2-oxo-3-({[4-(piperidin-1-ylmethyl)phenyl]amino}methylene) indoline-5-carboxylic acid and (1S)-1-phenylpropan-1-amine:

NMR (300 MHz, DMSO-$d_6$) δ ppm: 10.74 (d,1H), 10.71 (s,1H), 8.66 (d.1H), 8.44 (d,1 H), 8.11 (s,1 H), 7.60 (d,1 H), 7.42,7.21 (m,9H), 6.88 (d,1 H), 4.94 (m,1 H), 3.40 (bs,2H), 2.33 (bs,4H), 1.84 (m,2H), 1.49 (bs,4H), 1.39 (bs,2H), 0.92 (t,3H);
$t_R$: 3.01 min; MS (ESI): *m*/*z* $(M+H)^+$ 495, $(M+H)^-$ 493.

(3*Z*)-2-Oxo-*N*-[(1*S*)-1-phenylethyl]-3-({[4-(piperidin-1-ylmethyl)phenyl]amino}methylene)indoline-5-carboxamide

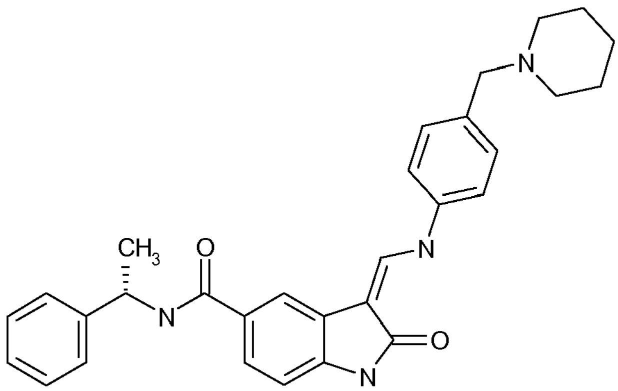

**75**

Compound **75** (70 mg, 36%) was obtained starting from (3*Z*)-2-oxo-3-({[4-(piperidin-1-ylmethyl)phenyl]amino}methylene) indoline-5-carboxylic acid and (1*S*)-1-phenylethanamine:

NMR (300 MHz, DMSO-$d_6$) δ ppm: 10.74 (d,1H), 10.73 (s,1H), 8.68 (d,1H), 8.51 (d,1H), 8.13 (s,1H), 7.61 (d,1H), 7.42 (m,4H), 7.32 (m,4H), 7.22 (t,1H), 6.89 (d,1H), 5.19 (m,1H), 3.49 (bs,2H), 2.40 (bs,4H), 1.52 (bs,4H), 1.49 (d,3H), 1.40 (bs,2H);
$t_R$: 2.90 min; MS (ESI): *m*/*z* $(M+H)^+$ 481, $(M+H)^-$ 479.

(3Z)-2-Oxo-3- ({[4-(piperidin-1-ylmethyl)phenyl]amino}methylene)-*N*-[(1*S*)-1,2,3,4-tetrahydronaphthalen-1-yl]indoline-5-carboxamide

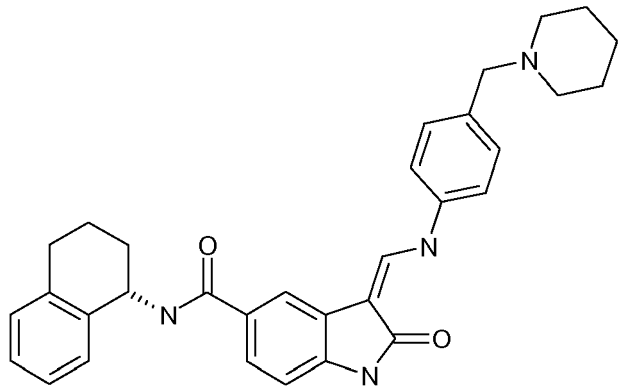

**76**

Compound **76** (95 mg, 47%) was obtained starting from (3*Z*)-2-oxo-3-({[4-(piperidin-1-ylmethyl)phenyl]amino}methylene) indoline-5-carboxylic acid and (1S)-1,2,3,4-tetrahydronaphthalen-1-amine:

NMR (300 MHz, DMSO-$d_6$) δ ppm: 10.72 (d,1H), 10.71 (s,1H), 8.64 (d,1H), 8.42 (s,1 H), 8.20 (s,1 H), 7.63 (d,1 H), 7.38-7.13 (m,10H), 6.87 (d,1 H), 5.27 (bs,1 H), 3.39 (s,2H), 2.78 (bs,2H), 2.52 (bs,1 H), 2.31 (bs,4H), 2.00 (m,2H), 1.83 (m,2H), 1.49 (bs,4H), 1.39 (bs,2H);
$t_R$: 3.09 min;, MS (ESI): *m/z* $(M+H)^+$ 507, $(M+H)^-$ 505.

(3*Z*)-*N*-[(1*R*)-1-Cyclohexylethyl]-2-oxo-3-({[4-(piperidin-1-ylmethyl)phenyl]amino}methylene)indoline-5-carboxamide

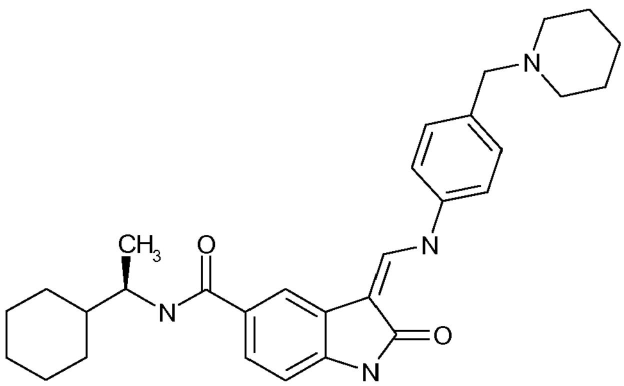

**77**

Compound **77** (70 mg, 36%) was obtained starting from (3*Z*)-2-oxo-3-({[4-(piperidin-1-ylmethyl)phenyl]amino}methylene) indoline-5-carboxylic acid and (1*R*)-1-cyclohexylethanamine:

NMR (300 MHz, DMSO-$d_6$) δ ppm: 10.74 (d,1H), 10.70 (s,1H), 8.66 (d,1H), 8.09 (s,1H), 7.80 (d,1H), 7.55 (d,1H), 7.36 (d,2H), 7.29 (d,2H), 6.86 (d,1H), 3.86 (m,1H), 3.41 (bs,2H), 2.33 (bs,4H), 1.77 (m,4H), 1.50 (bs,1H), 1.49 (bs,4H), 1.40 (bs,2H), 1.18 (bs,4H), 1.11 (d,3H), 1.02 (m,2H);
$t_R$: 3.18 min; MS (ESI): *m/z* $(M+H)^+$ 487, $(M+H)^-$ 485.

(3*Z*)-2-Oxo-*N*-[(1*R*)-1-phenylethyl]-3-({[4-(piperidin-1-ylmethyl)phenyl] amino}methylene)indoline-5-carboxamide

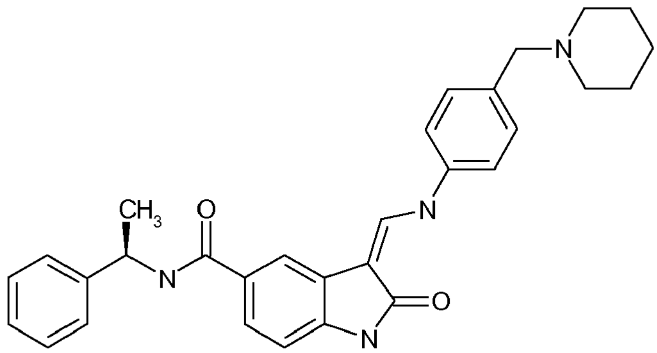

**78**

Compound 78 (35 mg, 18%) was obtained starting from (3*Z*)-2-oxo-3-({[4-(piperidin-1-ylmethyl)phenyl]amino}methylene) indoline-5-carboxylic acid and (1*R*)-1-phenylethanamine:

NMR (300 MHz, DMSO-$d_6$) δ ppm: 10.74 (d,1H), 10.71 (s,1H), 8.60 (d,1H), 8.49 (d,1H), 8.12 (s,1H), 7.61 (d,1H), 7.43-7.24 (m,8H), 7.22 (t,1H), 6.89 (d,1H), 5.19 (m,1H), 3.40 (s,2H), 2.32 (bs,4H), 1.49 (bs,9H), 1.40 (bs,2H); $t_R$: 2.84 min; MS (ESI): *m*/*z* $(M+H)^+$ 481, $(M+H)^-$ 479.

(3*Z*)-*N*-[(1*R*)-1- (3-Methoxyphenyl)ethyl]-2-oxo-3- ({[4-(piperidin-1-ylmethyl)phenyl]amino}methylene)indoline-5-carboxamide

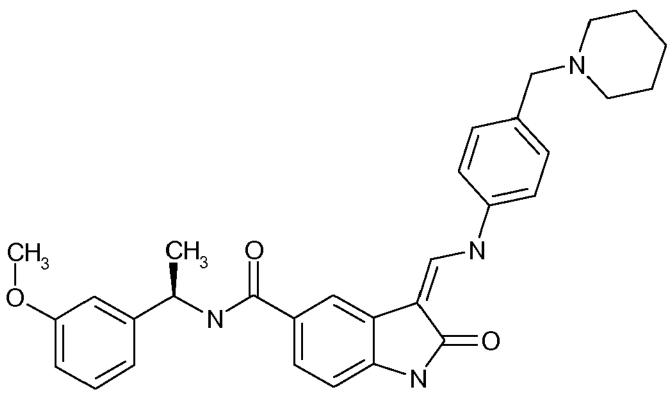

**79**

Compound **79** (40 mg, 19.5%) was obtained starting from (3*Z*)-2-oxo-3-({[4-(piperidin-1-ylmethyl)phenyl]amino}methylene) indoline-5-carboxylic acid and (1*R*)-1- (3-methoxyphenyl)ethanamine:

NMR (300 MHz, DMSO-$d_6$) δ ppm: 10.75 (d.1H), 10.71 (s,1H), 8.66 (d,1H), 8,49 (d,1H), 8.12 (s,1H), 7.61 (d,1H), 7.38-7.21 (m,5H), 6.98 (bs,2H), 6.89 (d,1H), 6.79 (d,1H), 5.20 (m,1H), 3.74 (s,3H), 3.40 (s,2H), 2.32 (bs,4H), 1.49 (s,9H), 1.40 (bs.2H);
$t_R$: 2.89 min; MS (ESI): *m*/*z* $(M+H)^+$ 511, $(M+H)^-$ 509.

(3*Z*)-*N*-[(1R)-1- (3-Chlorophenyl)ethyl]-2-oxo-3- ({[4-(piperidin-1-ylmethyl)phenyl]amino}methylene)indoline-5-carboxamide

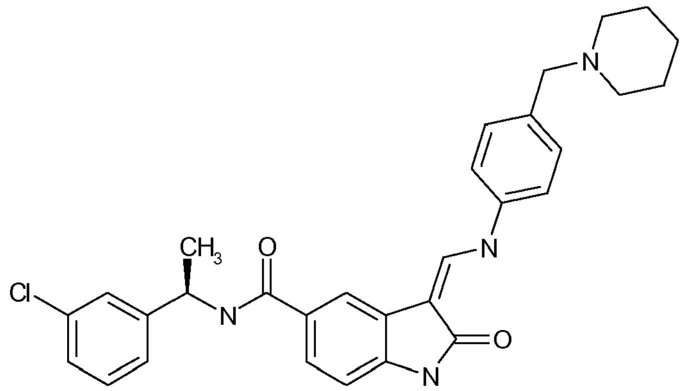

**80**

Compound **80** (30 mg, 14%) was obtained starting from (3*Z*)-2-oxo-3-({[4-(piperidin-1-ylmethyl)phenyl]amino}methylene) indoline-5-carboxylic acid and (1*R*)-1- (3-chlorophenyl)ethanamine:

NMR (300 MHz, DMSO-$d_6$) δ ppm: 10.74 (d,1H), 10.72 (s,1H), 8.68 (d,1H), 8.57 (d,1H), 8.12 (s,1H), 7.46-7.27 (m,8H), 6.89 (d,1H), 3.39 (s,2H), 2.32 (bs,4H), 1.49 (bs,4H), 1.39 (bs,2H), 1.23 (bs,3H);
$t_R$: 3.08 min, MS (ESI): *m*/*z* $(M+H)^+$ 515, $(M+H)^-$ 513.

**Part B: Biological evaluation**

**General cell culture techniques:**

Cell lines were routinely assayed for mycoplasma contamination and cultured at 95 % air, 5 % $CO_2$ and 37 °C in a Hera-Cell-150 incubator. Before seeding, the cell amount was determined using a Coulter Counter system (Coulter Electronics) and the corresponding cell amount for seeding was calculated. All cells were cultured according to the appropriate recommendations of ATCC protocols.

For analysis using the following assays, cells were seeded at different plate size and suitable cell-density. After 24 h the cells were washed with PBS and fresh media containing DMSO respectively the appropriate treatment with TKIs, peptides or chemicals, was replaced. Starving for insulin dependent examinations was performed in glucose- and FCS-free DMEM media for 4 h.

**Table 2:** Condition for the used cell lines:

| Cell line | Culture media | Initial cell amount 96-/ 12- / 6-well |
|---|---|---|
| 3T3-L1 | DMEM 1.0 g/L glucose, 10 % (v/v) NCS, 2 mM L-glutamine, 1x Pen/Strep | 5 x103 / 5 x104 / 1 x105 |
| Beta TC-6 | DMEM 4.5 g/L glucose, 15 % (v/v) FCS, 2 mM L-glutamine, 1x Pen/Strep | 3 x104 / 3 x105 / 6 x105 |
| C2C12 | DMEM 4.5 g/L glucose, 10 % (v/v) FCS, 2 mM L-glutamine, 1x Pen/Strep | 3 x103 / 3 x104 / 6 x104 |
| RIN-5AH-T2B | RPMI-1640 4.5 g/L glucose, 10 % (v/v) FCS, 2 mM L-glutamine, 1x Pen/Strep | 3x104 / 3 x105 / 6 x105 |

**Table 3:** Control compounds:

| Com | Structure | Com | Structure |
|---|---|---|---|
| **c1** | 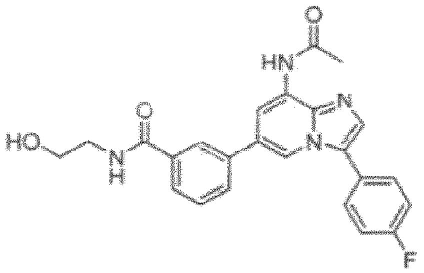 | **c3** | 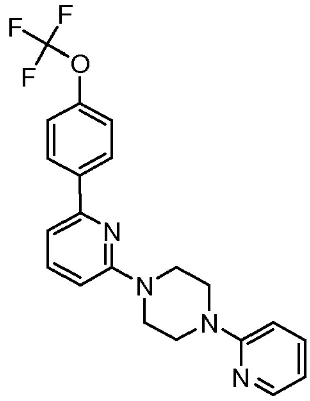 |
| **c2** | 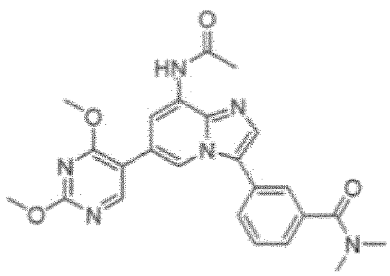 | **c4** | 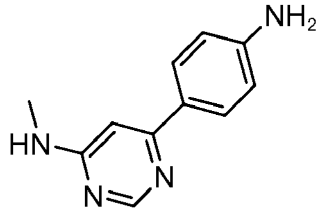 |
| Sunitinib (Sut, Sutent) | 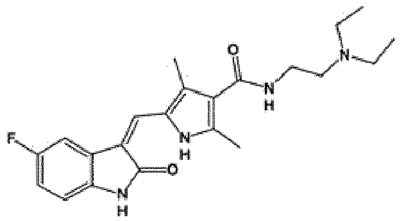 | Akt1 | 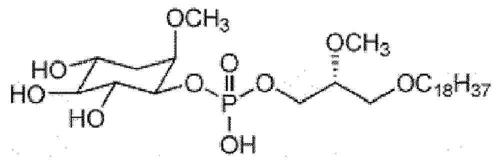 |
| Exendin -4 | His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-$NH_2$ | GLP-1 | His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-$NH_2$ |
| MEK | 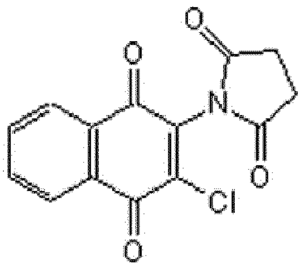 | | "Com" refers to "Compound" |

## Example 6: Cytotoxicity Assays

Using a MTT (3-(4,5-dimethylthiazol-2-yl)-2,5-diphenyl tetrazolium bromide) approach, the potential citotoxic effect of the compounds of the present invention on the viability and proliferation of the beta-TC6 and C2C12 cells was determined *in vitro* after a 72 h treatment.

For MTT transformation, 1/5 of total volume of a 5 mg/mL MTT stock solution was added to the cells as well as control wells and incubated at 37 °C, 5 % $CO_2$ (v/v) for 1 h. Then 1/2 of total volume of MTT-stop solution was added

and plates were incubated overnight in the dark at 25 °C. The optical density (OD) was measured using a multiwell spectrophotometer at a wavelength of 570 nm.

**<u>Example 7:</u> High throughput screening based on GRK5 kinase activity assay**

Screening for possible inhibitory compounds of GRK5 was performed using the *ADP Glo™ Kinase Assay technology* (Promega) according to the protocol as describe below. As suitable substrate for full length GRK5 casein was identified. Increasing concentrations of GRK5 are incubated together with 100 μM ATP in the absence and presence of 10 μM casein in a reaction buffer. Within this incubation non-phosphorylated casein is converted into phosphorylated casein and ATP is converted into ADP. Thereafter nonconverted ATP is digested into AMP by addition of the ADP-Glo™ Reagent. Subsequently the ADP that is produced by GRK5 activity is phosphorylated back to ATP by addition of Promega Kinase Detection Reagent. These ATP levels serve as measure for the GRK5 activity and are quantified by a luciferase/luciferin reaction. A linear dependence between GRK5 concentration and luminescence signal can be observed. In the absence of casein increasing GRK5 concentration result only in a minor increase of luminescence demonstrating the specificity of the luminescence signal for GRK5 activity.

Km(ATP) was determined by measuring the GRK5 activity at increasing ATP concentrations. Km(ATP) was quantified to be 18.7 μM by fitting the GRK5 activity to the Michaelis Menten equation :

$$v\ (\text{GRK5 activity}) = (\text{Vmax} \times [\text{ATP}]) / (\text{Km(ATP)} + [\text{ATP}])$$

For GRK5 kinase activity assay, the following protocol was used:

The assay was performed in: 384 well U bottom, PP, black, low volume (Corning, 3676) assay plates at reaction temperature of 25°C. The reaction buffer used was composed of: 20 mM MES pH 6.0, 1 mM DTT, 10 mM $MgCl_2$, 0.01 % Tween20 and the reaction volume was 6μL.

Firstly 4 μL 6/4 fold concentrated substrate and 6/4 fold concentrated ATP in 1 fold concentrated reaction buffer are added to each well of the assay plate. Subsequently 67 nL 1000 fold concentrated test compound in 100 % DMSO are added to each well except to C- and C+ wells using pin tool. Then 67 nL 100 % DMSO are pipetted to C- (no kinase, no compound) and C+ (no compound) wells using pin-tool and 2 μl reaction buffer are added to C- wells. 2 μl 6/2 fold concentrated full length GRK5 (Millipore, #14-714) in reaction buffer is added to each well except C- wells. After incubation for 120 min. at room temperature 6 μL ADP-Glo™ Reagent (ADP Glo™ Kinase Assay Kit: Promega, V9101) are added to each well to stop the kinase reaction and deplete the unconsumed ATP. After a second incubation for 40 min. at room temperature 12 μlL of Kinase Detection Reagent are added to convert ADP to ATP and start luciferase / luciferin reaction for detection of ATP. The final assay concentrations are 20 nM GRK5, 18.7 μM ATP and 10 μM of the substrate casein. Finally, after incubation for 40 min at room temperature the luminescence intensity was measured.

**<u>Example 8.1</u>: $IC_{50}$ determination of compounds inhibiting GRK5 by ADP Glo™ Kinase Assay technology (Promega) (Table 4)**

In order to determine the $IC_{50}$ values for all compounds ≥ 35 % inhibition, triplicates at 12 different concentrations with a dilution factor of three were used. The maximal compound assay concentration was set to 100 μM, representing the compound assay concentration used in the primary screen. For all assay plates, z-factor values were found to be significantly larger than 0.5, proving statistical relevance of the data.

The activity of the compounds was classified according to $IC_{50}$ for binding sites of Grk5 into the following ranges:

| | | |
|---|---|---|
| $IC_{50} \leq$ | 5 μM | ++++ |
| 5 μM < $IC_{50} \leq$ | 20 μM | +++ |
| 20 μM < $IC_{50} \leq$ | 30 μM | ++ |
| $IC_{50} >$ | 30 μM | + |

**<u>Table 4:</u>**

| Compound | GRK5 $IC_{50}$ [μM] |
|---|---|
| 2 | ++++ |
| 6 | ++++ |
| 7 | +++ |

(continued)

| Compound | GRK5 $IC_{50}$ [$\mu$M] |
|---|---|
| 10 | ++++ |
| 11 | ++++ |
| 12 | ++++ |
| 13 | ++++ |
| 14 | ++++ |
| 15 | ++++ |
| 21 | ++++ |
| 30 | +++ |
| 32 | ++++ |
| 46 | ++++ |
| 47 | +++ |
| 48 | ++++ |
| 51 | +++ |
| 52 | + |
| 53 | +++ |
| 54 | + |
| 55 | ++++ |
| 56 | + |
| 57 | + |
| 59 | ++++ |
| 60 | ++++ |
| 61 | ++++ |
| 62 | + |
| 63 | ++++ |
| 64 | + |
| 65 | ++++ |
| 67 | ++++ |
| 69 | ++++ |
| 70 | ++++ |
| 71 | ++++ |
| 72 | ++++ |
| 73 | ++++ |
| 78 | ++++ |
| 79 | ++++ |
| 80 | ++++ |

**<u>Example 8.2:</u> $IC_{50}$ determination of compounds inhibiting GRK5 by Millipore KinaseProfiler™ (Table 5)**

$IC_{50}$ values are determined by a radiometric based filtration binding assay, namely Millipore KinaseProfiler™. GRK5 is incubated with 8 mM MOPS pH 7.0, 0.2 mM EDTA, 2 mg/mL casein, 10 mM MgAcetate and [$\gamma$-$^{33}$P-APT] (specific activity approx. 500 cpm/pmol, concentration as required). The reaction is initiated by the addition of the MgATP mix. After incubation for 40 minutes at room temperature, the reaction is stopped by the addition of 3 % phosphoric acid solution. 10 $\mu$L of the reaction is then spotted onto a P30 filtermat and washed three times for 5 minutes in 75 mM phosphoric acid and once in methanol prior to drying and scintillation counting.

The activity of the compounds was classified according to $IC_{50}$ for binding sites of GRK5 into the following ranges:

| | | |
|---|---|---|
| $IC_{50} \leq$ | 5 $\mu$M | ++++ |
| 5 $\mu$M < $IC_{50} \leq$ | 20 $\mu$M | +++ |
| 20 $\mu$M < $IC_{50} \leq$ | 30 $\mu$M | ++ |
| $IC_{50}$ > | 30 $\mu$M | + |

**Table 5:**

| Compound | GRK5 $IC_{50}$ [$\mu$M] |
|---|---|
| 12 | ++++ |
| 16 | ++++ |
| 30 | +++ |
| 46 | ++++ |
| 47 | +++ |
| 48 | ++++ |
| 52 | + |
| 53 | ++++ |
| 54 | + |
| 55 | ++++ |
| 56 | + |
| 57 | + |
| 59 | ++++ |
| 62 | + |
| 63 | ++++ |
| 64 | + |
| 65 | + |
| 70 | ++++ |
| 71 | ++++ |
| 72 | ++++ |
| 73 | ++++ |

**Example 9: Release of insulin after treatment with the compounds of the present invention**

After having identified the most promising compounds acting as GRK5 inhibitors and determined the $IC_{50}$ values, the effect of the compounds of the example 2 on the release of insulin by beta-TC6 was determined. Cells were cultured overnight, washed with PBS and then treated with 5 $\mu$M of test compounds as well as non-inhibitor controls and Sunitinib as positive control, for 2 h in a high glucose (4.5 g/L) DMEM at 37°C and 5 % (v/v) $CO_2$. Release of insulin was detected using a rat/mouse insulin ELISA.

To order to measure the insulin released by beta-TC6 insulinoma (mouse) cells upon treatment with the compounds of the present invention, beta-TC6 insulinoma (mouse)cells were washed and incubated for 2 h in high (4.5 g/L) or low (1.125 g/L) glucose media at 37 °C. After incubation, the supernatant of the beta-TC6 insulinoma cells was diluted 1:20. The insulin release was measured with rat/mouse insulin ELISA (Merck Millipore Darmstadt, Cat. # EZRMI-13K) by following the manufacture protocol. The enzyme activity of the horseradish peroxidase of the immobilized biotinylated antibodies was monitored spectrophotometrically by the increased absorbency at 490 nm, and corrected by the absorbency at 610 nm.

**Example 10: Glucose uptake after treatment of C2C12 cells with compounds of the present invention**

To investigate the impact of the GRK5 inhibitors on glucose uptake, the fluorescent D-glucose analog 2-[*N*-(7-nitrobenz-2-oxa-1,3-diazol-4-yl)-amino]-2-deoxy-D-glucose (2-NBDG) was used. 2-NBDG is a fluorescently-labeled deoxyglucose analog that is commonly used to directly monitor glucose uptake by living cells. For 2-NBDG detection, cells were cultured for 1 h together with 5 $\mu$M of an inventive compound in glucose free media supplied with 100 $\mu$M 2-NBDG at 37 °C before collecting. Furthermore, as internal controls, samples of glucose free media, respectively glucose free media with test compound, were tested. Subsequently, cells were washed, trypsinized and harvested in ice-cold PBS before centrifuged at 1.6 x$10^3$ rpm at 4°C. Cells were analyzed by flow cytometry (FACS Calibur, BD Bioscience, respectively a BD Accuri® C6 Flow Cytometer). For evaluation, cells were gated using the side- and forward scatter SSC/FSC and quantified by excited at 488 nm and collected at 533 nM (FL1).

**<u>Example 11:</u> Glucose mediated effect on insulin release by treatment of beta-TC6 cells with compounds of the present invention**

Hypoglycemia can cause impairment of cognitive function, motoric control or even consciousness. For safety reasons, it is important that the inventive compounds are glucose dependent and do not enhance the insulin release in low glucose environment. Therefore, the glucose dependent insulin release in beta-TC6 cells using media with 1.125 mM (20 mg/dL) glucose was verified. Cells were cultured for 24 h in a 96-well plate, washed with PBS and then appropriate media was replaced for 2 h at 37°C and 5 % (v/v) $CO_2$. Afterwards, the supernatant was used to detect the amount of insulin released in a rat/mouse insulin ELISA (Merck Millipore Darmstadt, Cat. # EZRMI-13K) by following the manufacture protocol. The enzyme activity of the horseradish peroxidase of the immobilized biotinylated antibodies was monitored spectrophotometrically by the increased absorbency at 490 nm, and corrected by the absorbency at 610 nm.

Treatment of beta-TC6 cells with compounds of the invention did not lead to increased insulin release like observed in high glucose media. Sunitinib seemed to decrease the insulin release (0.77 $\pm$ 0.03), whereas all the inventive compounds ranged close to the DMSO control.

**<u>Example 12:</u> Evaluation of insulin dependence of the inventive compounds**

To investigate if the 2-NBDG uptake of the inventive compound inhibitors of GRK5 are insulin dependent, 3T3-L pre-adipocytes were differentiated to matured adipocytes. Afterwards, cells were starved for 4 h before they were washed and glucose free media supplied with 100 $\mu$M 2-NBDG, in the presence and absence of 10 $\mu$g/ml insulin the test compound was added for 1 h at 37 °C. Furthermore, as internal controls, samples of glucose free media or glucose free media with compound were measured (data not shown). Fluorescence was analyzed by flow cytometer (FACS Calibur, BD Bioscience, respectively a BD Accuri® C6 Flow Cytometer). For evaluation, cells were gated using the side- and forward scatter SSC/FSC and quantified by excited at 488 and detected at 533 nm (FL1).

**<u>Example 13:</u> Comparison of the influence on insulin release of the inventive compounds and commercially available kinase inhibitors**

Based on literature references, several commercially available inhibitors were compared with compounds according to the invention. For comparison we chose the Akt1-Inhibitor II (Calbiochem #124008), which should lead to decreased insulin release, as well as the Map2K3-Inhibitor II (Calbiochem #444938), Exendin-4 (Sigma #E7144) and GLP-1 (Sigma #G8147), which should lead to an increased insulin release. Cells were cultured for 24 h before treated with the inventive compounds and control inhibitors for 2 h at 37 °C and 5 % (v/v) $CO_2$. Insulin in the supernatant was detected by mouse/rat insulin ELISA.

**<u>Example 14:</u> Insulin HTRF assay principle**

For further screening of compounds derived from compounds described above for their ability to regulate insulin release an insulin HTRF® (Homogeneous Time-Resolved Fluorescence; Cisbio International, France) was used. This assay is based on two antibodies against insulin binding to different epitopes of insulin. One of these antibodies is coupled to Europium (Ab-Eu-Cryptate) and the other one to the fluorophore XL665 (Ab-XL665). If the insulin is secreted into the supernatant of cell culture, both antibodies can bind to insulin and come therefore into close proximity, which allows upon excitation that energy transfer between (FRET) the long-life fluorescent donor Europium (cryptate) and the acceptors XL665. FRET increases proportionally with insulin concentration.

The automated assay protocol is as follows:

On day 1, 15k beta-TC6 cells per well were seeded for 24 or 48 h in 50 $\mu$L complete medium. On day 2 or respective on day 3, the medium is removed and cells are washed with 60 $\mu$L 1xPBS and 20 $\mu$L induction buffer is added using BioTek washer. Subsequently, 10 $\mu$L Sunitininb (5 -$\mu$M f.c.) or the test compound in induction buffer; 0.5% f.c. DMSO (CyBi-well) is transferred to the cells. The cells are then incubated at 37°C for 2, 3, 4h.

The HTRF assay is carried out using Greiner 384-well 784075 assay plates.
First 10 $\mu$L supernatant is transferred from the cell plates in HTRF plates (CyBi-well). 10 $\mu$L of 0, 1, 2, and 4 ng/ml insulin standard controls in assay medium is used as a control. To each well 10 $\mu$L of combined 1:25 Ab-XL665 (Acc) and 1:20 Ab-Eu-Cryptate (WellMate) are added and incubated for 120 min at RT in the dark. Finally HTRF is measured on ViewLux ultra high throughput microplate imager (PerkinElmer).

The evaluation software DataFactoty derives relative activities Ar according to the following equation:

$$Ar = \frac{V - R_{(0\% control)}}{R_{(100\% control)} - R_{0\% control)}} \times 100$$

| | |
|---|---|
| Ar | relative activity in % |
| V | Raw data value of test well |
| $R_{(0\% \text{ control})}$ | Median raw data value of 0% control wells |
| $R_{(100\% \text{ control})}$ | Median raw data value of 100% control wells |

## Claims

**1.** The compound of general formula (**I**)

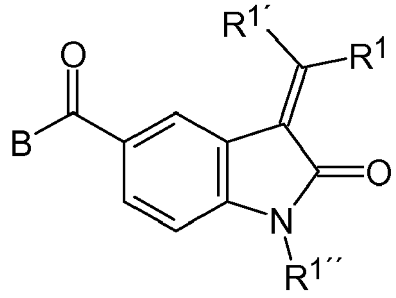

(**I**)

wherein,
B represents:

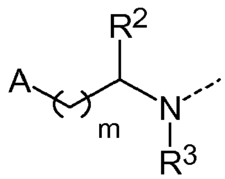

or $-OR^{16}$
$R^1$ and $R^{1'}$ are each independently selected from: -Ph, -H,

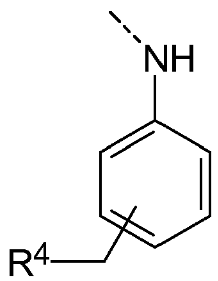

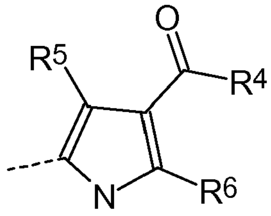

and

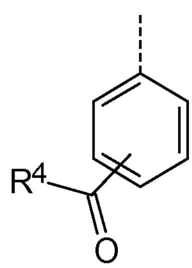

and $R^1$ and $R^{1'}$ cannot be simultaneously -H and -Ph;
$R^{1''}$ represents -H or $-C(O)R^{18}$;
$R^2$ represents: $-R^{19}$, $-C(O)NH_2$, or $-CO_2R^{20}$;
$R^4$ represents: $-R^7$, $-NH-(CH_2)_p-R^{7'}$

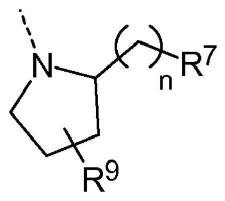

or

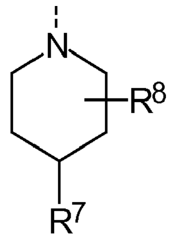

$R^7$ represents:
$-NR^{10}R^{11}$,

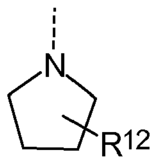 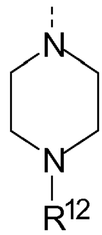 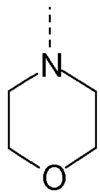

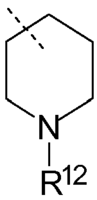 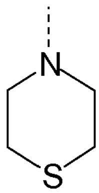 or 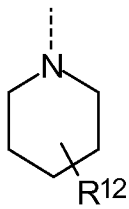

and $R^{10}$ and $R^{11}$ cannot be simultaneously -H;
$R^{7'}$ represents:

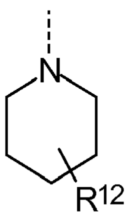 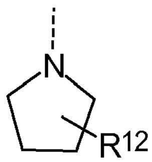 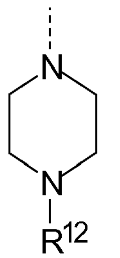 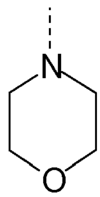

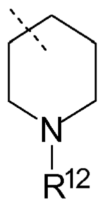 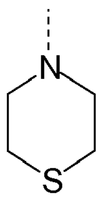 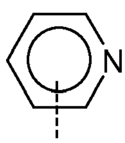 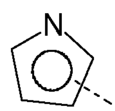

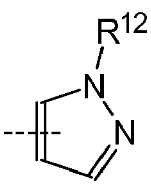 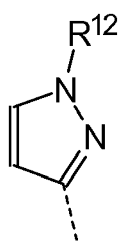 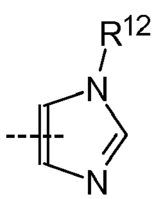 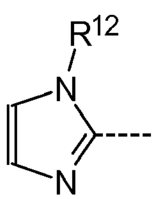

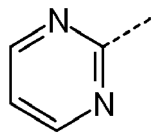
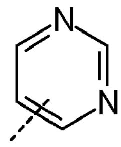
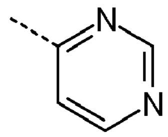
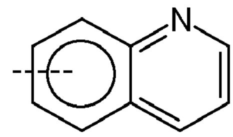

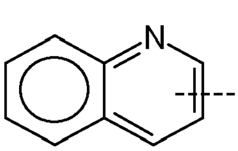
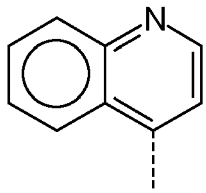
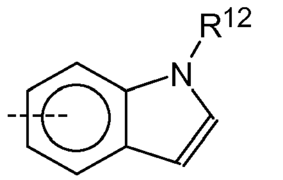
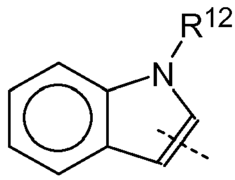

or $-NR^{10}R^{11}$, and $R^{10}$ and $R^{11}$ cannot be simultaneously -H;
A represents:

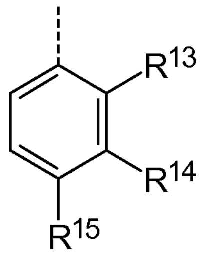

or

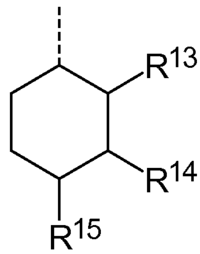

$R^{13}$, $R^{14}$ and $R^{15}$ are each independently selected from the group consisting of: $-R^{21}$, $-R^{22}$, $-R^{23}$, $-OR^{21}$, $-OR^{22}$, $-OR^{23}$, -F, -Cl, -Br and -I,
$R^{14}$ together with $R^{15}$ may form with the two carbon of the benzene or cyclohexane they are attached to a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;
$R^{13}$ together with $R^2$ may form a carbocyclic 4-, 5- or 6-membered ring and that 4-, 5- or 6-membered ring can be saturated or unsaturated, or a heterocyclic 5- or 6-membered ring and that 5- or 6-membered ring can be saturated or unsaturated;
$R^3$, $R^8$, $R^9$, $R^{10}$, $R^{11}$, $R^{12}$, $R^{16}$, $R^{17}$, $R^{18}$, $R^{19}$, $R^{20}$, $R^{21}$, $R^{22}$ and $R^{23}$ are each independently selected from: -H, $-CH_3$, $-C_2H_5$, $-C_3H_7$, $-C_4H_9$, $-C_5H_{11}$, -Ph, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-CH_2Ph$;
m is an integer number selected from 0 and 1,
n is an integer number selected from 1, 2, 3, 4, 5 and 6,
p is an integer number selected from 0, 1 and 2,
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**2.** Compound according to claim 1, wherein B represents

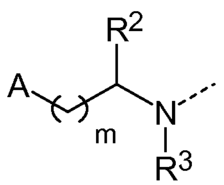

.

**3.** Compound according to claim 1 or 2, wherein m=0, A represents

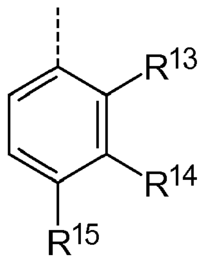

$R^1$ represents the following:

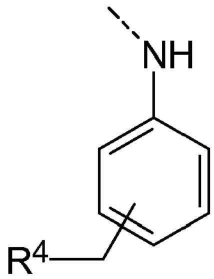 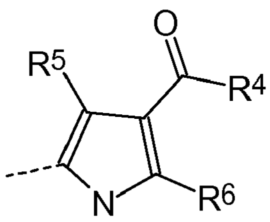 and 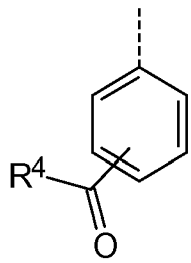

and $R^{1'}$ is -Ph or -H.

**4.** Compound according to any one of claims 1 - 3, wherein $R^1$ represents:

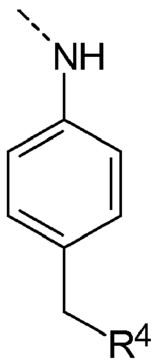

**5.** Compound according to any one of claims 1 - 3, wherein, $R^1$ represents:

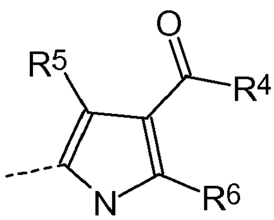

**6.** Compound according to any one of claims 1 - 4, wherein $R^2$ is selected from: $-CH_3$, $-C_2H_5$.

**7.** Compound according to claim 1, wherein B is -OH.

**8.** Compound according to claim 1, wherein the compound is selected from the following group:

No. Name

1 ((3Z)-3-{[3,5-dimethyl-4-(piperidin-1-ylcarbonyl)-1H-pyrrol-2-yl]methylene}-2-oxoindoline-5-carboxylic acid

2 (((3Z)-3-({3,5-dimethyl-4-[(2-pyrrolidin-1-ylethyl)carbamoyl]-1H-pyrrol-2-yl}methylene)-2-oxoindoline-5-carboxylic acid

3 ((3Z)-3-[(4-{[2-(diethylamino)ethyl]carbamoyl}-3,5-dimethyl-1H-pyrrol-2-yl)methylene]-2-oxoindoline-5-carboxylic acid

4 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxylic acid

5 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxylic acid

6 ((3Z)-3-({3,5-dimethyl-4-[(2-pyrrolidin-1-ylethyl)carbamoyl]-1H-pyrrol-2-yl}methylene)-2-oxo-N-[(1R)-1-phenylpropyl]indoline-5-carboxamide

7 ((3Z)-3-({3,5-dimethyl-4-[(4-methylpiperazin-1-yl)carbonyl]-1H-pyrrol-2-yl}methylene)-2-oxo-N-[(1R)-1-phenylpropyl]indoline-5-carboxamide

8 ((3Z)-N-[1-(3-chlorophenyl)propyl]-3-({3,5-dimethyl-4-[(4-methylpiperazin-1-yl)carbonyl]-1H-pyrrol-2-yl}methylene)-2-oxoindoline-5-carboxamide

9 ((3Z)-N-[1-(3,4-difluorophenyl)propyl]-3-({3,5-dimethyl-4-[(4-methylpiperazin-1-yl)carbonyl]-1H-pyrrol-2-yl}methylene)-2-oxoindoline-5-carboxamide

10 ((3Z)-N-[1-(3,4-difluorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

11 ((3Z)-N-[1-(3-chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

12 ((3Z)-N-[1-(4-chlorophenyl)propyl]-3-({3,5-dimethyl-4-[(4-methylpiperazin-1-yl)carbonyl]-1H-pyrrol-2-yl}methylene)-2-oxoindoline-5-carboxamide

13 ((3Z)-N-[1-(4-chlorophenyl)propyl]-3-({3,5-dimethyl-4-[(2-pyrrolidin-1-ylethyl)carbamoyl]-1H-pyrrol-2-yl}methylene)-2-oxoindoline-5-carboxamide

14 ((3Z)-3-{[3,5-dimethyl-4-(piperidin-1-ylcarbonyl)-1H-pyrrol-2-yl]methylene}-2-oxo-N-[(1R)-1-phenylethyl]indoline-5-carboxamide

15 (3Z)-3-({3,5-dimethyl-4-[(4-methylpiperazin-1-yl)carbonyl]-1H-pyrrol-2-yl}methylene)-2-oxo-N-[(1R)-1-phenylethyl]indoline-5-carboxamide

16 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1R)-1-phenylethyl]indoline-5-carboxamide

17 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1R)-1-phenylethyl]indoline-5-carboxamide

18 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1S)-1-phenylethyl]indoline-5-carboxamide

19 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1S)-1-phenylethyl]indoline-5-carboxamide

20 ((3Z)-3-({3,5-dimethyl-4-[(4-methylpiperazin-1-yl)carbonyl]-1H-pyrrol-2-yl}methylene)-N-[(1S)-1-(4-methoxyphenyl)ethyl]-2-oxoindoline-5-carboxamide

21 ((3Z)-3-({3,5-dimethyl-4-[(4-methylpiperazin-1-yl)carbonyl]-1H-pyrrol-2-yl}methylene)-N-[(1R)-1-(4-methoxyphenyl)ethyl]-2-oxoindoline-5-carboxamide

22 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(4-methoxyphenyl)ethyl]-2-oxoindoline-5-carboxamide

23 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(3-methoxyphenyl)ethyl]-2-oxoindoline-5-carboxamide

24 ((3Z)-N-[(1R)-1-cyclohexylethyl]-3-({3,5-dimethyl-4-[(4-methylpiperazin-1-yl)carbonyl]-1H-pyrrol-2-yl}methylene)-2-oxoindoline-5-carboxamide

25 ((3Z)-N-[(1R)-1-(3-chlorophenyl)ethyl]-3-({3,5-dimethyl-4-[(4-methylpiperazin-1-yl)carbonyl]-1H-pyrrol-2-yl}methylene)-2-oxoindoline-5-carboxamide

26 ((3Z)-N-[(1R)-1-(3-chlorophenyl)ethyl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide

27 ((3Z)-3-({3,5-dimethyl-4-[(4-methylpiperazin-1-yl)carbonyl]-1H-pyrrol-2-yl}methylene)-N-[(1R)-1-(2-naphthyl)ethyl]-2-oxoindoline-5-carboxamide

28 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1R)-1-(2-naphthyl)ethyl]-2-oxoindoline-5-carboxamide

29 (3Z)-N-[(1S)-2,3-dihydro-1H-inden-1-yl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide

30 ((3Z)-N-[(1R)-2,3-dihydro-1H-inden-1-yl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide

31 ((3Z)-3-({3,5-dimethyl-4-[(4-methylpiperazin-1-yl)carbonyl]-1H-pyrrol-2-yl}methylene)-N-methyl-2-oxo-N-[(1R)-1-phenylethyl]indoline-5-carboxamide

32 ((3Z)-3-({3,5-dimethyl-4-[(4-methylpiperazin-1-yl)carbonyl]-1H-pyrrol-2-yl}methylene)-N-[(1R)-1-(4-methylphenyl)ethyl]-2-oxoindoline-5-carboxamide

33 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1R)-1-(4-methylphenyl)ethyl]-2-oxoindoline-5-carboxamide

34 ((3Z)-3-({3,5-dimethyl-4-[(4-methylpiperazin-1-yl)carbonyl]-1H-pyrrol-2-yl}methylene)-N-[(1R)-1-(4-methylphenyl)ethyl]-2-oxoindoline-5-carboxamide

35 ((3Z)-N-[(1R)-1-(4-chlorophenyl)ethyl]-3-({3,5-dimethyl-4-[(4-methylpiperazin-1-yl)carbonyl]-1H-pyrrol-2-yl}methylene)-2-oxoindoline-5-carboxamide

36 ((3Z)-N-[(1S)-1-(4-chlorophenyl)ethyl]-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxamide

37 ((3Z)-N-[(1R)-1-(4-chlorophenyl)ethyl]-3-[(3,5-dimethyl-4-{[(2R)-2-(pyrrolidin-1-ylmethyl)pyrrolidin-1-yl]carbonyl}-1H-pyrrol-2-yl)methylene]-2-oxoindoline-5-carboxamide

38 ((3Z)-N-[(1R)-1-(3-chlorophenyl)ethyl]-3-[(3,5-dimethyl-4-{[(2S)-2-(pyrrolidin-1-ylmethyl)pyrrolidin-1-yl]carbonyl}-1H-pyrrol-2-yl)methylene]-2-oxoindoline-5-carboxamide

39 ((3Z)-N-[(1R)-1-(3-bromophenyl)ethyl]-3-({3,5-dimethyl-4-[(4-methylpiperazin-1-yl)carbonyl]-1H-pyrrol-2-yl}methylene)-2-oxoindoline-5-carboxamide

40 ((3Z)-N-[(1S)-1-(3-chlorophenyl)ethyl]-3-({3,5-dimethyl-4-[(4-methylpiperazin-1-yl)carbonyl]-1H-pyrrol-2-yl}methylene)-2-oxoindoline-5-carboxamide

41 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-N-[(1S)-1-(4-fluorophenyl)ethyl]-2-oxoindoline-5-carboxamide

42 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1S)-1-(4-fluorophenyl)ethyl]-2-oxoindoline-5-carboxamide

43 (3Z)-3-[(3,5-dimethyl-4-{[(2R)-2-(pyrrolidin-1-ylmethyl)pyrrolidin-1-yl]carbonyl}-1H-pyrrol-2-yl)methylene]-N-[(1R)-1-(4-fluorophenyl)ethyl]-2-oxoindoline-5-carboxamide

44 ((3Z)-3-[(3,5-dimethyl-4-{[4-(4-methylpiperazin-1-yl)piperidin-1-yl]carbonyl}-1 H-pyrrol-2-yl)methylene]-2-oxo-N-[(1R)-1-phenylethyl]indoline-5-carboxamide

45 ((3Z)-3-({3,5-dimethyl-4-[(4-methylpiperazin-1-yl)carbonyl]-1H-pyrrol-2-yl}methylene)-2-oxo-N-[(1S)-1-phenylpropyl]indoline-5-carboxamide

46 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1R)-1-phenylpropyl]indoline-5-carboxamide

47 ((3Z)-3-[(3,5-dimethyl-4-{[(2S)-2-(pyrrolidin-1-ylmethyl)pyrrolidin-1-yl]carbonyl}-1H-pyrrol-2-yl)methylene]-2-oxo-N-[(1S)-1-phenylpropyl]indoline-5-carboxamide

48 ((3Z)-3-[(3,5-dimethyl-4-{[(2R)-2-(pyrrolidin-1-ylmethyl)pyrrolidin-1-yl]carbonyl}-1H-pyrrol-2-yl)methylene]-2-oxo-N-[(1R)-1-phenylpropyl]indoline-5-carboxamide

49 ((3Z)-N-[(1S)-1-(3-bromophenyl)ethyl]-3-({3,5-dimethyl-4-[(4-methylpiperazin-1-yl)carbonyl]-1H-pyrrol-2-yl}methylene)-2-oxoindoline-5-carboxamide

50 ((3Z)-3-{[3,5-dimethyl-4-(pyrrolidin-1-ylcarbonyl)-1H-pyrrol-2-yl]methylene}-N-[(1S)-1-(4-methoxyphenyl)ethyl]-2-oxoindoline-5-carboxamide

51 ((3Z)-3-({3,5-dimethyl-4-[(4-methylpiperazin-1-yl)carbonyl]-1H-pyrrol-2-yl}methylene)-N-[1-(3-methylphenyl)ethyl]-2-oxoindoline-5-carboxamide

52 ((3Z)-3-[(3,5-dimethyl-4-{[4-(4-methylpiperazin-1-yl)piperidin-1-yl]carbonyl}-1 H-pyrrol-2-yl)methylene]-2-oxo-N-[(1S)-1-phenylpropyl]indoline-5-carboxamide

53 ((3Z)-3-[(3,5-dimethyl-4-{[4-(4-methylpiperazin-1-yl)piperidin-1-yl]carbonyl}-1 H-pyrrol-2-yl)methylene]-2-oxo-N-[(1R)-1-phenylpropyl]indoline-5-carboxamide

54 ((3Z)-3-{[3,5-dimethyl-4-(piperidin-1-ylcarbonyl)-1H-pyrrol-2-yl]methylenel-2-oxo-N-[(1S)-1-phenylethyl]indoline-5-carboxamide

55 ((3Z)-N-[(1R)-1-(3-chlorophenyl)ethyl]-3-{[3,5-dimethyl-4-(piperidin-1-ylcarbonyl)-1H-pyrrol-2-yl]methylene}-2-oxoindoline-5-carboxamide

56 ((3Z)-N-[(1S)-1-(3-chlorophenyl)ethyl]-3-{[3,5-dimethyl-4-(piperidin-1-ylcarbonyl)-1H-pyrrol-2-yl]methylene}-2-oxoindoline-5-carboxamide

57 ((3Z)-3-{[3,5-dimethyl-4-(piperidin-1-ylcarbonyl)-1H-pyrrol-2-yl]methylene}-2-oxo-N-[(1S)-1-phenylpropyl]indoline-5-carboxamide

58 ((3Z)-3-{4-[(4-methylpiperazin-1-yl)carbonyl]benzylidene}-2-oxo-N-[(1S)-1-phenylpropyl]indoline-5-carboxamide

59 (3Z)-3-[(4-{[2-(diethylamino)ethyl]carbamoyl}-3,5-dimethyl-1H-pyrrol-2-yl)methylene]-2-oxo-N-[(1R)-1-phenylethyl]indoline-5-carboxamide
60 ((3Z)-3-[(4-{[2-(diethylamino)ethyl]carbamoyl}-3,5-dimethyl-1H-pyrrol-2-yl)methylene]-2-oxo-N-[(1S)-1-phenylethyl]indoline-5-carboxamide
61 ((3Z)-3-[(4-{[2-(diethylamino)ethyl]carbamoyl}-3,5-dimethyl-1H-pyrrol-2-yl)methylene]-2-oxo-N-[(1R)-1-phenylpropyl]indoline-5-carboxamide
62 ((3Z)-3-[(4-{[2-(diethylamino)ethyl]carbamoyl}-3,5-dimethyl-1H-pyrrol-2-yl)methylene]-2-oxo-N-[(1S)-1-phenylpropyl]indoline-5-carboxamide
63 ((3Z)-3-{[3,5-dimethyl-4-(piperidin-1-ylcarbonyl)-1H-pyrrol-2-yl]methylene}-2-oxo-N-[(1R)-1-phenylpropyl]indoline-5-carboxamide
64 ((3Z)-3-{[3,5-dimethyl-4-(piperidin-1-ylcarbonyl)-1H-pyrrol-2-yl]methylene}-N-[(1S)-1-(4-fluorophenyl)ethyl]-2-oxoindoline-5-carboxamide
65 ((3Z)-3-{[3,5-dimethyl-4-(piperidin-1-ylcarbonyl)-1H-pyrrol-2-yl]methylene}-N-[(1S)-1-(4-methoxyphenyl)ethyl]-2-oxoindoline-5-carboxamide
66 ((3Z)-N-[(1R)-1-(4-chlorophenyl)ethyl]-3-{[3,5-dimethyl-4-(piperidin-1-ylcarbonyl)-1H-pyrrol-2-yl]methylene}-2-oxoindoline-5-carboxamide
67 ((3Z)-N-[(1R)-1-(4-chlorophenyl)ethyl]-3-{[3,5-dimethyl-4-(pyrrolidin-1-ylcarbonyl)-1H-pyrrol-2-yl]methylene}-2-oxoindoline-5-carboxamide
68 ((3Z)-2-oxo-3-({[4-(piperidin-1-ylmethyl)phenyl]amino}methylene)indoline-5-carboxylic acid
69 ((3Z)-2-oxo-N-[(1R)-1-phenylpropyl]-3-({[4-(piperidin-1-ylmethyl)phenyl]amino}methylene)-indoline-5-carboxamide
70 ((3Z)-N-[(1R)-1-(4-methoxyphenyl)ethyl]-2-oxo-3-({[4-(piperidin-1-ylmethyl)phenyl]amino}methylene)indoline-5-carboxamide
71 ((3Z)-N-methyl-2-oxo-N-[(1S)-1-phenylethyl]-3-({[4-(piperidin-1-ylmethyl)phenyl]amino}methylene)indoline-5-carboxamide
72 ((3Z)-N-[(1R)-1-(4-methylphenyl)ethyl]-2-oxo-3-({[4-(piperidin-1-ylmethyl)phenyl]amino}methylene)indoline-5-carboxamide
73 ((3Z)-N-[(1R)-1-(4-chlorophenyl)ethyl]-2-oxo-3-({[4-(piperidin-1-ylmethyl)phenyl]amino}methylene)indoline-5-carboxamide
74 ((3Z)-2-oxo-N-[(1S)-1-phenylpropyl]-3-({[4-(piperidin-1-ylmethyl)phenyl]amino}methylene)indoline-5-carboxamide
75 ((3Z)-2-oxo-N-[(1S)-1-phenylethyl]-3-({[4-(piperidin-1-ylmethyl)phenyl]amino}methylene)indoline-5-carboxamide
76 ((3Z)-2-oxo-3-({[4-(piperidin-1-ylmethyl)phenyl]amino}methylene)-N-[(1S)-1,2,3,4-tetrahydronaphthalen-1-yl]indoline-5-carboxamide
77 ((3Z)-N-[(1R)-1-cyclohexylethyl]-2-oxo-3-({[4-(piperidin-1-ylmethyl)phenyl]amino}methylene)indoline-5-carboxamide
78 (3Z)-2-oxo-N-[(1R)-1-phenylethyl]-3-({[4-(piperidin-1-ylmethyl)phenyl] amino}methylene)indoline-5-carboxamide
79 ((3Z)-N-[(1R)-1-(3-methoxyphenyl)ethyl]-2-oxo-3-({[4-(piperidin-1-ylmethyl)phenyl]amino}methylene)indoline-5-carboxamide
80 ((3Z)-N-[(1R)-1-(3-chlorophenyl)ethyl]-2-oxo-3-({[4-(piperidin-1-ylmethyl)phenyl]amino}methylene)indoline-5-carboxamide
81 ((3Z)-2-oxo-N-[(1R)-1-phenylethyl]-3-(phenyl{[3-(piperidin-1-ylmethyl)phenyl]amino}methylene)indoline-5-carboxamide
82 ((3Z)-N-[(1S)-1-(4-fluorophenyl)ethyl]-2-oxo-3-(phenyl{[3-(piperidin-1-ylmethyl)phenyl]amino}methylene)indoline-5-carboxamide
83 ((3Z)-N-[(1R)-1-(4-fluorophenyl)ethyl]-2-oxo-3-(phenyl{[3-(piperidin-1-ylmethyl)phenyl]amino}methylene)indoline-5-carboxamide
84 ((3Z)-2-oxo-3-(phenyl{[3-(piperidin-1-ylmethyl)phenyl]amino}methylene)-N-[(1 R)-1-phenylpropyl]indoline-5-carboxamide
85 ((3Z)-N-[(1S)-1-(4-chlorophenyl)ethyl]-2-oxo-3-(phenyl{[3-(piperidin-1-ylmethyl)phenyl]amino}methylene)indoline-5-carboxamide
86 ((3Z)-N-[(1R)-1-(4-chlorophenyl)ethyl]-2-oxo-3-(phenyl{[3-(piperidin-1-ylmethyl)phenyl]amino}methylene)indoline-5-carboxamide
87 ((3Z)-2-oxo-N-[(1S)-1-phenylethyl]-3-(phenyl{[3-(piperidin-1-ylmethyl)phenyl]amino}methylene)indoline-5-carboxamide
88 ((3Z)-2-oxo-N-[(1R)-1-phenylethyl]-3-(phenyl{[3-(piperidin-1-ylmethyl)phenyl]amino}methylene)indoline-5-carboxamide
89 ((3Z)-2-oxo-3-(phenyl{[3-(piperidin-1-ylmethyl)phenyl]amino}methylene)-N-[(1 S)-1-phenylpropyl]indoline-5-carboxamide
90 ((3Z)-2-oxo-3-(phenyl{[3-(piperidin-1-ylmethyl)phenyl]amino}methylene)-N-[(1 R)-1-phenylpropyl]indoline-5-carboxamide
91 ((3Z)-N-[(1R)-1-(4-methoxyphenyl)ethyl]-2-oxo-3-({[4-(piperidin-1-ylmethyl)phenyl]amino}methylene)indoline-5-carboxamide
92 ((3Z)-N-[(1S)-1-(4-fluorophenyl)ethyl]-2-oxo-3-({[4-(piperidin-1-ylmethyl)phenyl]amino}methylene)indoline-5-carboxamide
93 ((3Z)-N-[(1R)-1-(3-methoxyphenyl)ethyl]-2-oxo-3-({[4-(piperidin-1-ylmethyl)phenyl]amino}methylene)indoline-5-carboxamide
94 ((3Z)-N-[(1R)-1-(4-fluorophenyl)ethyl]-2-oxo-3-({[4-(piperidin-1-ylmethyl)phenyl]amino}methylene)indoline-5-carboxamide
95 ((3Z)-N-[(1R)-1-(3-chlorophenyl)ethyl]-2-oxo-3-({[4-(piperidin-1-ylmethyl)phenyl]amino}methylene)indoline-5-carboxamide
96 ((3Z)-3-({[3-(morpholin-4-ylmethyl)phenyl]amino}methylene)-2-oxoindoline-5-carboxylic acid
97 ((3Z)-3-({[3-(morpholin-4-ylmethyl)phenyl]amino}methylene)-2-oxo-N-[(1R)-1-phenylpropyl]indoline-5-carboxamide
98 ((3Z)-3-({[3-(morpholin-4-ylmethyl)phenyl]amino}methylene)-2-oxo-N-[(1S)-1-phenylpropyl]indoline-5-carboxamide
99 ((3Z)-3-({[3-(morpholin-4-ylmethyl)phenyl]amino}methylene)-2-oxo-N-[(1S)-1-phenylethyl]indoline-5-carboxamide
100 ((3Z)-3-({[3-(morpholin-4-ylmethyl)phenyl]amino}methylene)-2-oxo-N-[(1R)-1-phenylethyl]indoline-5-carboxamide
101 ((3Z)-N-[(1R)-1-(4-fluorophenyl)ethyl]-3-({[3-(morpholin-4-ylmethyl)phenyl]amino}methylene)-2-oxoindoline-5-carboxamide
102 ((3Z)-N-[(1S)-1-(4-fluorophenyl)ethyl]-3-({[3-(morpholin-4-ylmethyl)phenyl]amino}methylene)-2-oxoindoline-5-carboxamide
103 ((3Z)-N-[(1S)-1-(4-methoxyphenyl)ethyl]-3-({[3-(morpholin-4-ylmethyl)phenyl]amino}methylene)-2-oxoindoline-5-carboxamide
104 3Z)-N-[(1R)-1-(4-methoxyphenyl)ethyl]-3-({[3-(morpholin-4-ylmethyl)phenyl]amino}methylene)-2-oxoindoline-5-carboxamide
105 ((3Z)-2-oxo-3-({[4-(piperidin-1-ylmethyl)phenyl]amino}methylene)indoline-6-carboxylic acid
106 ((3Z)-2-oxo-N-[(1R)-1-phenylethyl]-3-({[4-(piperidin-1-ylmethyl)phenyl]amino}methylene)indoline-6-carboxamide
107 ((3Z)-2-oxo-N-[(1S)-1-phenylethyl]-3-({[4-(piperidin-1-ylmethyl)phenyl]amino}methylene)indoline-6-carboxamide
108 ((3Z)-2-oxo-N-[(1S)-1-phenylpropyl]-3-({[4-(piperidin-1-ylmethyl)phenyl]amino}methylene)indoline-6-carboxamide
109 ((3Z)-2-oxo-N-[(1R)-1-phenylpropyl]-3-({[4-(piperidin-1-ylmethyl)phenyl]amino}methylene)indoline-6-carboxamide
110 ((3Z)-N-[(1R)-1-(4-fluorophenyl)ethyl]-2-oxo-3-({[4-(piperidin-1-ylmethyl)phenyl]amino}methylene)indoline-6-carboxamide
111 ((3Z)-N-[(1S)-1-(4-fluorophenyl)ethyl]-2-oxo-3-({[4-(piperidin-1-ylmethyl)phenyl]amino}methylene)indoline-6-carboxamide
112 ((3Z)-N-[(1S)-1-(3-methoxyphenyl)ethyl]-2-oxo-3-({[4-(piperidin-1-ylmethyl)phenyl]amino}methylene)indoline-6-carboxamide
113 ((3Z)-N-[(1R)-1-(3-methoxyphenyl)ethyl]-2-oxo-3-({[4-(piperidin-1-ylmethyl)phenyl]amino}methylene)indoline-6-carboxamide
114 ((3Z)-3-({3,5-dimethyl-4-[(2-pyrrolidin-1-ylethyl)carbamoyl]-1H-pyrrol-2-yl}methylene)-2-oxo-N-[(1R)-1-phenylethyl]indoline-5-carboxamide
115 ((3Z)-3-({3,5-dimethyl-4-[(2-pyrrolidin-1-ylethyl)carbamoyl]-1H-pyrrol-2-yl}methylene)-2-oxo-N-[(1S)-1-phenylethyl]indoline-5-carboxamide
116 (3Z)-3-({3,5-dimethyl-4-[(2-pyrrolidin-1-ylethyl)carbamoyl]-1H-pyrrol-2-yl}methylene)-N-[(1S)-1-(4-fluorophenyl)ethyl]-2-oxoindoline-5-carboxamide
117 ((3Z)-3-({3,5-dimethyl-4-[(2-pyrrolidin-1-ylethyl)carbamoyl]-1H-pyrrol-2-yl}methylene)-N-[(1R)-1-(4-fluorophenyl)ethyl]-2-oxoindoline-5-carboxamide
118 ((3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-5-carboxamide
119 ((3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-5-carboxamid
120 ((3Z)-3-({3,5-dimethyl-4-[(2-pyrrolidin-1-ylethyl)carbamoyl]-1H-pyrrol-2-yl}methylene)-N-[(1S)-1-(3-methoxyphenyl)ethyl]-2-oxoindoline-5-carboxamide
121 ((3Z)-3-({3,5-dimethyl-4-[(2-pyrrolidin-1-ylethyl)carbamoyl]-1H-pyrrol-2-yl}methylene)-N-[(1R)-1-(3-methoxyphenyl)ethyl]-2-oxoindoline-5-carboxamide
122 ((3Z)-3-({3,5-dimethyl-4-[(2-pyrrolidin-1-ylethyl)carbamoyl]-1H-pyrrol-2-yl}methylene)-2-oxo-N-[(1R)-1-phenylethyl]indoline-6-carboxamide
123 ((3Z)-3-({3,5-dimethyl-4-[(2-pyrrolidin-1-ylethyl)carbamoyl]-1H-pyrrol-2-yl}methylene)-2-oxo-N-[(1S)-1-phenylethyl]indoline-6-carboxamide
124 ((3Z)-3-({3,5-dimethyl-4-[(2-pyrrolidin-1-ylethyl)carbamoyl]-1H-pyrrol-2-yl}methylene)-2-oxo-N-[(1S)-1-phenylpropyl]indoline-6-carboxamide
125 ((3Z)-3-({3,5-dimethyl-4-[(2-pyrrolidin-1-ylethyl)carbamoyl]-1H-pyrrol-2-yl}methylene)-2-oxo-N-[(1R)-1-phenylpropyl]indoline-6-carboxamide
126 ((3Z)-3-({3,5-dimethyl-4-[(2-pyrrolidin-1-ylethyl)carbamoyl]-1H-pyrrol-2-yl}methylene)-N-[(1R)-1-(4-fluorophenyl)ethyl]-2-oxoindoline-6-carboxamide
127 ((3Z)-3-({3,5-dimethyl-4-[(2-pyrrolidin-1-ylethyl)carbamoyl]-1H-pyrrol-2-yl}methylene)-N-[(1S)-1-(4-fluorophenyl)ethyl]-2-oxoindoline-6-carboxamide
128 ((3Z)-3-({3,5-dimethyl-4-[(2-pyrrolidin-1-ylethyl)carbamoyl]-1H-pyrrol-2-yl}methylene)-N-[(1S)-1-(4-methoxyphenyl)ethyl]-2-oxoindoline-6-carboxamide
129 ((3Z)-3-({3,5-dimethyl-4-[(2-pyrrolidin-1-ylethyl)carbamoyl]-1H-pyrrol-2-yl}methylene)-N-[(1R)-1-(4-methoxyphenyl)ethyl]-2-oxoindoline-6-carboxamide
130 ((3Z)-3-({3,5-dimethyl-4-[(1-methylpiperidin-4-yl)carbamoyl]-1H-pyrrol-2-yl}methylene)-2-oxo-N-[(1R)-1-phenylpropyl]indoline-5-carboxamide
131 ((3Z)-3-{[3,5-dimethyl-4-(piperidin-4-ylcarbamoyl)-1H-pyrrol-2-yl]methylene}-2-oxo-N-[(1R)-1-phenylpropyl]indoline-5-carboxamide
132 ((3Z)-3-[(3,5-dimethyl-4-{[(3S)-1-methylpiperidin-3-yl]carbamoyl}-1H-pyrrol-2-yl)methylene]-2-oxo-N-[(1R)-1-phenylpropyl]indoline-5-carboxamide
133 (3Z)-3-{[4-({(2S)-2-[(diethylamino)methyl]pyrrolidin-1-yl}carbonyl)-3,5-dimethyl-1H-pyrrol-2-yl]methylene}-2-oxo-N-[(1R)-1-phenylpropyl]indoline-5-carboxamide
134 ((3Z)-3-{[4-({(2S)-2-[(dimethylamino)methyl]pyrrolidin-1-yl}carbonyl)-3,5-dimethyl-1 H-pyrrol-2-yl]methylene}-2-oxo-N-[(1R)-1-phenylpropyl]indoline-5-carboxamide
135 ((3Z)-3-({3,5-dimethyl-4-[(3S)-piperidin-3-ylcarbamoyl]-1H-pyrrol-2-yl}methylene)-2-oxo-N-[(1R)-1-phenylpropyl]indoline-5-carboxamide
136 ((3Z)-3-{[4-({(2R)-2-[(diethylamino)methyl]pyrrolidin-1-yl}carbonyl)-3,5-dimethyl-1 H-pyrrol-2-yl]methylene}-2-oxo-N-[(1R)-1-phenylpropyl]indoline-5-carboxamide
137 ((3Z)-3-({3,5-dimethyl-4-[(2-piperidin-1-ylethyl)carbamoyl]-1H-pyrrol-2-yl}methylene)-2-oxo-N-[(1R)-1-phenylpropyl]indoline-5-carboxamide
138 ((3Z)-3-[(3,5-dimethyl-4-{[2-(4-methylpiperazin-1-yl)ethyl]carbamoyl}-1H-pyrrol-2-yl)methylene]-2-oxo-N-[(1R)-1-phenylpropyl]indoline-5-carboxamide
139 ((3Z)-3-({3,5-dimethyl-4-[(2-morpholin-4-ylethyl)carbamoyl]-1H-pyrrol-2-yl}methylene)-2-oxo-N-[(1R)-1-phenylpropyl]indoline-5-carboxamide

**9.** Compound according to claim 1 for medical use.

**10.** Compound according to any one of the claims 1 - 9 for the inhibition of GRK5 and/or for use in glucose dependent regulation of insulin production and/or release of insulin and/or for increasing glucose uptake..

**11.** Compound according to any of the claims 1 - 10 for use in the treatment or prophylaxis of a metabolic disease or cancer.

**12.** Compound according to claim 11, wherein the metabolic disease is selected from diabetes, obesity and impaired adipogenesis.

**13.** Compound according to claims 11 or 12, wherein the metabolic disease is diabetes mellitus type 2.

**14.** Compound according to claim 11, wherein the cancer is selected from the group consisting of: adenocarcinoma,

choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

**15.** Pharmaceutical composition comprising at least one compound according to any one of the claims 1 - 8 together with at least one pharmaceutically acceptable carrier, solvent or excipient.

Europäisches Patentamt
European Patent Office
Office européen des brevets

# EUROPEAN SEARCH REPORT

Application Number
EP 13 18 0792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/73297 A1 (BOEHRINGER INGELHEIM PHARMA [DE]; HECKEL ARMIN [DE]; WALTER RAINER [DE) 7 December 2000 (2000-12-07)<br>* examples 1,2,4,VI,VII *<br>* claims 1,9-10 *<br>----- | 1-4,6,7,9,11,14,15 | INV.<br>C07D401/14<br>C07D403/06<br>C07D403/14 |
| X | GERALD J. ROTH ET AL: "Design, Synthesis, and Evaluation of Indolinones as Inhibitors of the Transforming Growth Factor [beta] Receptor I (TGF[beta]RI)", JOURNAL OF MEDICINAL CHEMISTRY, vol. 53, no. 20, 28 October 2010 (2010-10-28), pages 7287-7295, XP055079035, ISSN: 0022-2623, DOI: 10.1021/jm100812a<br>* compounds 21,23 *<br>----- | 1,4,7 | |
| X | DE 102 37 423 A1 (BOEHRINGER INGELHEIM PHARMA [DE]) 19 February 2004 (2004-02-19)<br>* claim 1; compounds U,V,W,X,Z,AB *<br>* claims 2,11 *<br>----- | 1-4,9,11,12,15 | |
| | | | TECHNICAL FIELDS SEARCHED (IPC) |
| A,D | WO 2012/028335 A2 (MAX PLANCK GESELLSCHAFT [DE]; BAECKER MATHIAS [DE]; ULLRICH AXEL [DE]) 8 March 2012 (2012-03-08)<br>* page 3, line 21 - line 28 *<br>* page 27; example 5 *<br>----- | 1-15 | C07D |

The present search report has been drawn up for all claims

1

| Place of search | Date of completion of the search | Examiner |
|---|---|---|
| Munich | 13 September 2013 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 0792

This annex lists the patent family members relating to the patent documents cited in the above-mentioned European search report. The members are as contained in the European Patent Office EDP file on
The European Patent Office is in no way liable for these particulars which are merely given for the purpose of information.

13-09-2013

| Patent document cited in search report | Publication date | Patent family member(s) | Publication date |
|---|---|---|---|
| WO 0073297 A1 | 07-12-2000 | AU 5215700 A | 18-12-2000 |
| | | DE 19924401 A1 | 30-11-2000 |
| | | WO 0073297 A1 | 07-12-2000 |
| DE 10237423 A1 | 19-02-2004 | AU 2003255413 A1 | 11-03-2004 |
| | | CA 2495350 A1 | 04-03-2004 |
| | | DE 10237423 A1 | 19-02-2004 |
| | | EP 1530466 A2 | 18-05-2005 |
| | | EP 2281561 A2 | 09-02-2011 |
| | | JP 2006514611 A | 11-05-2006 |
| | | WO 2004017948 A2 | 04-03-2004 |
| WO 2012028335 A2 | 08-03-2012 | AU 2011297893 A1 | 14-03-2013 |
| | | CA 2809194 A1 | 08-03-2012 |
| | | EP 2426202 A1 | 07-03-2012 |
| | | EP 2611919 A2 | 10-07-2013 |
| | | WO 2012028335 A2 | 08-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012028335 A
- WO 2012028335 A1


### Non-patent literature cited in the description


- *Biochem. Biophys. Res. Commun.,* 2012, vol. 421, 312
- *J. Clin. Neurosci.,* 2013, vol. 20 (7), 1014
- *J. Urol.,* 2012, vol. 187 (1), 322